(19) Europäisches Patentamt
European Patent Office
Office européen des brevets

(11) EP 3 712 145 A1

(12) EUROPEAN PATENT APPLICATION

(43) Date of publication:
**23.09.2020 Bulletin 2020/39**

(21) Application number: **19203036.9**

(22) Date of filing: **14.10.2019**

(51) Int Cl.:
*C07D 403/14* (2006.01)
*C07D 405/14* (2006.01)
*C07D 409/14* (2006.01)
*C09K 11/06* (2006.01)
*H01L 51/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.03.2019 KR 20190031464**

(71) Applicant: **Samsung Electronics Co., Ltd.**
**Gyeonggi-do 16677 (KR)**

(72) Inventors:
- **JEON, Soonok**
  **16678 Gyeonggi-do (KR)**
- **CHUNG, Yeonsook**
  **16678 Gyeonggi-do (KR)**

(74) Representative: **Elkington and Fife LLP**
**Prospect House**
**8 Pembroke Road**
**Sevenoaks, Kent TN13 1XR (GB)**

(54) **CONDENSED CYCLIC COMPOUND AND ORGANIC LIGHT-EMITTING DEVICE INCLUDING SUCH COMPOUND**

(57) A condensed-cyclic compound represented by Formula 1, wherein $Y_{11}$ is a group represented by Formulae 2-1 or 2-2:

Formula 1

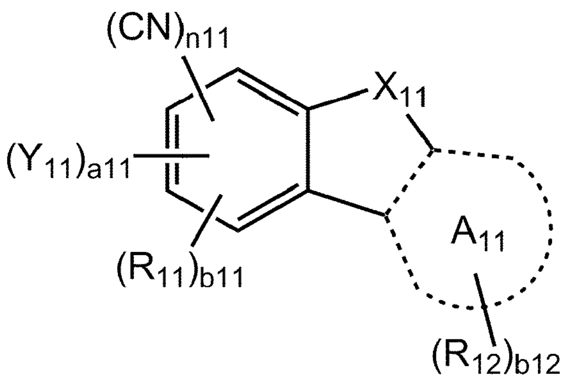

EP 3 712 145 A1

Formula 2-1

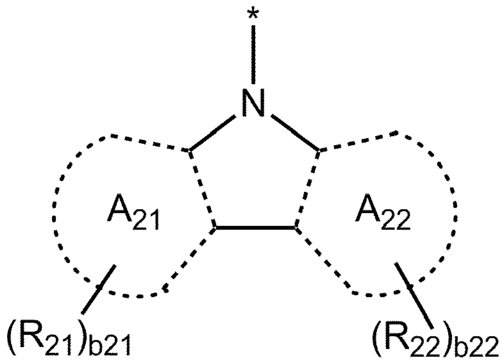

Formula 2-2

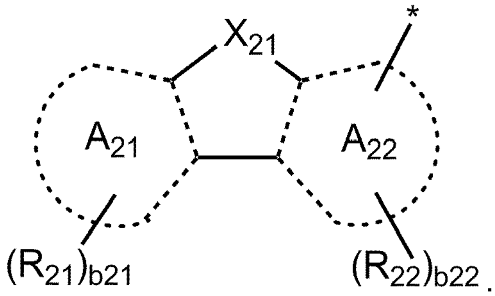

## Description

FIELD OF THE INVENTION

One or more embodiments relate to a condensed-cyclic compound and an organic light-emitting device including the same.

BACKGROUND OF THE INVENTION

Organic light-emitting devices are self-emissive devices and, compared with devices of the related art, have a wide viewing angle, a high contrast ratio, and a short response time, and exhibit excellent characteristics in terms of luminance, driving voltage, and response speed.

In an example, an organic light-emitting device includes an anode, a cathode, and an organic layer between the anode and the cathode, wherein the organic layer includes an emission layer. A hole transport region may be between the anode and the emission layer, and an electron transport region may be between the emission layer and the cathode. Holes provided from the anode may move toward the emission layer through the hole transport region, and electrons provided from the cathode may move toward the emission layer through the electron transport region. The holes and the electrons, which are carriers, recombine in the emission layer to produce excitons. These excitons transition from an excited state to a ground state, thereby generating light.

SUMMARY OF THE INVENTION

One or more embodiments include a condensed-cyclic compound having excellent delayed fluorescence emission characteristics and an organic light-emitting device that has high efficiency and/or a long lifespan due to the inclusion of the condensed-cyclic compound.

Additional aspects will be set forth in part in the description which follows and, in part, will be apparent from the description, or may be learned by practice of the presented embodiments.

According to one or more embodiments, a condensed-cyclic compound represented by Formula 1 is provided:

Formula 1

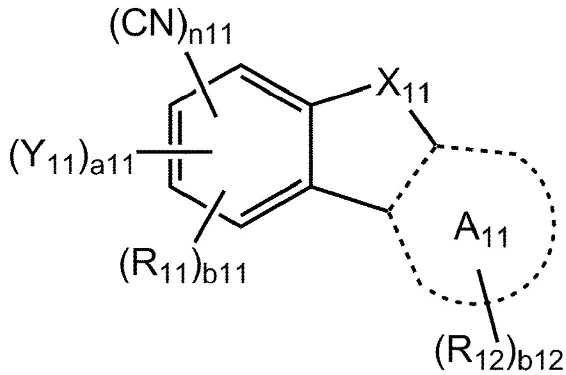

Formula 2-1

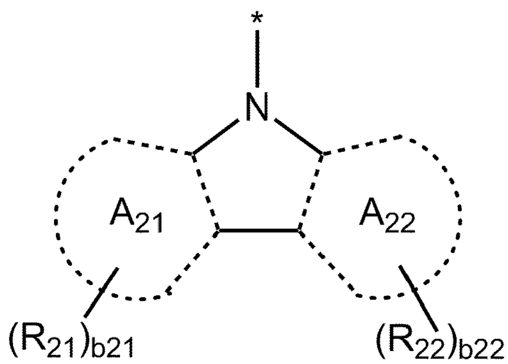

Formula 2-2

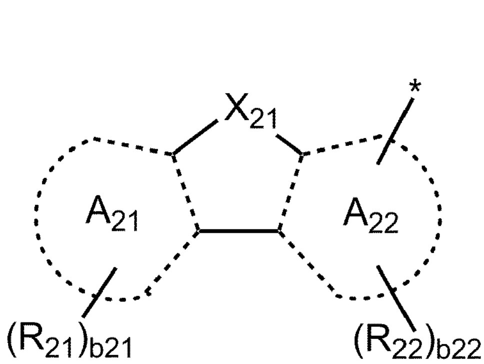

wherein, in Formulae 1, 2-1, and 2-2,

n11 may be 1 or 2;
$Y_{11}$ may be a group represented by Formulae 2-1 or 2-2;
a11 may be 2 or 3;
$X_{11}$ may be O, S, $N(R_{16})$, or $C(R_{16})(R_{17})$;
$A_{11}$, $A_{21}$, and $A_{22}$ may each independently be a $C_5$-$C_{60}$ carbocyclic group or a $C_1$-$C_{60}$ heterocyclic group;
$X_{21}$ may be O, S, $N(R_{23})$, or $C(R_{23})(R_{24})$;
$R_{11}$, $R_{12}$, and $R_{21}$ to $R_{24}$ may each independently be hydrogen, deuterium, -F, -Cl, -Br, -I, a hydroxyl group, a cyano group, a nitro group, an amidino group, a hydrazino group, a hydrazono group, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkylaryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkylheteroaryl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, $-Si(Q_1)(Q_2)(Q_3)$, $-B(Q_1)(Q_2)$, $-N(Q_1)(Q_2)$, $-P(Q_1)(Q_2)$, $-C(=O)(Q_1)$, $-S(=O)(Q_1)$, $-S(=O)_2(Q_1)$, $-P(=O)(Q_1)(Q_2)$, or-$P(=S)(Q_1)(Q_2)$,
b11 may be 0 or 1;
b12, b21, and b22 may each independently be 1, 2, 3, 4, 5, 6, 7, 8, 9, or 10;
the sum of n11, a11, and b11 may be 4;
$Q_1$ to $Q_3$ may each independently be hydrogen, deuterium, -F, -Cl, -Br, -I, a hydroxyl group, a cyano group, a nitro group, an amidino group, a hydrazino group, a hydrazono group, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_7$-$C_{60}$ alkylaryl group, a $C_6$-$C_{60}$ aryloxy group, a $C_6$-$C_{60}$ arylthio group, a $C_1$-$C_{60}$ heteroaryl group, a $C_2$-$C_{60}$ alkylheteroaryl group, a $C_1$-$C_{60}$ heteroaryloxy group, a $C_1$-$C_{60}$ heteroarylthio group, a monovalent non-aromatic condensed polycyclic group, a monovalent non-aromatic condensed heteropolycyclic group, a $C_1$-$C_{60}$ alkyl group which is substituted with deuterium, -F, a cyano group, a $C_1$-$C_{60}$ alkyl group, and a $C_6$-$C_{60}$ aryl group, and a $C_6$-$C_{60}$ aryl group which is substituted with at least one selected from deuterium, -F, a cyano group, a $C_1$-$C_{60}$ alkyl group, and a $C_6$-$C_{60}$ aryl group; and
* indicates a binding site to a neighboring atom.

According to one or more embodiments, an organic light-emitting device includes a first electrode; a second electrode; and an organic layer including an emission layer between the first electrode and the second electrode, wherein the organic layer includes the condensed-cyclic compound.

## BRIEF DESCRIPTION OF THE DRAWING

These and/or other aspects will become apparent and more readily appreciated from the following description of the embodiments, taken in conjunction with
FIGURE which shows a schematic cross-sectional view of an organic light-emitting device according to an embodiment.

## DETAILED DESCRIPTION OF THE EMBODIMENTS

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description.
It will be understood that when an element is referred to as being "on" another element, it can be directly on the other element or intervening elements may be present therebetween. In contrast, when an element is referred to as being "directly on" another element, there are no intervening elements present.
The terminology used herein is for the purpose of describing particular embodiments only and is not intended to be limiting. As used herein, the singular forms "a," "an," and "the" are intended to include the plural forms, including "at least one," unless the content clearly indicates otherwise. "Or" means "and/or." As used herein, the term "and/or" includes any and all combinations of one or more of the associated listed items. Expressions such as "at least one of,"

when preceding a list of elements, modify the entire list of elements and do not modify the individual elements of the list. It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" can mean within one or more standard deviations, or within ± 30%, 20%, 10%, or 5% of the stated value.

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Exemplary embodiments are described herein with reference to cross section illustrations that are schematic illustrations of idealized embodiments. As such, variations from the shapes of the illustrations as a result, for example, of manufacturing techniques and/or tolerances, are to be expected. Thus, embodiments described herein should not be construed as limited to the particular shapes of regions as illustrated herein but are to include deviations in shapes that result, for example, from manufacturing. For example, a region illustrated or described as flat may, typically, have rough and/or nonlinear features. Moreover, sharp angles that are illustrated may be rounded. Thus, the regions illustrated in the figures are schematic in nature and their shapes are not intended to illustrate the precise shape of a region and are not intended to limit the scope of the present claims.

The condensed-cyclic compound may be represented by Formula 1.

Formula 1

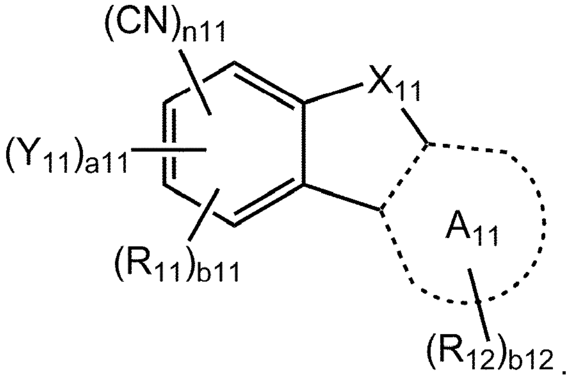

n11 in Formula 1 indicates the substitution number of cyano groups (CN), and n11 may be 1 or 2.

For example, n11 in Formula 1 may be 1, but embodiments are not limited thereto.

$Y_{11}$ in Formula 1 may be a group represented by one of Formulae 2-1 or 2-2:

Formula 2-1

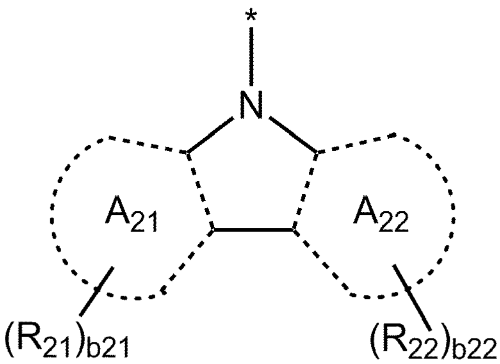

Formula 2-2

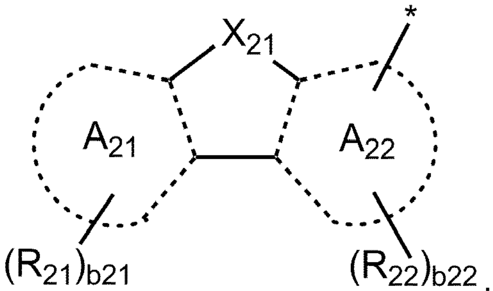

In Formulae 2-1 and 2-2, $A_{21}$, $A_{22}$, $X_{21}$, $R_{21}$, $R_{22}$, b21 and b22 may be understood by referring to the related description to be presented later, and * indicates a binding site to a neighboring atom.

a11 in Formula 1 indicates the substitution number of $Y_{11}$, and a11 may be 2 or 3. A plurality of $Y_{11}$(s) may be identical to or different from each other.

$X_{11}$ in Formula 1 may be O, S, N($R_{16}$), or C($R_{18}$)($R_{17}$), and $R_{16}$ and $R_{17}$ may be understood by referring to the related description to be presented later.

For example, $X_{11}$ in Formula 1 may be O, S, or N($R_{16}$), but embodiments are not limited thereto.

$A_{11}$, $A_{21}$ and $A_{22}$ in Formula 1, 2-1 and 2-2 may each independently be a $C_5$-$C_{60}$ carbocyclic group, or a $C_1$-$C_{60}$ heterocyclic group.

For example, $A_{11}$, $A_{21}$ and $A_{22}$ in Formulae 1, 2-1 and 2-2 may each independently be a benzene group, a naphthalene group, an anthracene group, a phenanthrene group, a phenalene group, a triphenylene group, a pyridine group, a pyrimidine group, a pyrazine group, a pyridazine group, a triazine group, a quinoline group, an isoquinoline group, a 2,6-naphthyridine group, a 1,8-naphthyridine group, a 1,5-naphthyridine group, a 1,6-naphthyridine group, a 1,7-naphthyridine group, a 2,7-naphthyridine group, a quinoxaline group, a phthalazine group, a quinazoline group, a phenanthroline group, a benzoquinoline group, a benzoisoquinoline group, a benzoquinoxaline group, a benzoquinazoline group, a furan group, a thiophene group, a silole group, an indene group, a fluorene group, an indole group, a carbazole group, a benzofuran group, a dibenzofuran group, a benzothiophene group, a dibenzothiophene group, a benzosilole group, a dibenzosilole group, an indenopyridine group, an indolopyridine group, a benzofuropyridine group, a benzothienopyridine group, a benzosilolopyridine group, an indenopyrimidine group, an indolopyrimidine group, a benzofuropyrimidine group, a benzothienopyrimidine group or a benzosilolopyrimidine group, but embodiments of the present disclosure are not limited thereto.

In one or more embodiments, $A_{11}$ in Formulae 1, 2-1, and 2-2 may be a benzene group, or a naphthalene group; $A_{21}$ and $A_{22}$ may each independently be a benzene group, a naphthalene group, a fluorene group, a carbazole group, a dibenzofuran group, or a dibenzothiophene group, but embodiments are not limited thereto.

In one or more embodiments, $A_{11}$ in Formulae 1, 2-1, and 2-2 may be a benzene group, or a naphthalene group; $A_{21}$ may be a benzene group, or a naphthalene group;

$A_{22}$ may be a benzene group, a naphthalene group, a fluorene group, a carbazole group, a dibenzofuran group, or a dibenzothiophene group, but embodiments are not limited thereto.

In one or more embodiments, $A_{11}$ in Formulae 1, 2-1, and 2-2 may be a benzene group, or a naphthalene group; $A_{21}$ may be a benzene group; and

$A_{22}$ may be a benzene group, a fluorene group, a carbazole group, a dibenzothiophene group, or a dibenzothiophene group, but embodiments are not limited thereto.

$X_{21}$ in Formulae 2-1 and 2-2 may be O, S, N($R_{23}$), or C($R_{23}$)($R_{24}$), and $R_{23}$ and $R_{24}$ may be understood by referring to the related description to be presented later.

$R_{11}$, $R_{12}$, and $R_{21}$ to $R_{24}$ in Formulae 1, 2-1 and 2-2 may each independently be hydrogen, deuterium, -F, -Cl, -Br, -I, a hydroxyl group, a cyano group, a nitro group, an amidino group, a hydrazino group, a hydrazono group, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkylaryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkylheteroaryl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, -Si($Q_1$)($Q_2$)($Q_3$), -B($Q_1$)($Q_2$), -N($Q_1$)($Q_2$), -P($Q_1$)($Q_2$), -C(=O)($Q_1$), -S(=O)($Q_1$), -S(=O)$_2$($Q_1$), -P(=O)($Q_1$)($Q_2$), or -P(=S)($Q_1$)($Q_2$),

$Q_1$ to $Q_3$ may each independently be hydrogen, deuterium, -F, -Cl, -Br, -I, a hydroxyl group, a cyano group, a nitro group, an amidino group, a hydrazino group, a hydrazono group, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_7$-$C_{60}$ alkylaryl group, a $C_6$-$C_{60}$ aryloxy group, a $C_6$-$C_{60}$ arylthio group, a $C_1$-$C_{60}$ heteroaryl group, a $C_2$-$C_{60}$ alkylheteroaryl group, a $C_1$-$C_{60}$ heteroaryloxy group, a $C_1$-$C_{60}$ heteroarylthio group, a monovalent non-aromatic condensed polycyclic group, a monovalent non-aromatic condensed heteropolycyclic group, a $C_1$-$C_{60}$ alkyl group which is substituted with at least one selected from deuterium, -F, a cyano group, a $C_1$-$C_{60}$ alkyl group, and a $C_6$-$C_{60}$ aryl group, and a $C_6$-$C_{60}$ aryl group which is substituted with at least one selected from deuterium, -F, a cyano group, a $C_1$-$C_{60}$ alkyl group, and a $C_6$-$C_{60}$ aryl group.

For example, $R_{11}$, $R_{12}$ and $R_{21}$ to $R_{24}$ in Formulae 1, 2-1 and 2-2 may each independently be hydrogen,

deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, or a $C_1$-$C_{20}$ alkoxy group;

a $C_1$-$C_{20}$ alkyl group or a $C_1$-$C_{20}$ alkoxy group, each substituted with deuterium,-F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group, a cyclopentyl group, a cyclohexyl group, a cycloheptyl group, a cycloctyl group, a bicyclo[2.2.1]heptyl group, an adamantyl group, a norbornyl group, a norbornenyl group, a cyclopentenyl group, a cyclohexenyl group, a cycloheptenyl group, a phenyl group, a biphenyl group, a $C_7$-$C_{20}$ alkylphenyl group, a naphthyl group, a pyridinyl group, a pyrimidinyl group, or a combination thereof;

a cyclopentyl group, a cyclohexyl group, a cycloheptyl group, a cycloctyl group, a bicyclo[2.2.1]heptyl group, an adamantyl group, a norbornyl group, a norbornenyl group, a cyclopentenyl group, a cyclohexenyl group, a cycloheptenyl group, a phenyl group, a biphenyl group, a $C_7$-$C_{20}$ alkylphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a dibenzosilolyl group, a benzocarbazolyl group, a dibenzocarbazolyl group, an imidazopyridinyl group, or an imidazopyrimidinyl group;

a cyclopentyl group, a cyclohexyl group, a cycloheptyl group, a cycloctyl group, a bicyclo[2.2.1]heptyl group, an adamantyl group, a norbornyl group, a norbornenyl group, a cyclopentenyl group, a cyclohexenyl group, a cycloheptenyl group, a phenyl group, a biphenyl group, a $C_7$-$C_{20}$ alkylphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a dibenzosilolyl group, a benzocarbazolyl group, a dibenzocarbazolyl group, an imidazopyridinyl group, or an imidazopyrimidinyl group, each substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a cyclopentyl group, a cyclohexyl group, a cycloheptyl group, a cycloctyl group, a bicyclo[2.2.1]heptyl group, an adamantyl group, a norbornyl group, a norbornenyl group, a cyclopentenyl group, a cyclohexenyl group, a cycloheptenyl group, a phenyl group, a biphenyl group, a $C_7$-$C_{20}$ alkylphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a dibenzosilolyl group, a benzocarbazolyl group, a dibenzocarbazolyl group, an imidazopyridinyl group, an imidazopyrimidinyl group, $-Si(Q_{11})(Q_{12})(Q_{13})$, $-B(Q_{11})(Q_{12})$, $-N(Q_{11})(Q_{12})$, or a combination thereof; or

$-Si(Q_1)(Q_2)(Q_3)$, $-B(Q_1)(Q_2)$, or $-N(Q_1)(Q_2)$;

$Q_1$ to $Q_3$ and $Q_{11}$ to $Q_{13}$ may each independently be

a methyl group, an ethyl group, an n-propyl group, an isopropyl group, an n-butyl group, an isobutyl group, a sec-butyl group, a tert-butyl group, an n-pentyl group, an isopentyl group, a 2-methylbutyl group, a sec-pentyl group, a tert-pentyl group, a neopentyl group, 3-pentyl group, 3-methyl-2-butyl group, a phenyl group, a biphenyl group, a $C_7$-$C_{20}$ alkylphenyl group, or a naphthyl group; or

a methyl group, an ethyl group, an n-propyl group, an isopropyl group, an n-butyl group, an isobutyl group, a sec-butyl group, a tert-butyl group, an n-pentyl group, an isopentyl group, a 2-methylbutyl group, a sec-pentyl group, a tert-pentyl group, a neopentyl group, 3-pentyl group, 3-methyl-2-butyl group, a phenyl group, or a naphthyl group, each substituted

with deuterium, a phenyl group, or a combination thereof, but embodiments of the present disclosure are not limited thereto.

In one embodiment, $R_{11}$, $R_{12}$ and $R_{21}$ to $R_{24}$ in Formulae 1, 2-1, and 2-2 may each independently be hydrogen, deuterium, -F, a cyano group, a nitro group, $-CH_3$, $-CD_3$,$-CD_2H$, $-CDH_2$, $-CF_3$, $-CF_2H$, $-CFH_2$, a group represented by one of Formulae 9-1 to 9-27, a group obtained by substituting hydrogen of the group represented by one of Formulae 9-1 to 9-27 with deuterium, a group represented by one of Formulae 10-1 to 10-226,-Si($Q_1$)($Q_2$)($Q_3$), -B($Q_1$)($Q_2$), or -N($Q_1$)($Q_2$), but embodiments of the present disclosure are not limited thereto:

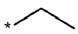
9-1
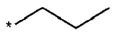
9-2
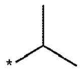
9-3
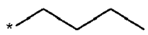
9-4
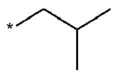
9-5
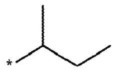
9-6
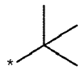
9-7
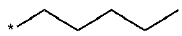
9-8
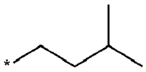
9-9
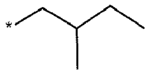
9-10
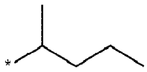
9-11
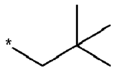
9-12
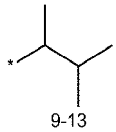
9-13
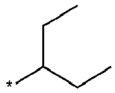
9-14
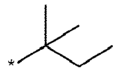
9-15
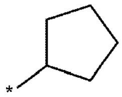
9-16
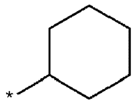
9-17
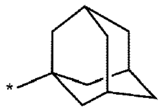
9-18
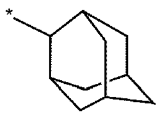
9-19
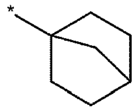
9-20
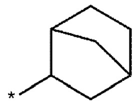
9-21
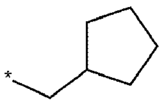
9-22
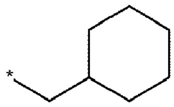
9-23
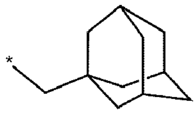
9-24
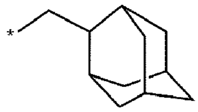
9-25
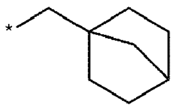
9-26
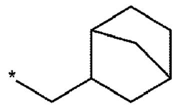
9-27
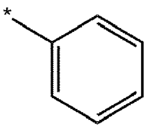
10-1
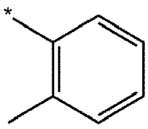
10-2
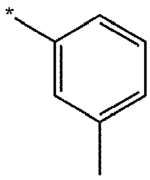
10-3
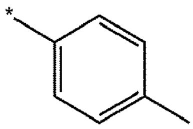
10-4
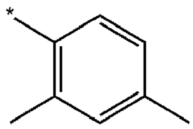
10-5
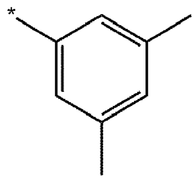
10-6
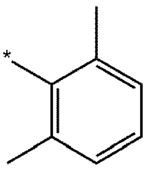
10-7
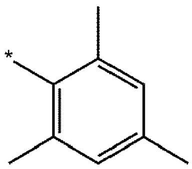
10-8
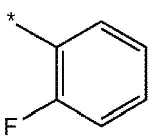
10-9
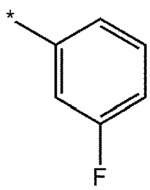
10-10
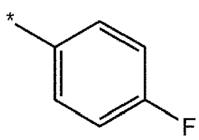
10-11
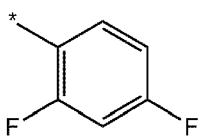
10-12

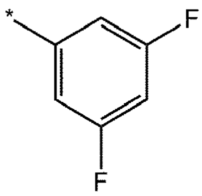
10-13
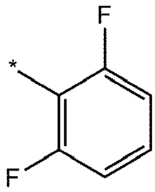
10-14
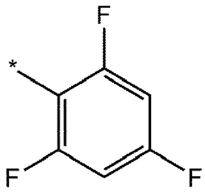
10-15
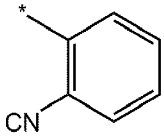
10-16
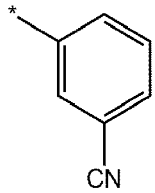
10-17
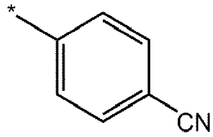
10-18

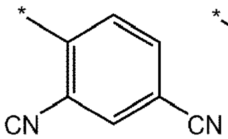
10-19
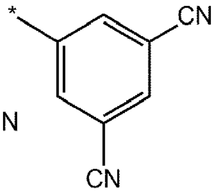
10-20
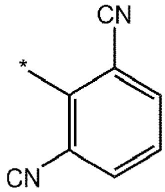
10-21
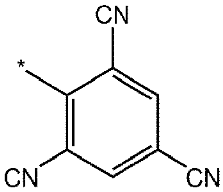
10-22
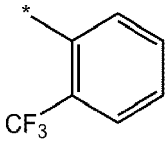
10-23
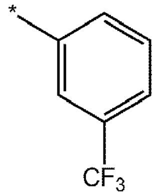
10-24

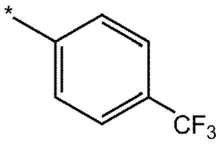
10-25
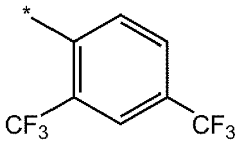
10-26
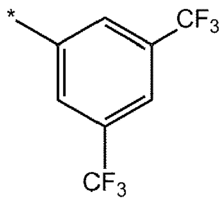
10-27
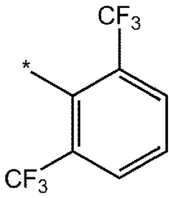
10-28
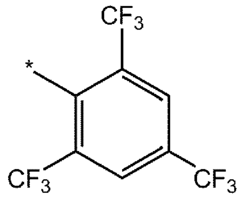
10-29

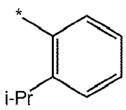
10-30
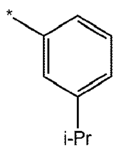
10-31
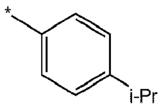
10-32
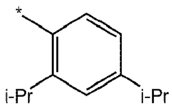
10-33
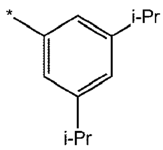
10-34
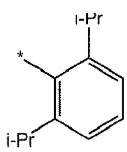
10-35
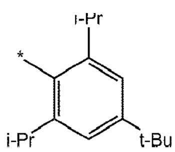
10-36

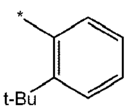
10-37
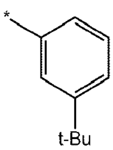
10-38
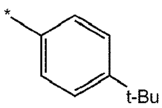
10-39
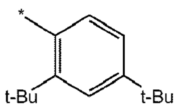
10-40
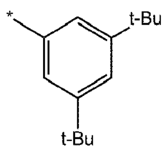
10-41
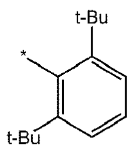
10-42
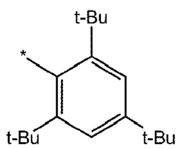
10-43

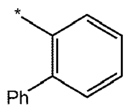
10-44

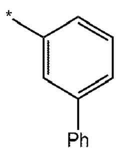
10-45

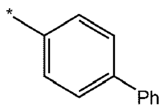
10-46

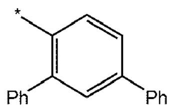
10-47

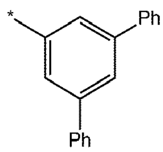
10-48

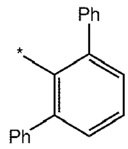
10-49

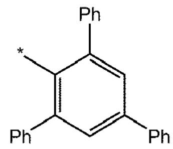
10-50

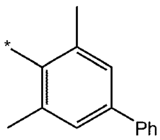
10-51

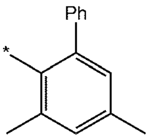
10-52

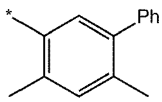
10-53

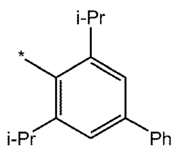
10-54

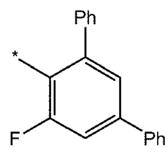
10-55

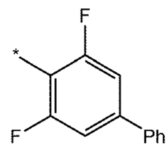
10-56

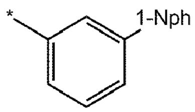
10-57

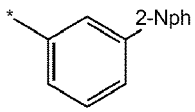
10-58

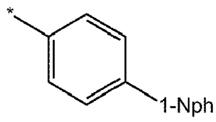
10-59

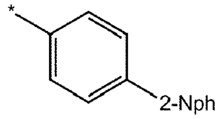
10-60

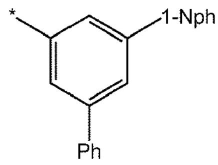
10-61

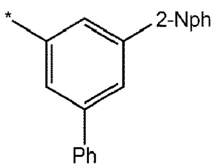
10-62

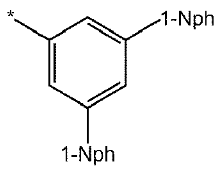
10-63

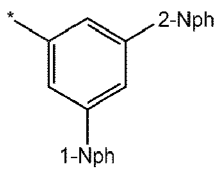
10-64

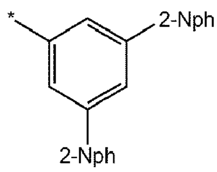
10-65

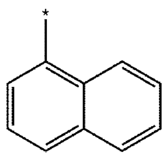
10-66

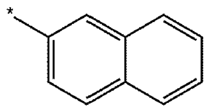
10-67

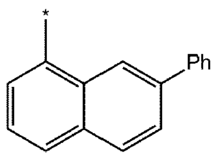
10-68

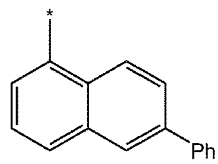
10-69

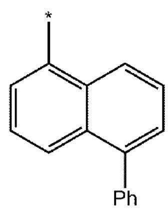
10-70

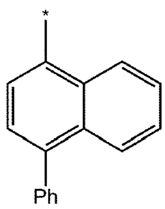
10-71

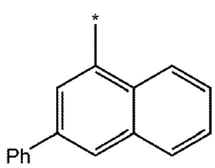
10-72

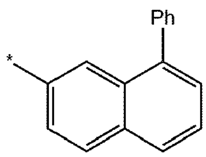
10-73

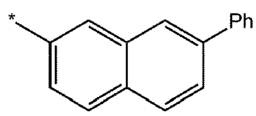
10-74

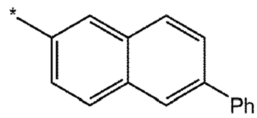
10-75

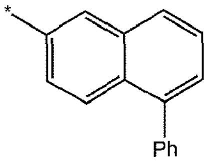
10-76

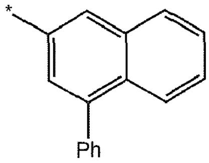
10-77

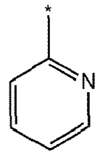
10-78

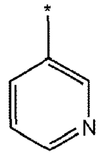
10-79

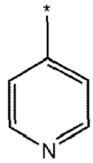
10-80

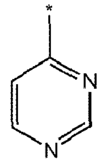
10-81

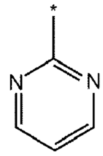
10-82

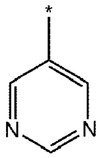
10-83

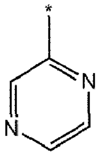
10-84

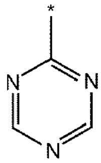
10-85

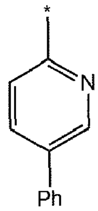
10-86

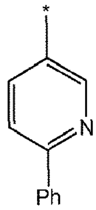
10-87

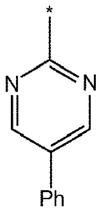
10-88

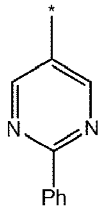
10-89

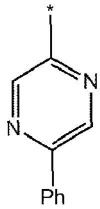
10-90

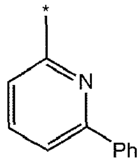
10-91

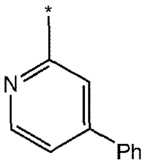
10-92

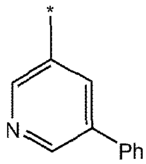
10-93

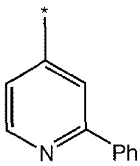
10-94

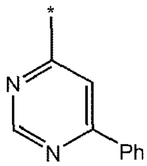
10-95

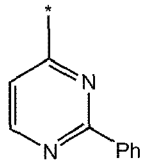
10-96

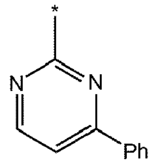
10-97

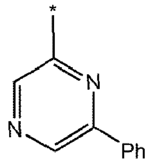
10-98

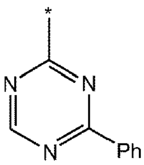
10-99

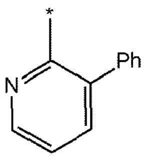
10-100

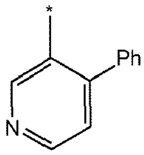
10-101

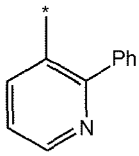
10-102

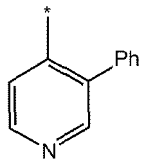
10-103

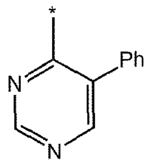
10-104

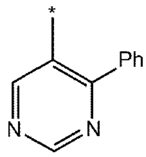
10-105

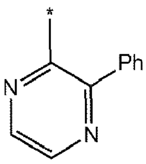
10-106

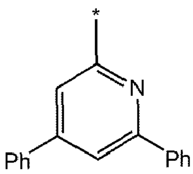
10-107

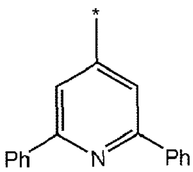
10-108

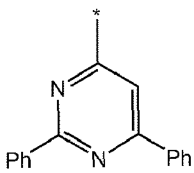
10-109

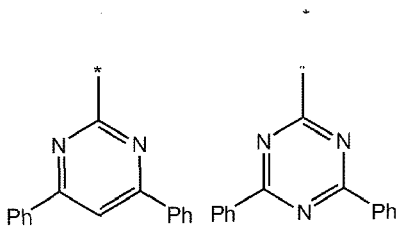
10-110

10-111

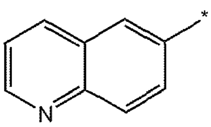
10-112

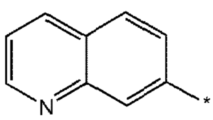
10-113

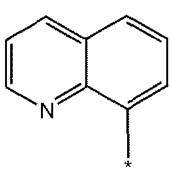
10-114

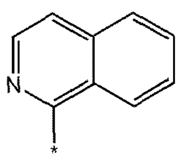
10-115

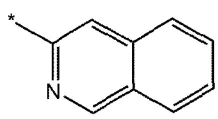
10-116

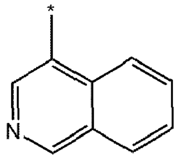
10-117

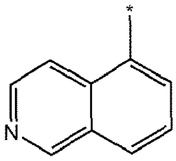
10-118

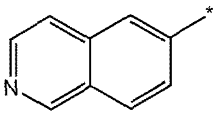
10-119

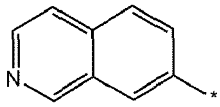
10-120

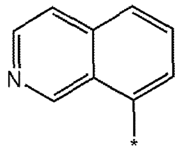
10-121

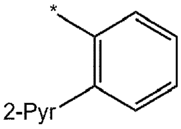
10-122

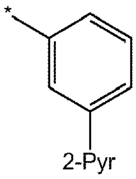
10-123

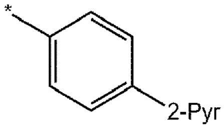
10-124

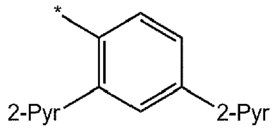
10-125

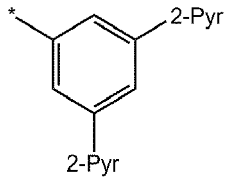
10-126

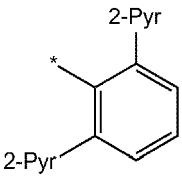
10-127

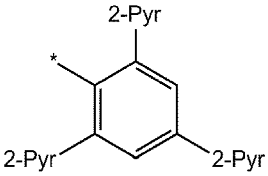
10-128

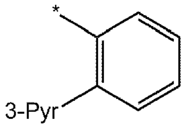
10-129

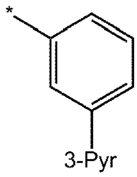
10-130

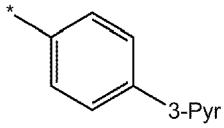
10-131

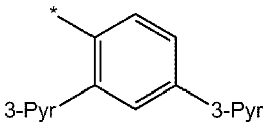
10-132

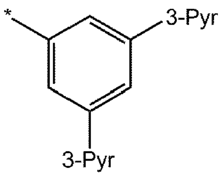
10-133

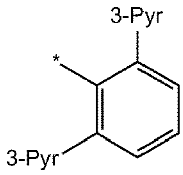
10-134

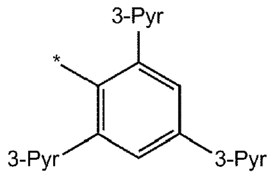
10-135

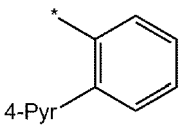
10-136

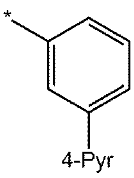
10-137

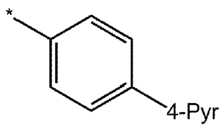
10-138

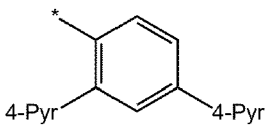
10-139

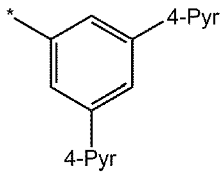
10-140

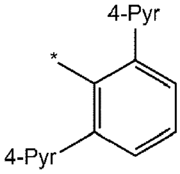
10-141

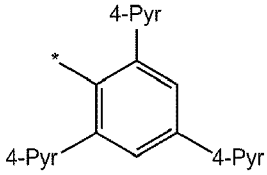
10-142

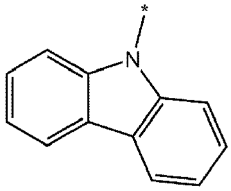
10-143

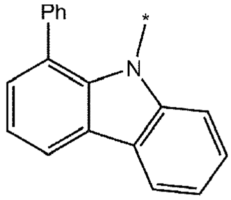
10-144

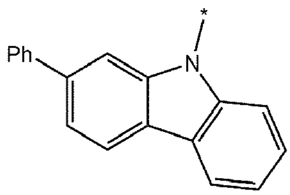
10-145

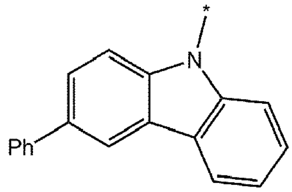
10-146

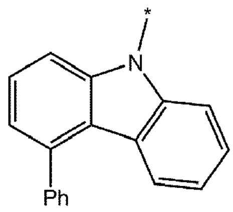
10-147

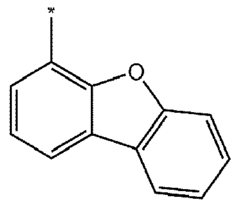
10-148

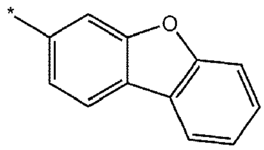
10-149

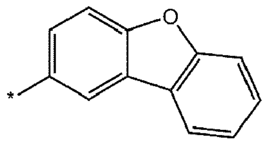
10-150

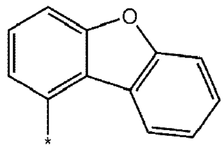
10-151

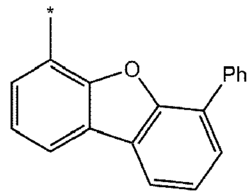
10-152

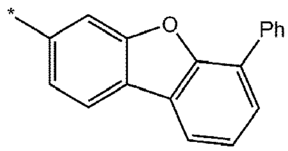
10-153

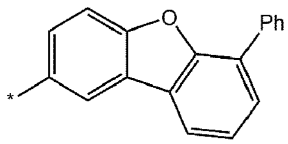
10-154

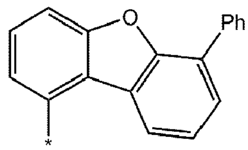
10-155

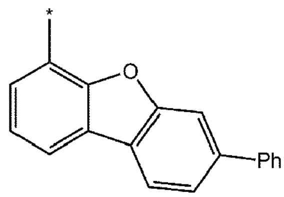
10-156

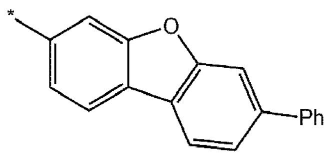
10-157

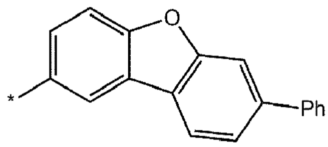
10-158

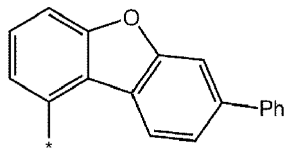
10-159

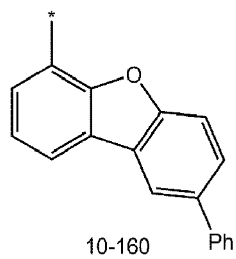
10-160

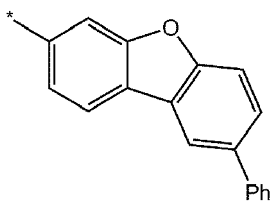
10-161

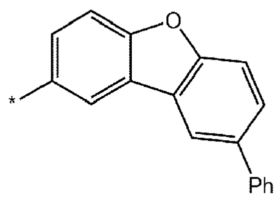
10-162

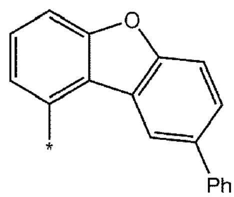
10-163

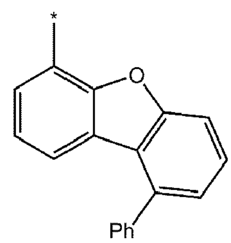
10-164

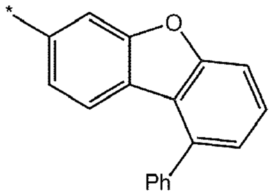
10-165

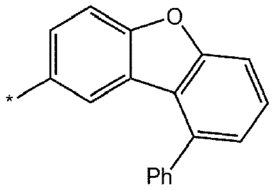
10-166

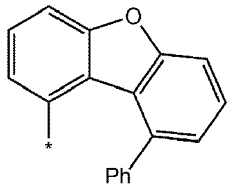
10-167

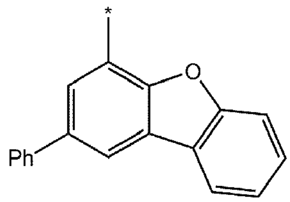
10-168

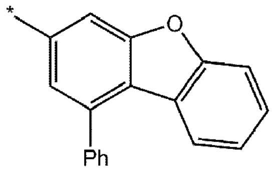
10-169

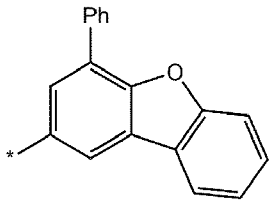
10-170

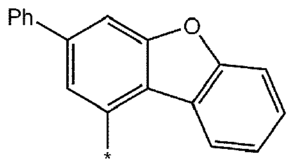
10-171

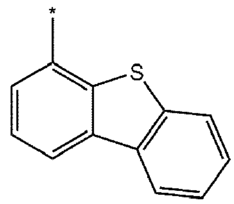
10-172

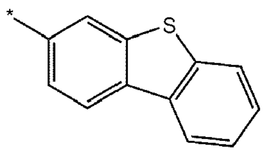
10-173

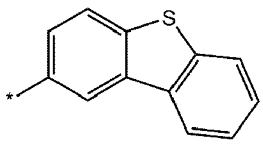
10-174

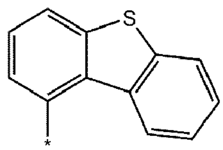
10-175

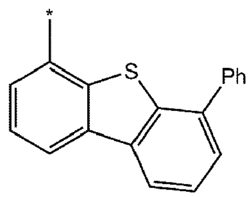
10-176

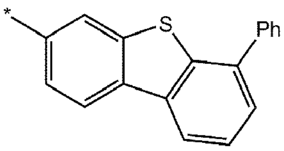
10-177

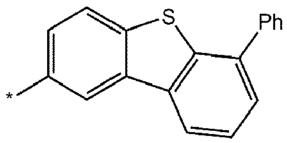
10-178

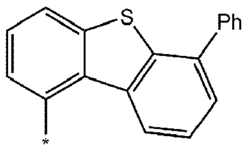
10-179

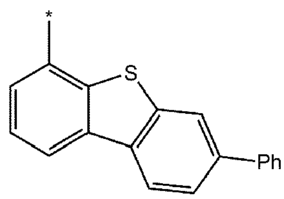
10-180

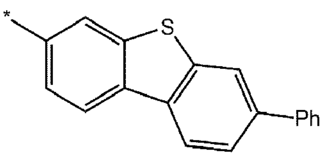
10-181

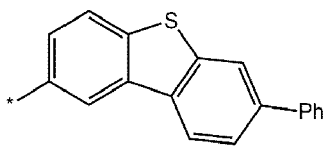
10-182

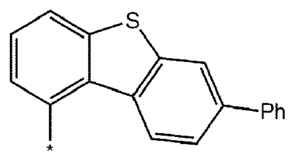
10-183

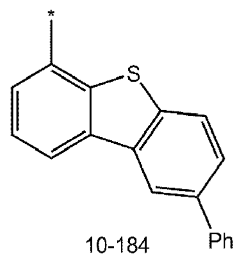
10-184

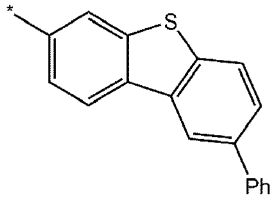

10-185

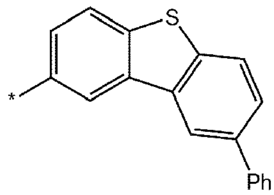

10-186

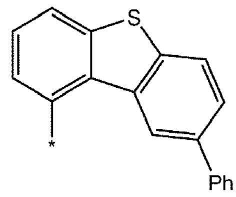

10-187

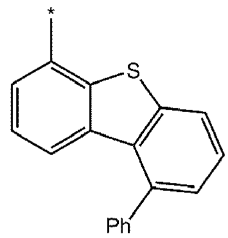

10-188

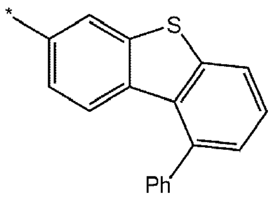

10-189

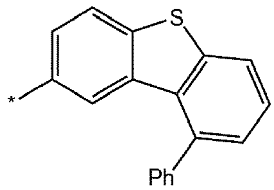

10-190

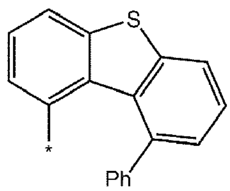

10-191

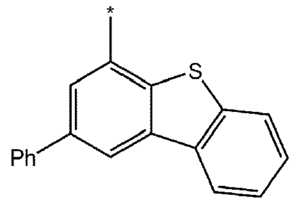

10-192

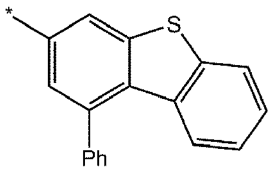

10-193

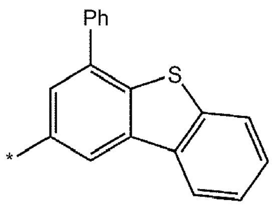

10-194

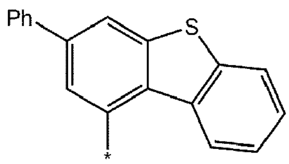

10-195

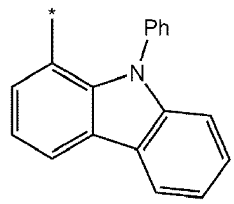

10-196

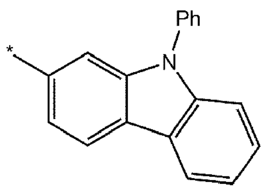

10-197

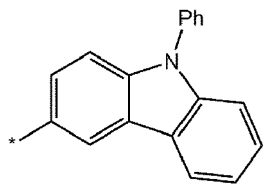

10-198

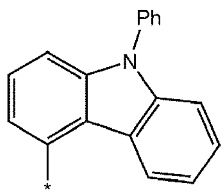

10-199

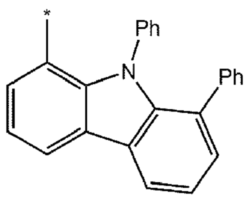

10-200

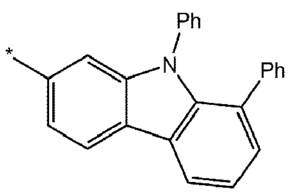

10-201

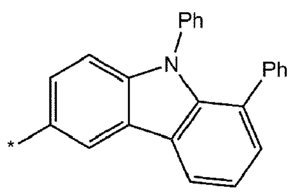

10-202

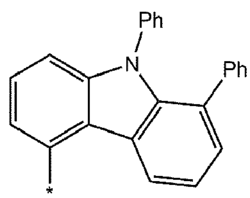

10-203

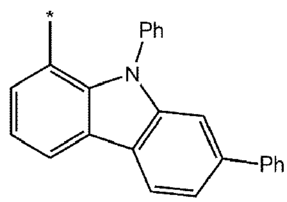

10-204

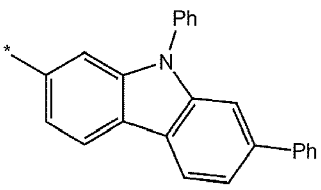
10-205
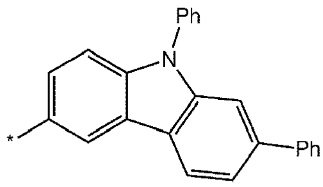
10-206
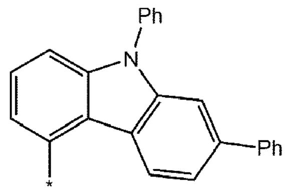
10-207
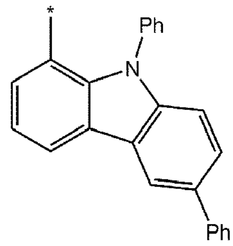
10-208

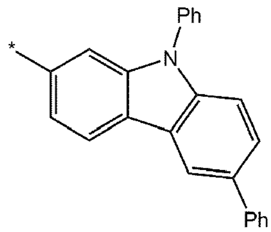
10-209
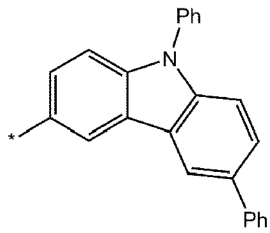
10-210
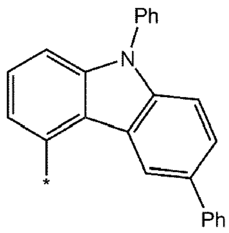
10-211
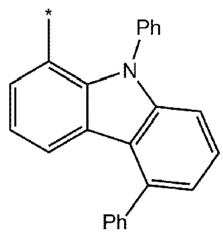
10-212

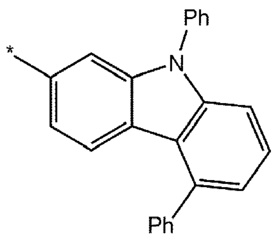
10-213
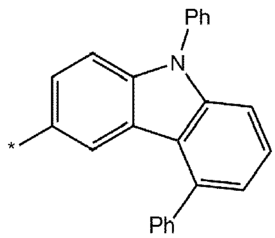
10-214
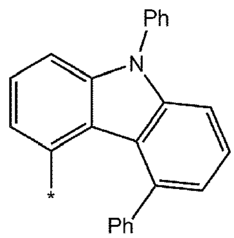
10-215
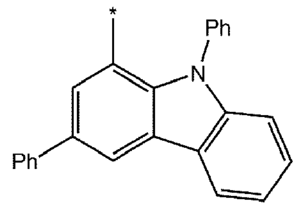
10-216

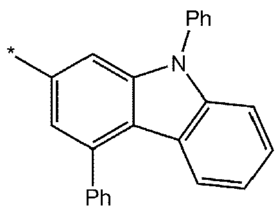
10-217
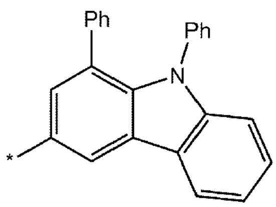
10-218
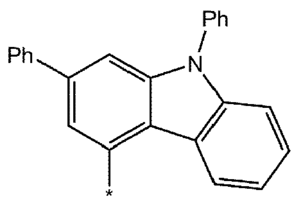
10-219

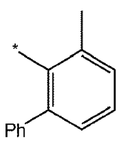
10-220
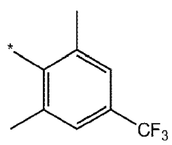
10-221
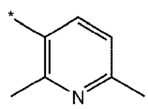
10-222
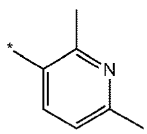
10-223
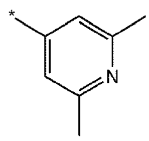
10-224
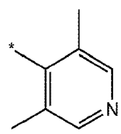
10-225
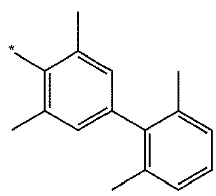
10-226
.

In Formulae 9-1 to 9-27 and 10-1 to 10-226,

* indicates a binding site to a neighboring atom,

i-Pr is an isopropyl group, and t-Bu is a tert-butyl group,
Ph is a phenyl group;
1-Nph is a 1-naphthyl group, and 2-Nph is 2-naphthyl group,
2-Pyr is a 2-pyridyl group, 3-Pyr is a 3-pyridyl group, and 4-Pyr is a 4-pyridyl group,
$Q_1$ to $Q_3$ may each independently be
a methyl group, an ethyl group, an n-propyl group, an isopropyl group, an n-butyl group, an isobutyl group, a sec-butyl group, a tert-butyl group, an n-pentyl group, an isopentyl group, a 2-methylbutyl group, a sec-pentyl group, a tert-pentyl group, a neopentyl group, 3-pentyl group, 3-methyl-2-butyl group, a phenyl group, a biphenyl group, a $C_7$-$C_{20}$ alkylphenyl group, or a naphthyl group; or
a methyl group, an ethyl group, an n-propyl group, an isopropyl group, an n-butyl group, an isobutyl group, a sec-butyl group, a tert-butyl group, an n-pentyl group, an isopentyl group, a 2-methylbutyl group, a sec-pentyl group, a tert-pentyl group, a neopentyl group, 3-pentyl group, 3-methyl-2-butyl group, a phenyl group, or a naphthyl group, each substituted with deuterium, a phenyl group, or a combination thereof.

In one or more embodiments, $R_{12}$ in Formula 1 may be hydrogen or a group represented by one of Formulae 10-1 and 10-107 to 10-111, but embodiments are not limited thereto.
In one or more embodiments, $R_{21}$ and $R_{22}$ in Formulae 2-1 and 2-2 may each independently be hydrogen, a group represented by Formula 9-7 or a group represented by Formula 10-1, but embodiments are not limited thereto.
b11 in Formula 1 indicates the substitution number of $R_{11}$, and b11 may be 0 or 1.
The sum of n11, a11, and b11 in Formula 1 may be 4.
b12, b21, and b22 in Formulae 1, 2-1, and 2-2 may each independently be 1, 2, 3, 4, 5, 6, 7, 8, 9, or 10.
In one or more embodiments, $Y_{11}$ in Formula 1 may be represented by any one of Formulae 2-11 to 2-17, but embodiments are not limited thereto:

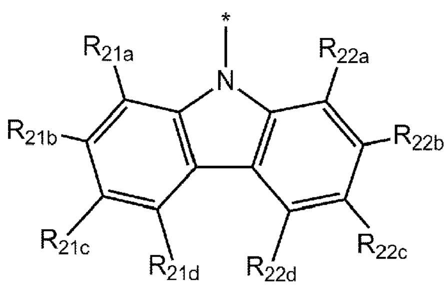
2-11

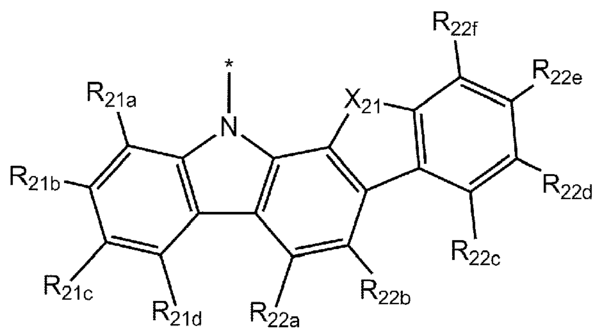
2-12

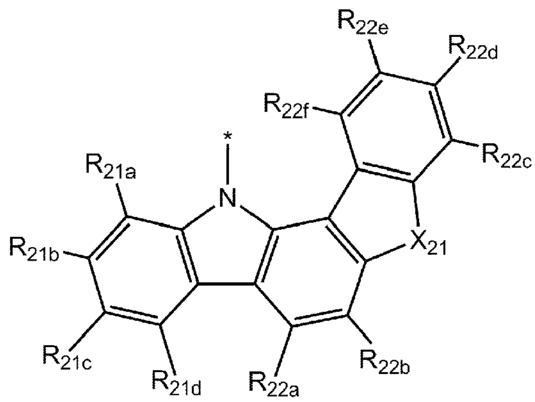
2-13

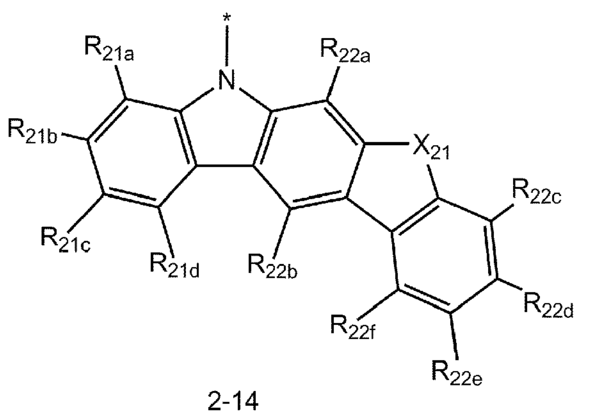
2-14

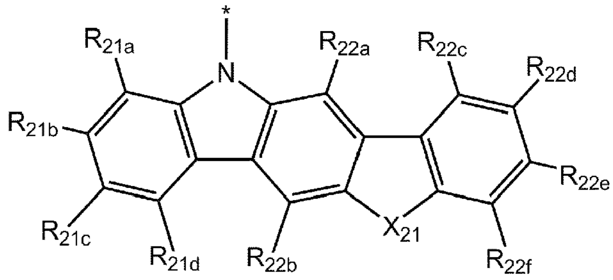

2-15

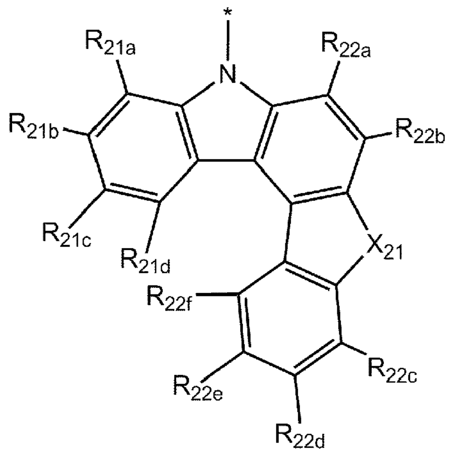

2-16

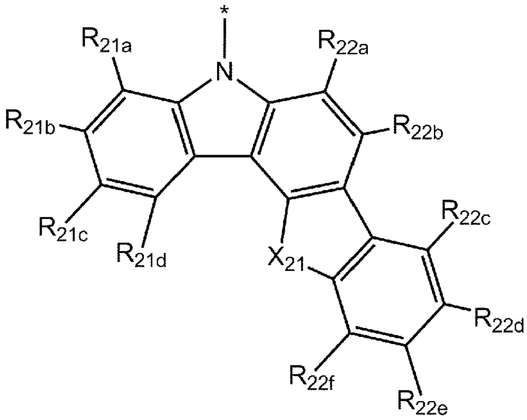

2-17

wherein, in Formulae 2-11 to 2-17,

$X_{21}$ may be O, S, N($R_{22g}$), or C($R_{22g}$)($R_{22h}$);
$R_{21a}$ to $R_{21d}$ may be understood by referring to the definition of $R_{21}$ of Formula 2-1;
$R_{22a}$ to $R_{22h}$ may be understood by referring to the definition of $R_{22}$ of Formula 2-1; and
* indicates a binding site to a neighboring atom.

In one embodiment, the condensed-cyclic compound represented by Formula 1 may be represented by one of Formulae 1-1 to 1-6, but embodiments are not limited thereto:

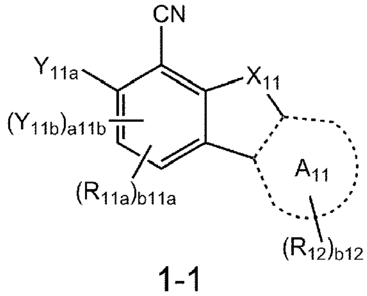

1-1

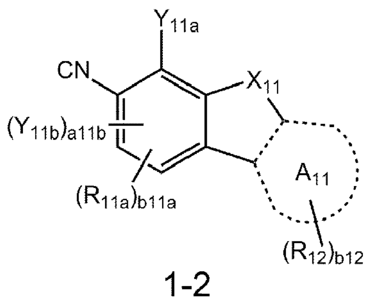

1-2

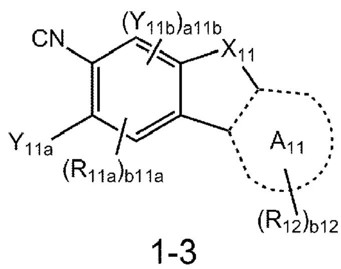

1-3

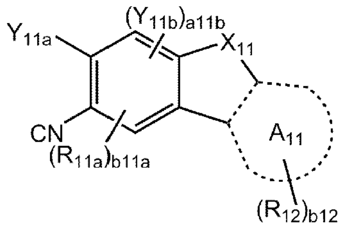

1-4

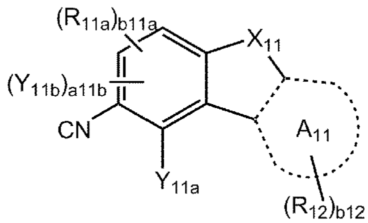

1-5

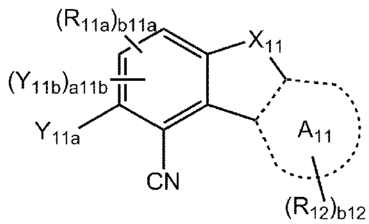

1-6

wherein, in Formulae 1-1 to 1-6,

$Y_{11a}$ and $Y_{11b}$ may each be understood by referring to the definition of $Y_{11}$ of Formula 1,
$R_{11a}$ may be understood by referring to the definition of $R_{11}$ of Formula 1,
a11b may be 1 or 2;
$X_{11}$, $A_{11}$, $R_{12}$, and b12 may each be understood by referring to the definitions provided in connection with Formula 1,
$A_{21}$, $A_{22}$, $X_{21}$, $R_{21}$ to $R_{24}$, $b_{21}$, and $b_{22}$ may each be understood by referring to the definitions provided in connection with Formulae 2-1 and 2-2, and
the sum of a11b and b11a may be 3.

For example, $A_{11}$ in Formula 1-1 to 1-6 may be a benzene group, but embodiments are not limited thereto.

For example, $Y_{11}$ in Formulae 1-1 to 1-6 may be represented by any one of Formulae 2-11 to 2-17, but embodiments are not limited thereto.

In one or more embodiments, the condensed-cyclic compound represented by Formula 1 may be represented by any one of Formulae 1-11 to 1-23, but embodiments are not limited thereto:

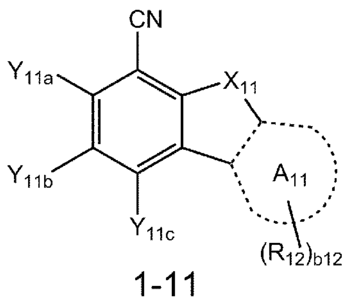

1-11

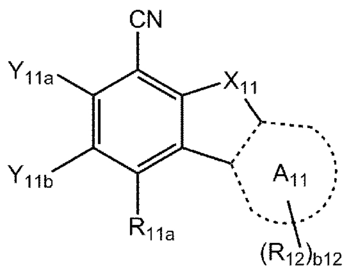

1-12

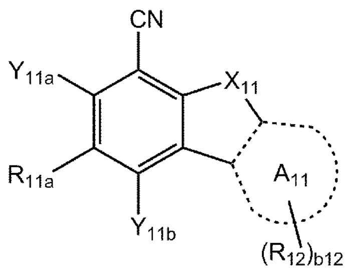

1-13

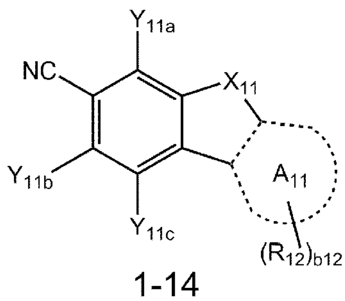

1-14

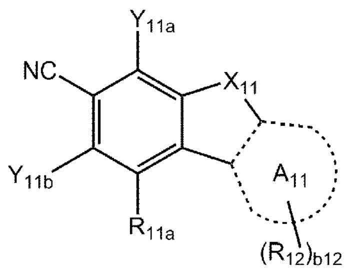

1-15

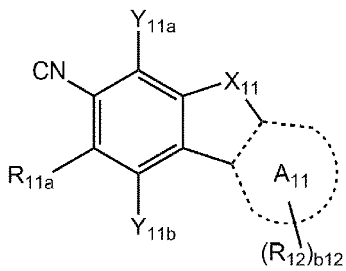

1-16

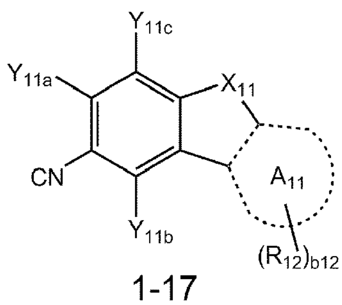

1-17

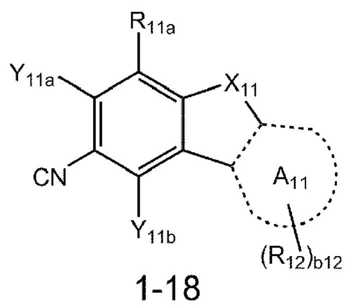

1-18

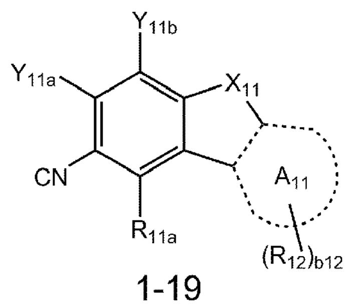

1-19

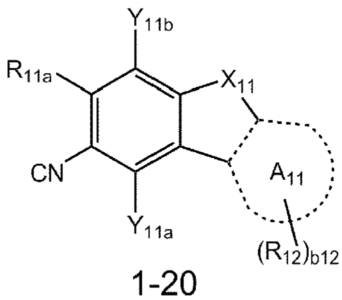

1-20

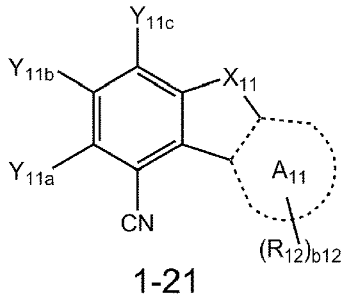

1-21

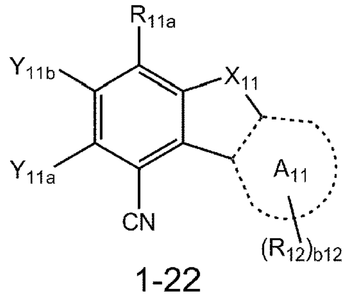

1-22

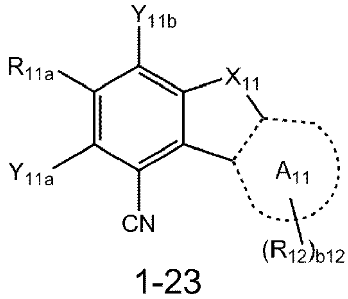

1-23

In Formulae 1-11 to 1-23,

$Y_{11a}$, $Y_{11b}$, and $Y_{11c}$ may each be understood by referring to the definition of $Y_{11}$ of Formula 1,
$R_{11a}$ may be understood by referring to the definition of $R_{11}$ of Formula 1,
$X_{11}$, $A_{11}$, $R_{12}$, and b12 may each be understood by referring to the definitions provided in connection with Formula 1, and
$A_{21}$, $A_{22}$, $X_{21}$, $R_{21}$ to $R_{24}$, b21, and b22 may each be understood by referring to the definitions provided in connection with Formulae 2-1 and 2-2.

For example, $A_{11}$ in Formula 1-11 to 1-23 may be a benzene group, but embodiments are not limited thereto.

For example, $Y_{11}$ in Formula 1-11 to 1-23 may be represented by any one of Formulae 2-11 to 2-17, but embodiments are not limited thereto.

In one embodiment, the condensed-cyclic compound represented by Formula 1 may be Compounds 1 to 336, but embodiments are not limited thereto:

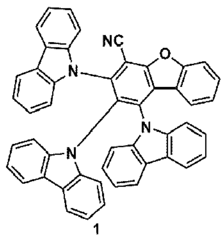

1

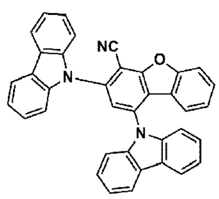

2

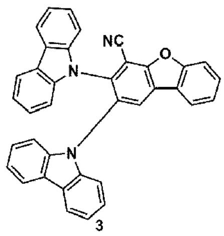

3

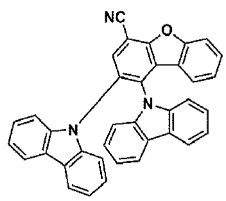

4

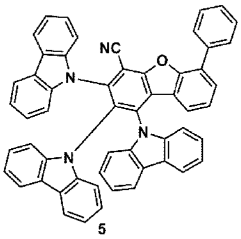

5

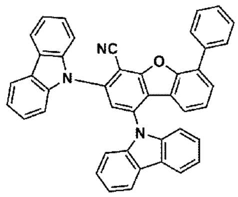

6

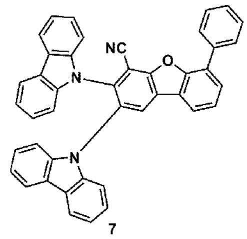

7

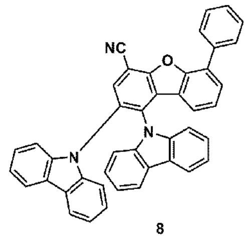

8

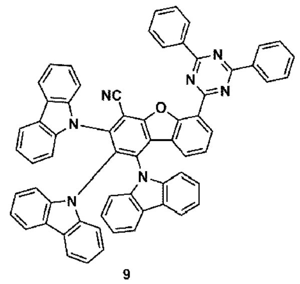
9

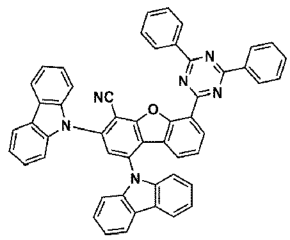
10

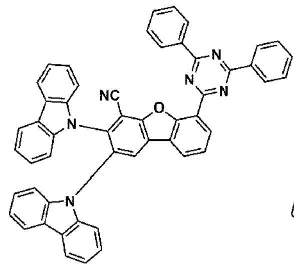
11

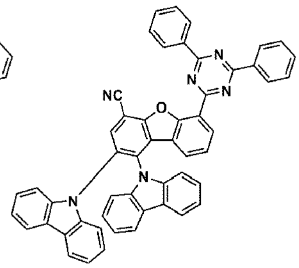
12

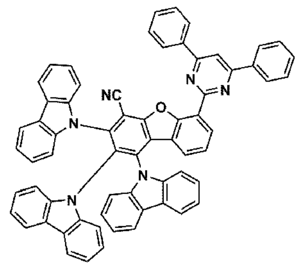
13

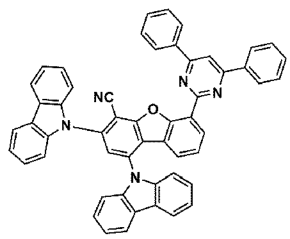
14

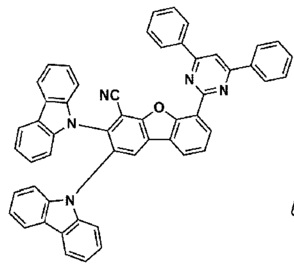
15

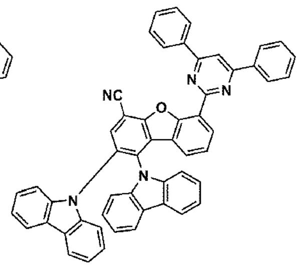
16

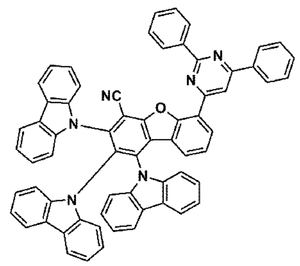
17

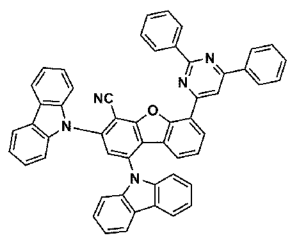
18

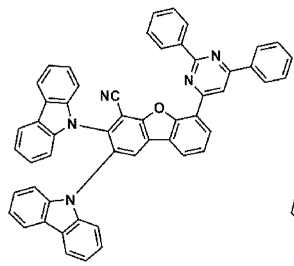
19

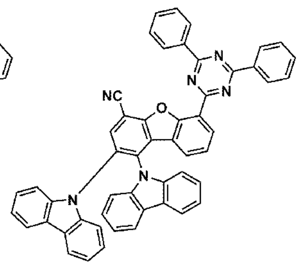
20

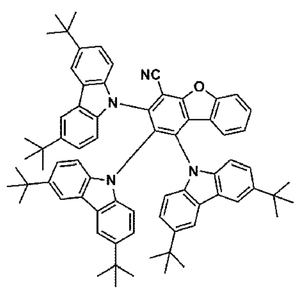
21

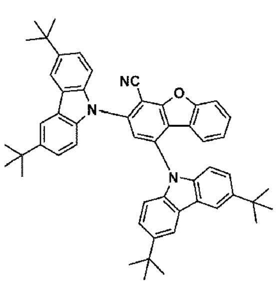
22

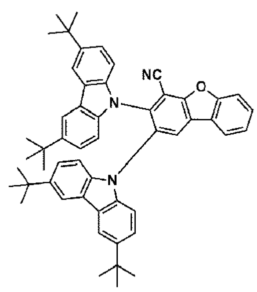
23

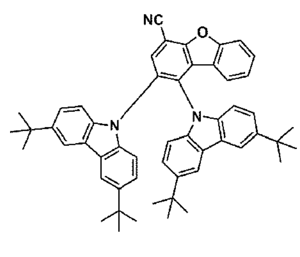
24

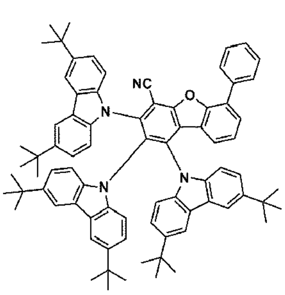
25

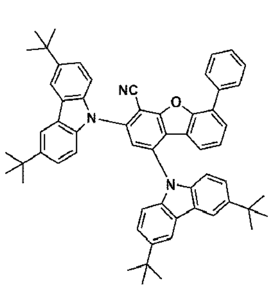
26

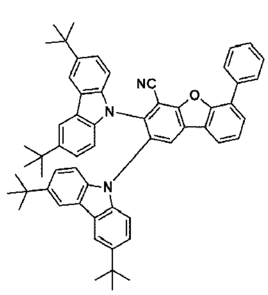
27

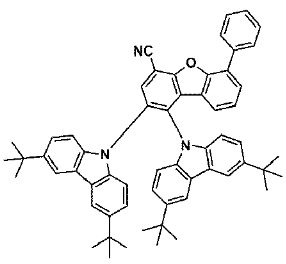
28

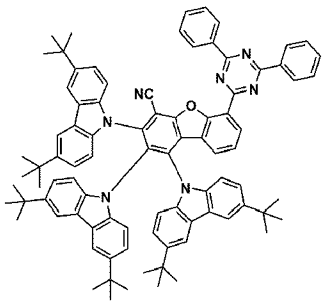

29

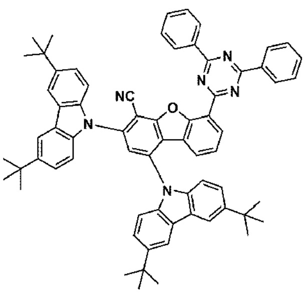

30

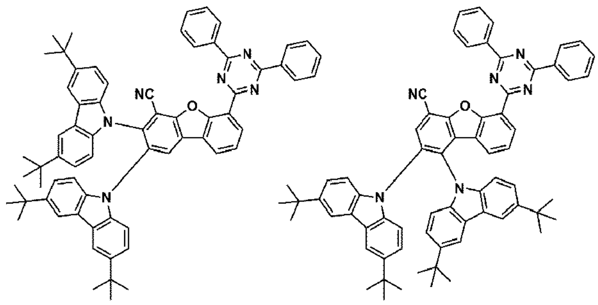

31

32

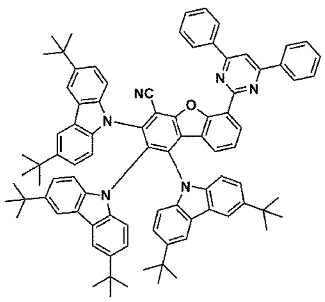

33

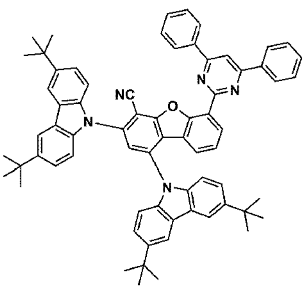

34

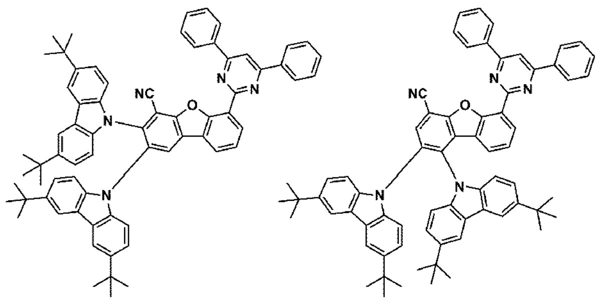

35

36

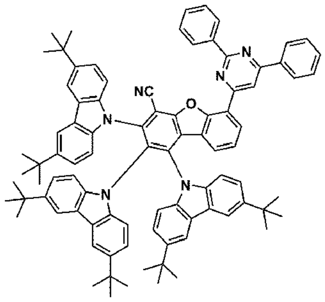

37

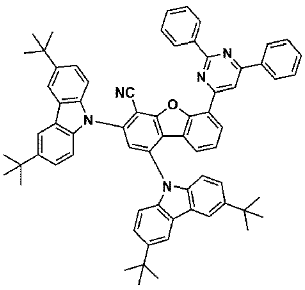

38

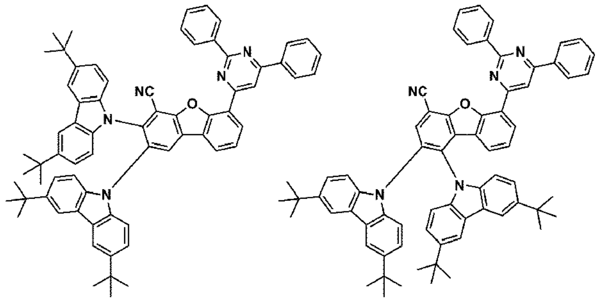

39

40

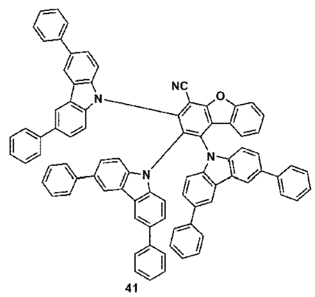

41

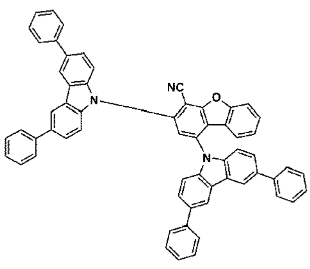

42

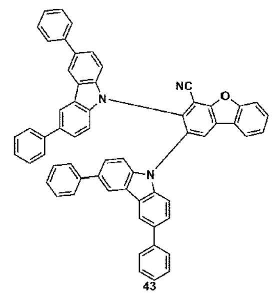

43

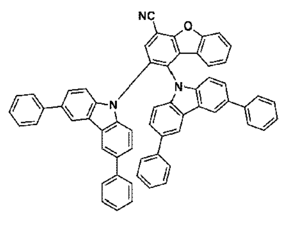

44

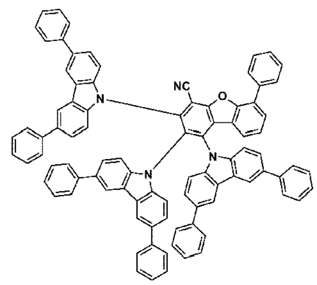

45

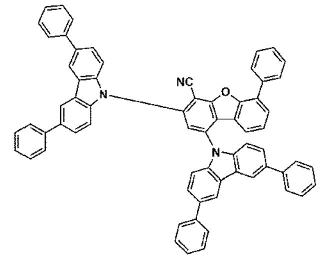

46

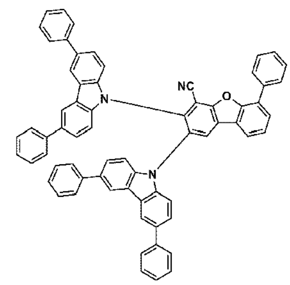

47

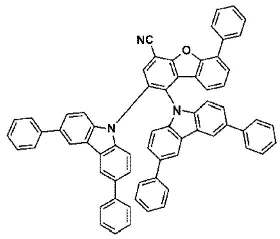

48

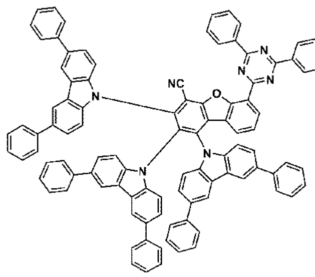

49

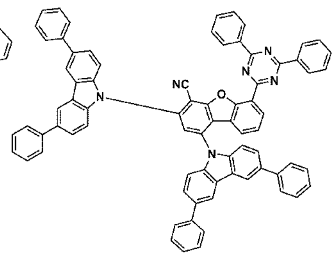

50

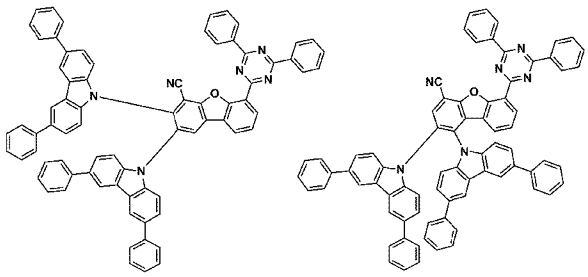

51

52

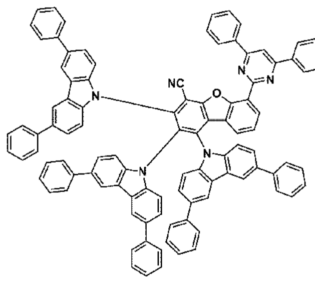

53

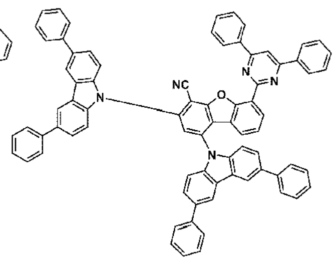

54

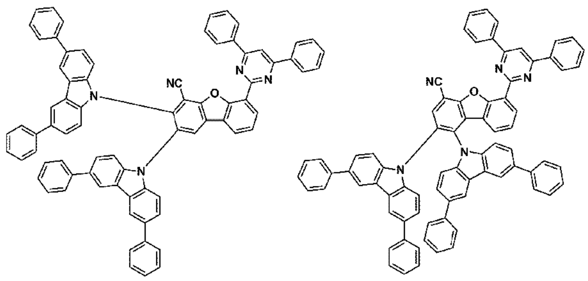

55

56

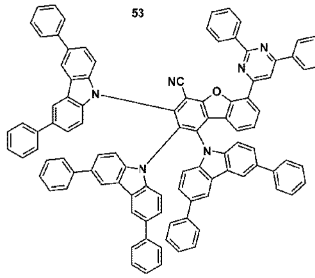

57

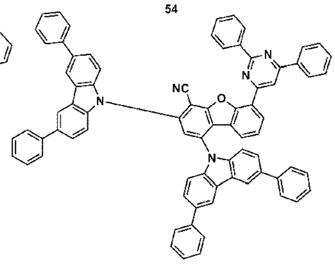

58

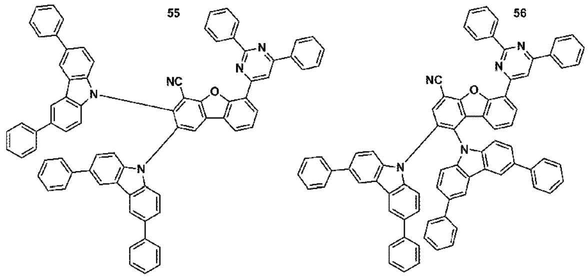

59

60

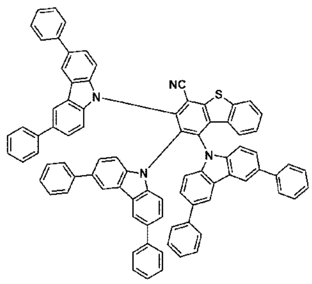

61

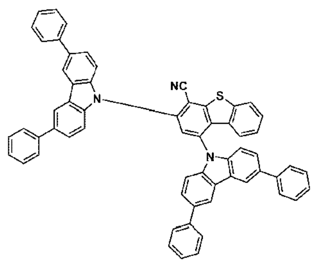

62

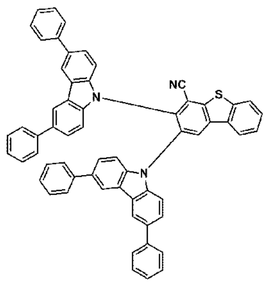

63

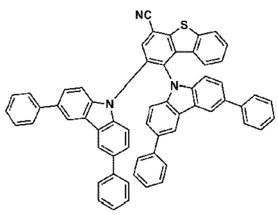

64

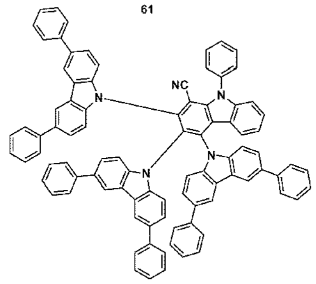

65

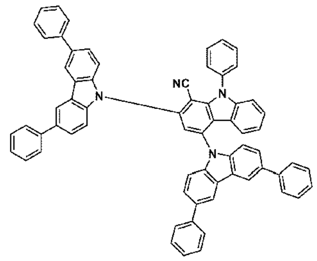

66

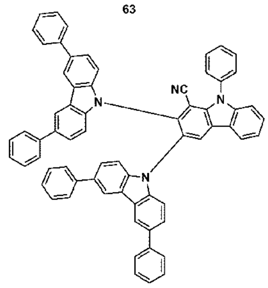

67

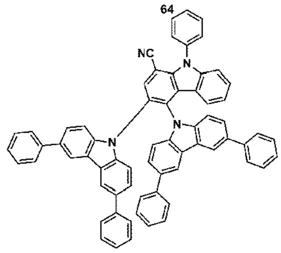

68

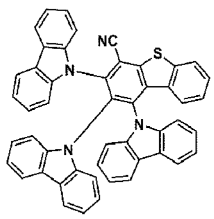

69

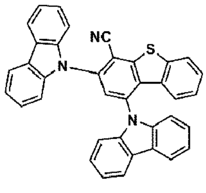

70

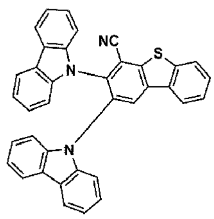

71

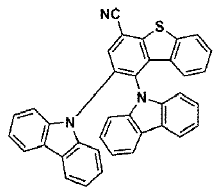

72

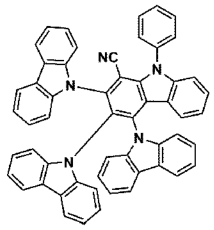

73

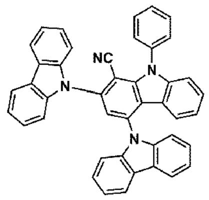

74

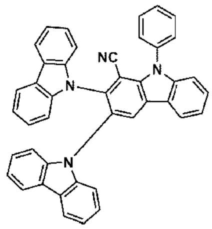

75

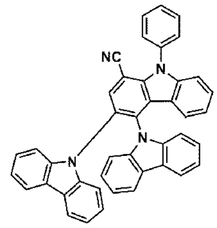

76

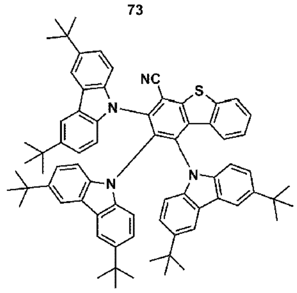

77

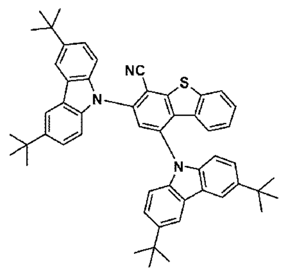

78

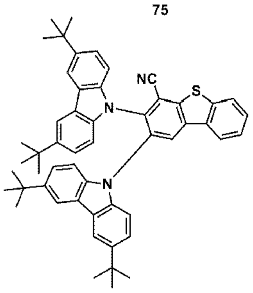

79

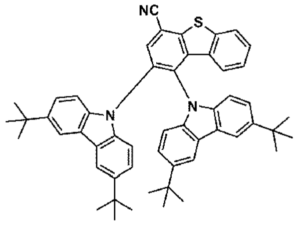

80

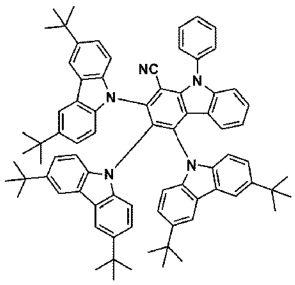
81

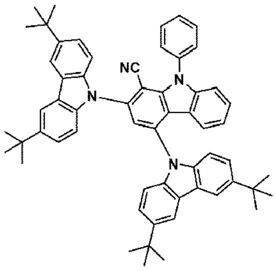
82

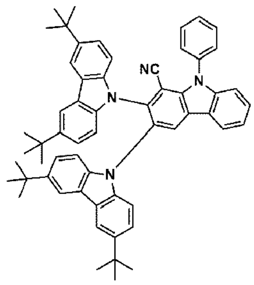
83

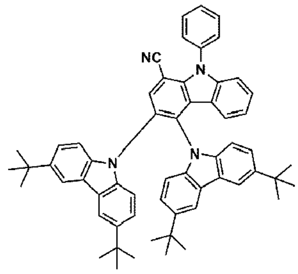
84

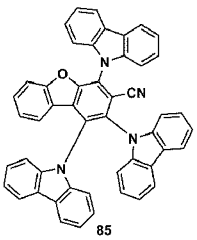
85

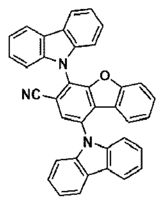
86

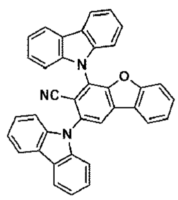
87

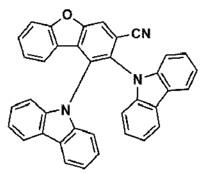
88

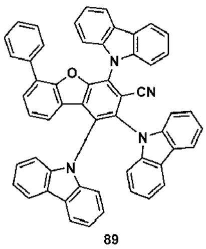
89

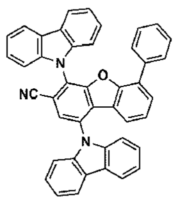
90

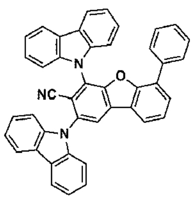
91

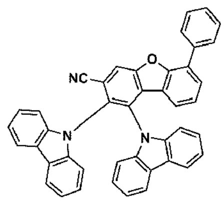
92

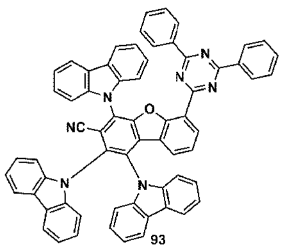
93

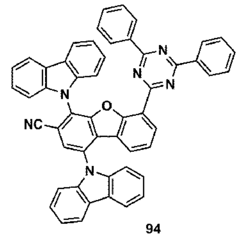
94

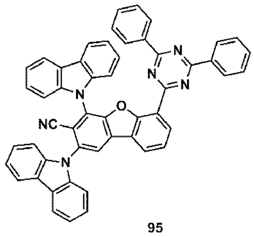
95

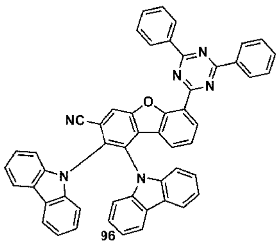
96

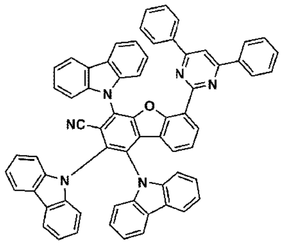
97

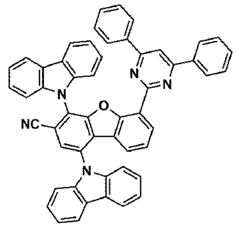
98

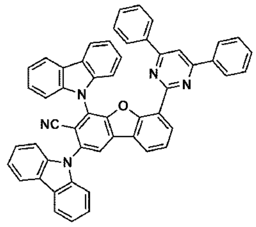
99

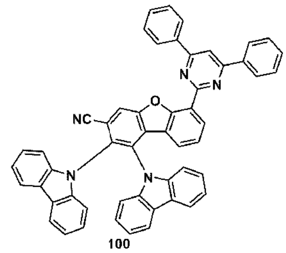
100

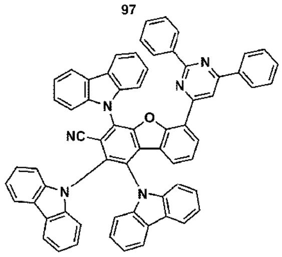
101

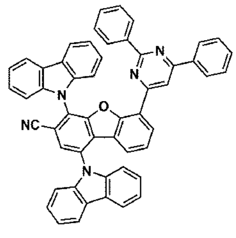
102

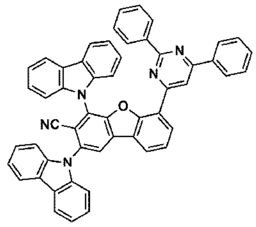
103

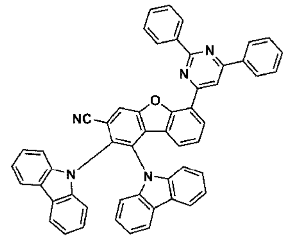
104

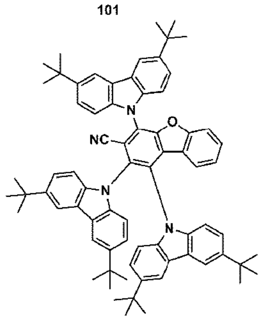
105

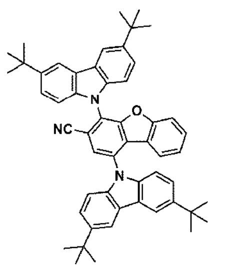
106

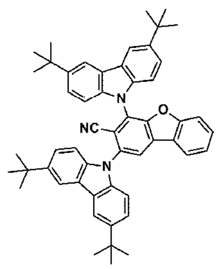
107

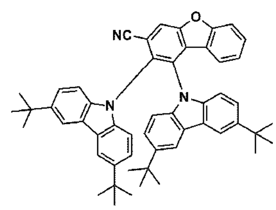
108

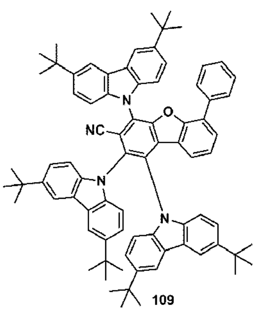
109

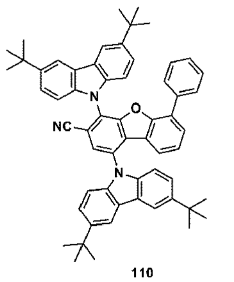
110

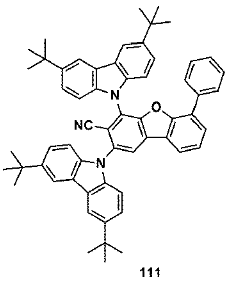
111

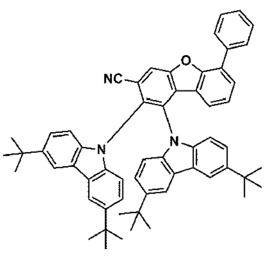
112

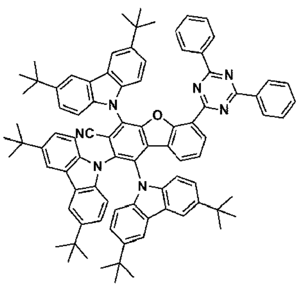
113

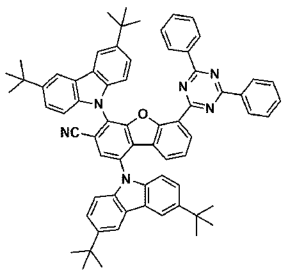
114

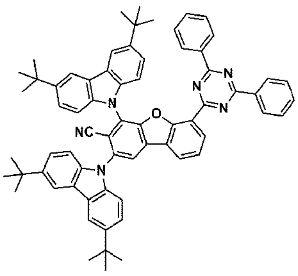
115

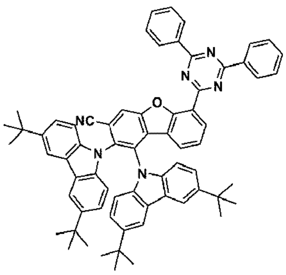
116

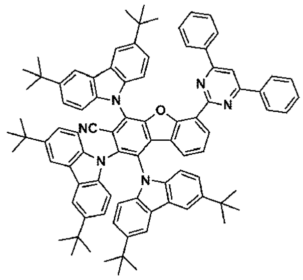
117

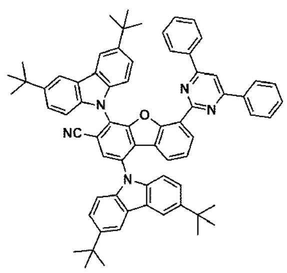
118

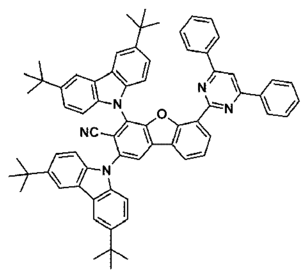
119

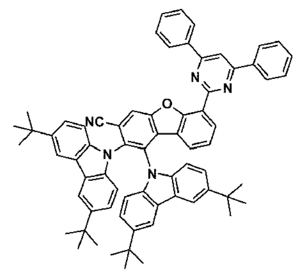
120

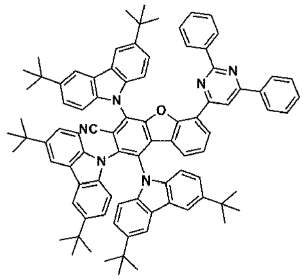
121

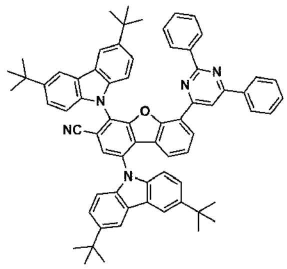
122

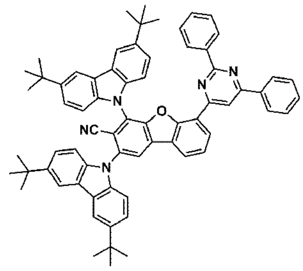
123

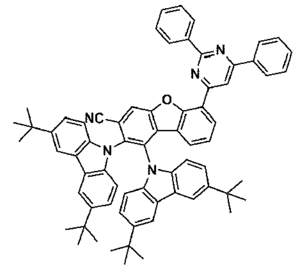
124

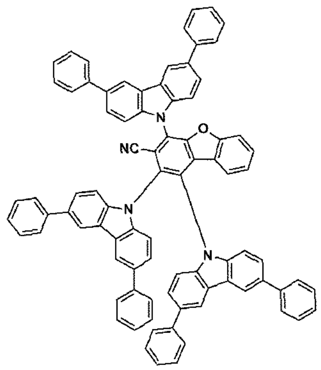
125

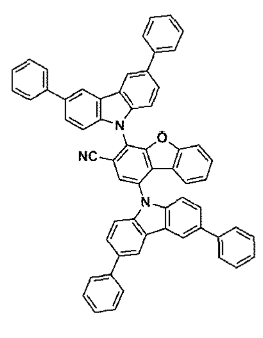
126

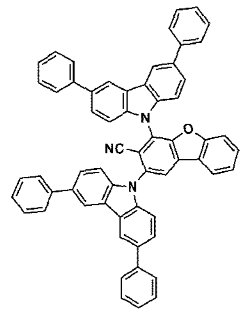
127

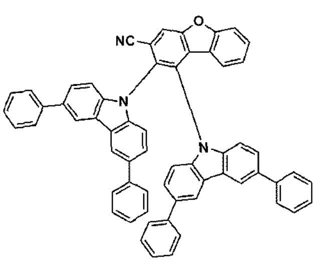
128

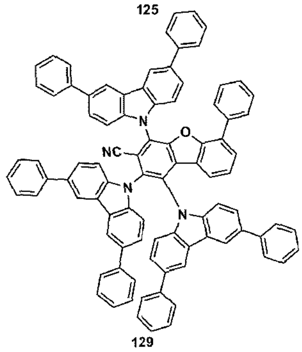
129

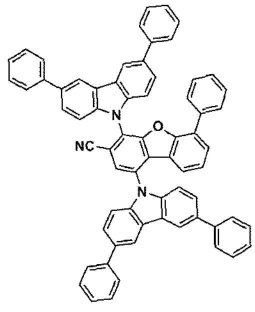
130

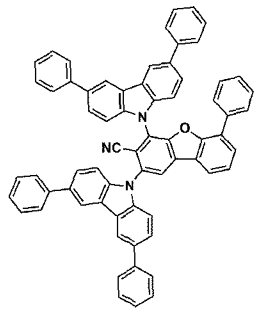
131

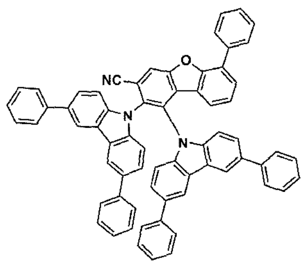
132

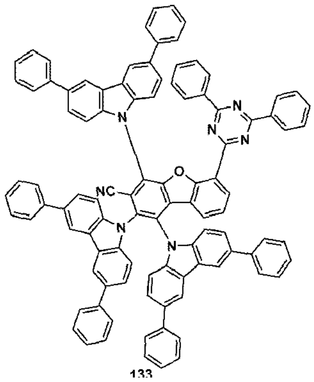
133

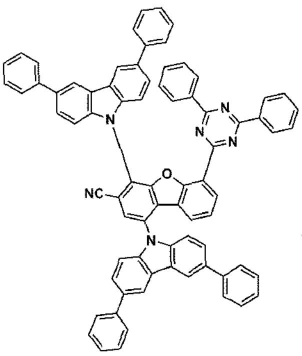
134

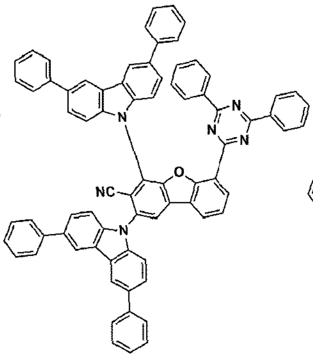
135

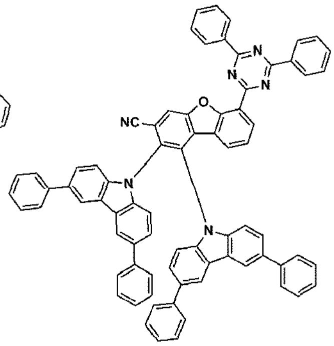
136

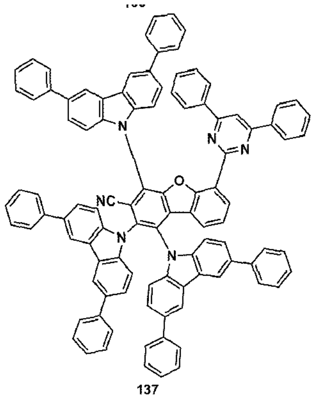
137

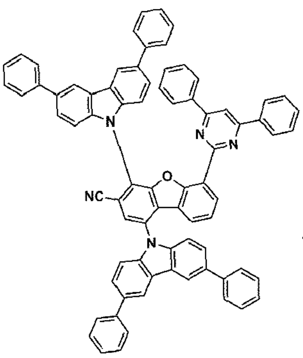
138

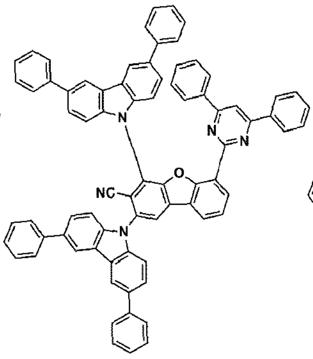
139

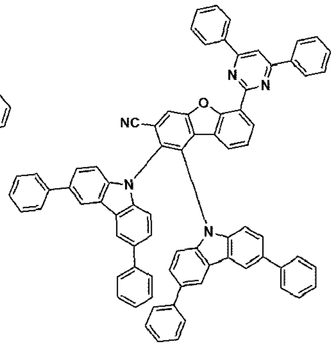
140

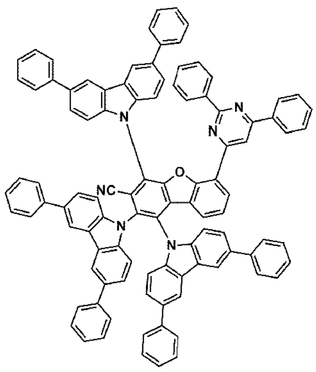
141

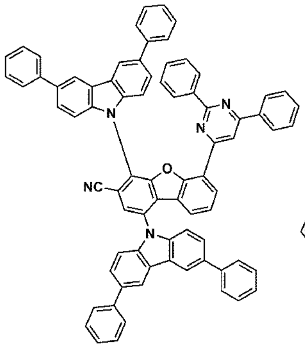
142

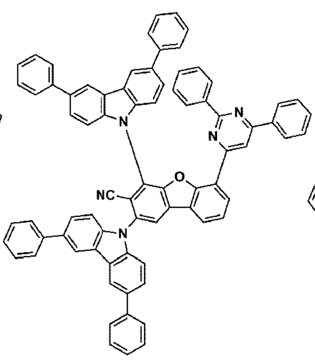
143

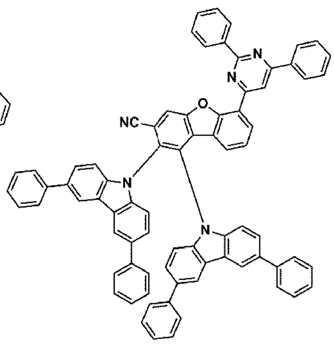
144

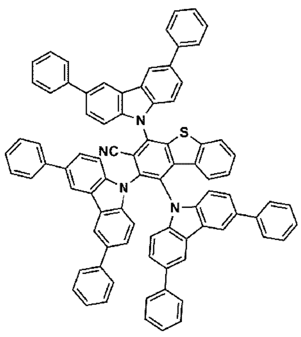
145

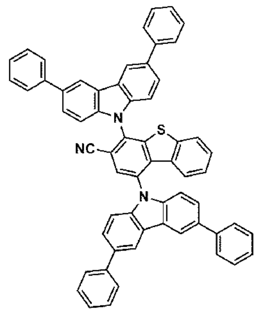
146

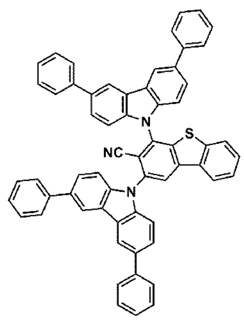
147

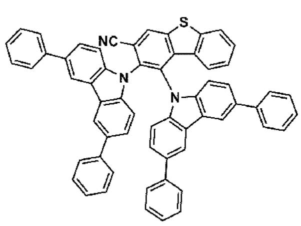
148

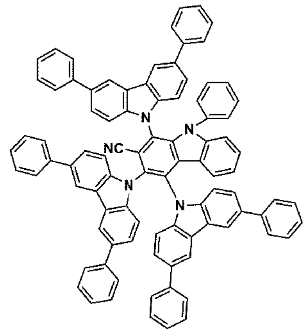

149

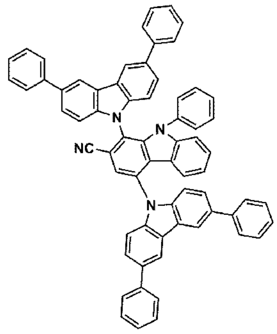

150

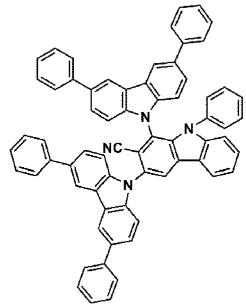

151

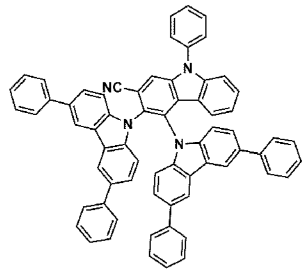

152

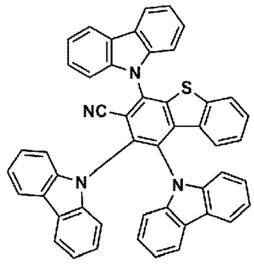

153

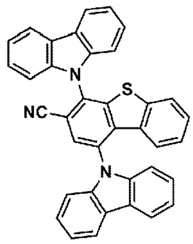

154

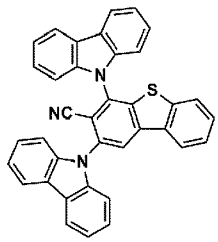

155

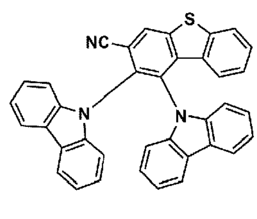

156

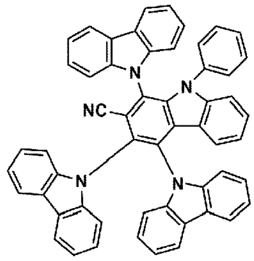

157

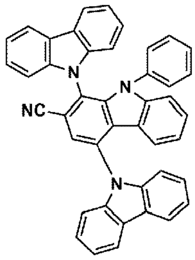

158

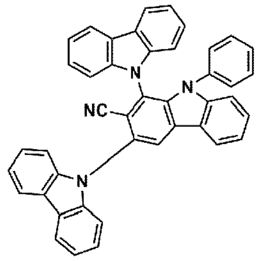

159

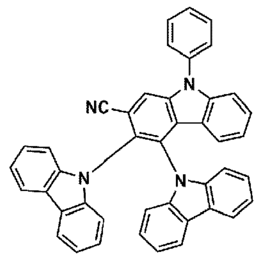

160

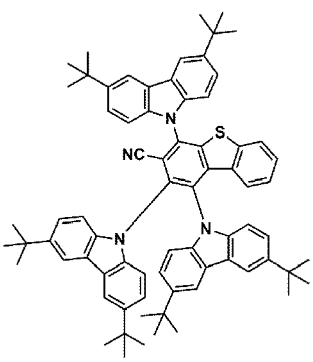

161

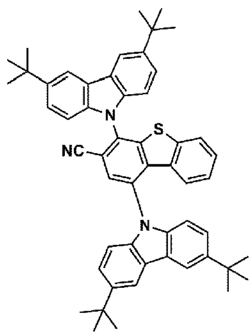

162

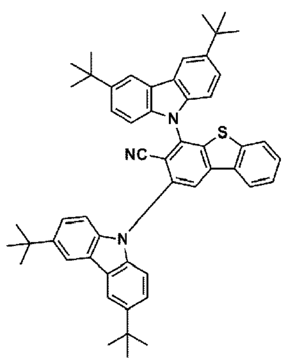

163

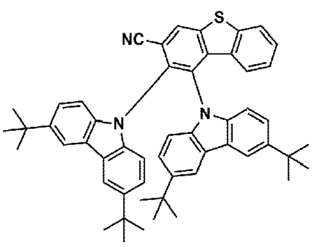

164

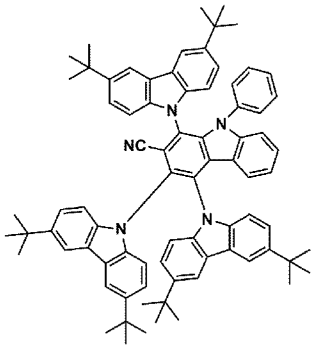
165

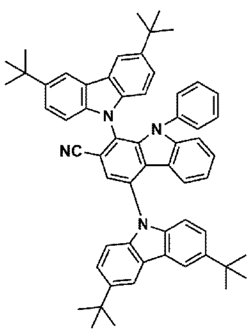
166

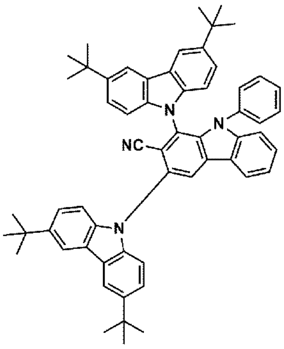
167

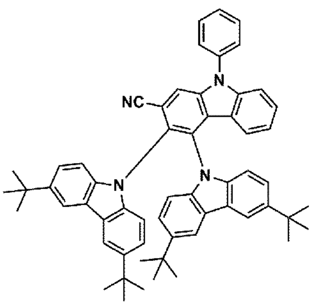
168

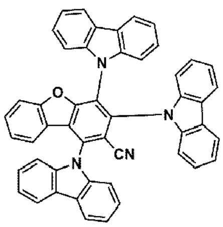
169

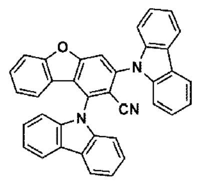
170

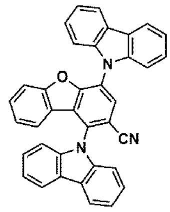
171

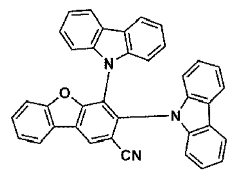
172

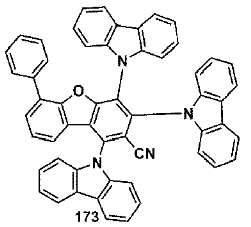
173

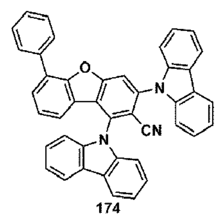
174

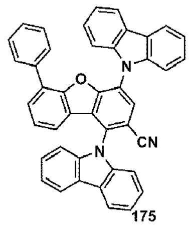
175

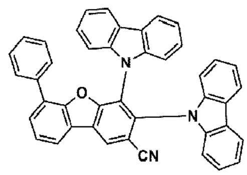
176

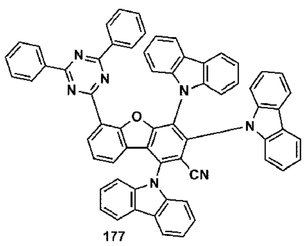
177

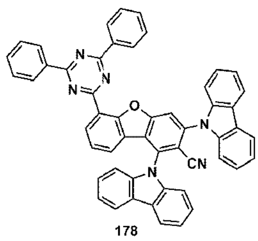
178

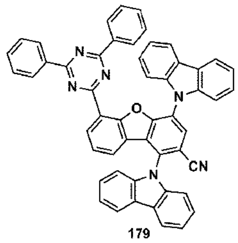
179

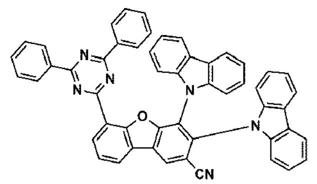
180

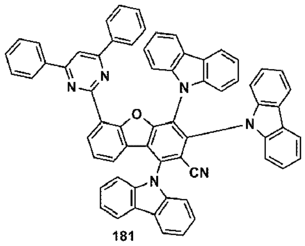
181

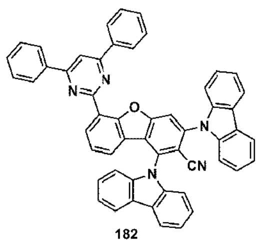
182

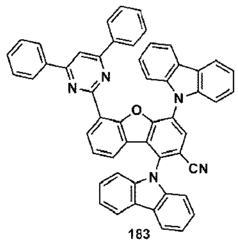
183

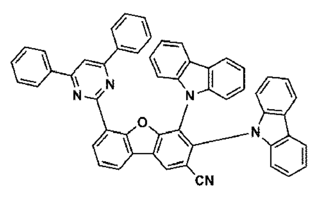
184

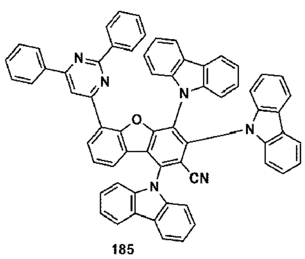
185

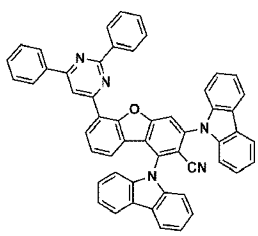
186

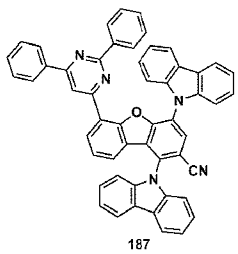
187

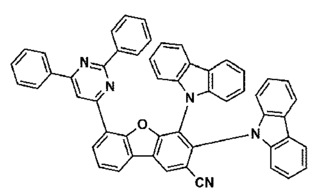
188

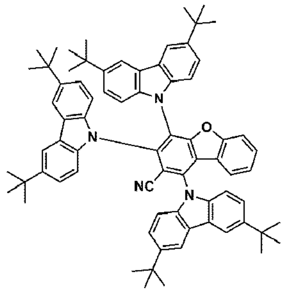
189

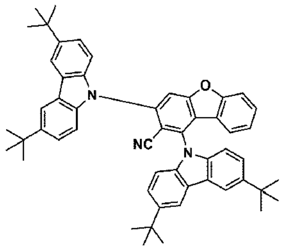
190

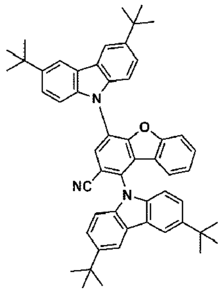
191

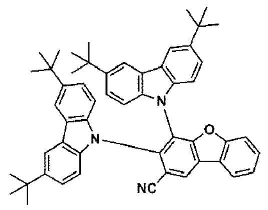
192

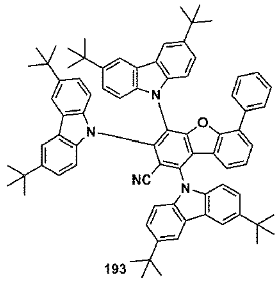
193

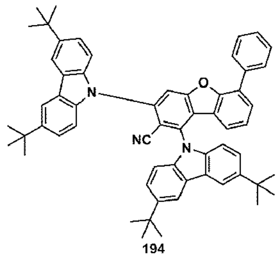
194

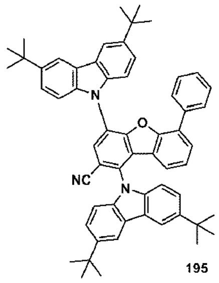
195

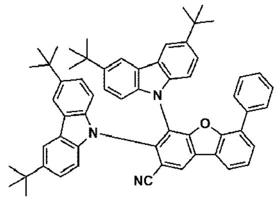
196

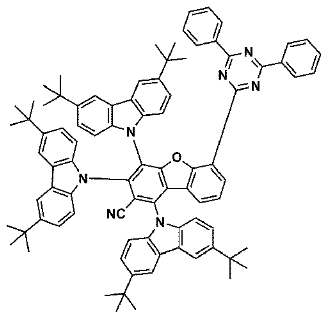
197

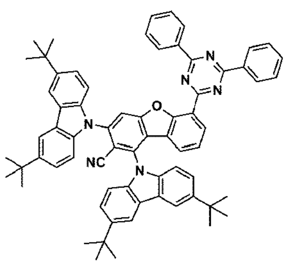
198

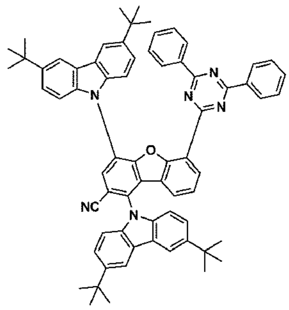
199

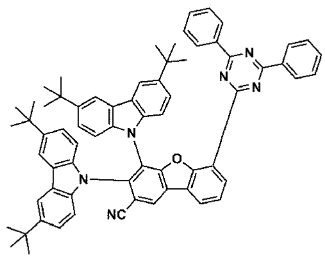
200

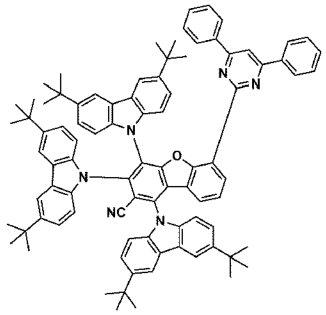
201

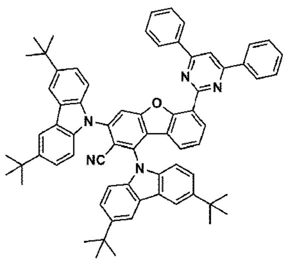
202

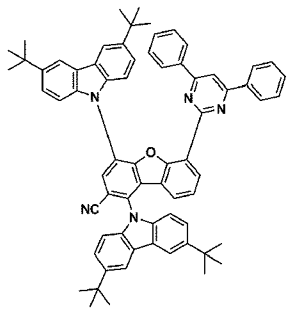
203

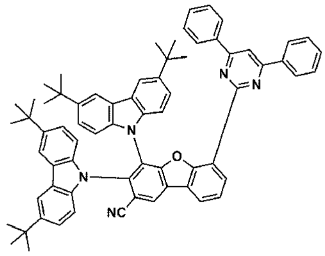
204

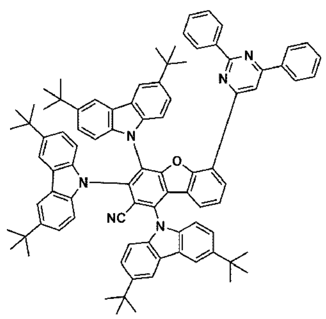
205

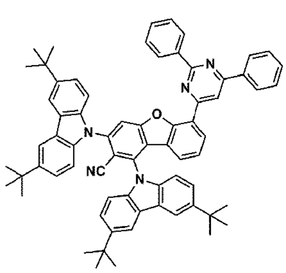
206

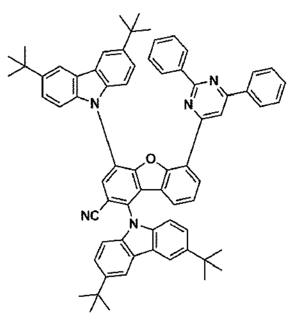
207

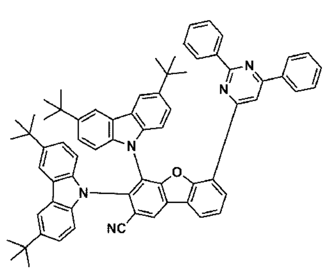
208

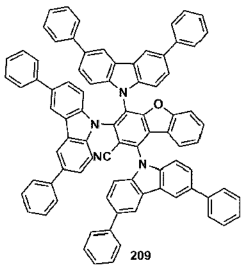
209

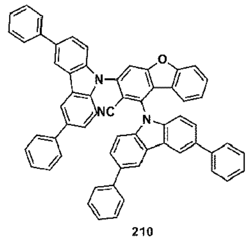
210

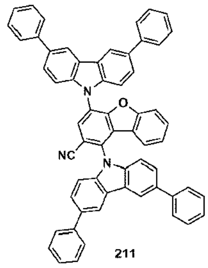
211

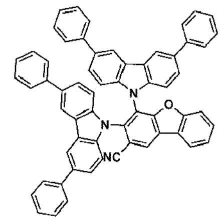
212

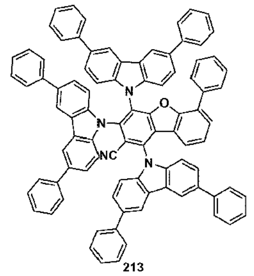
213

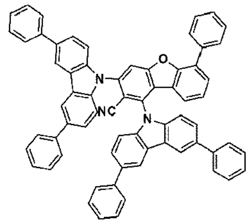
214

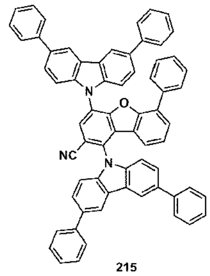
215

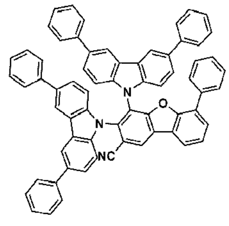
216

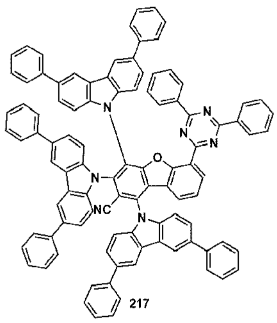
217

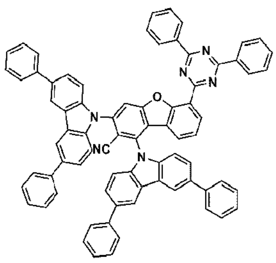
218

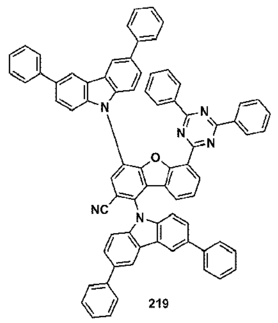
219

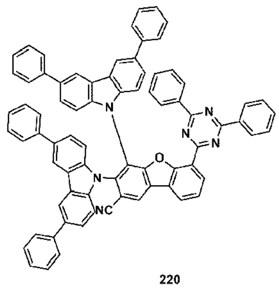
220

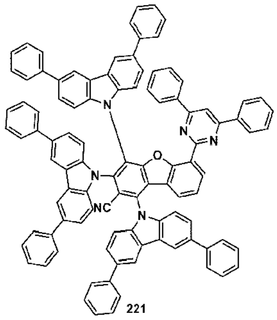
221

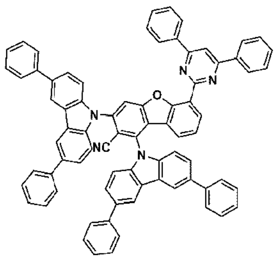
222

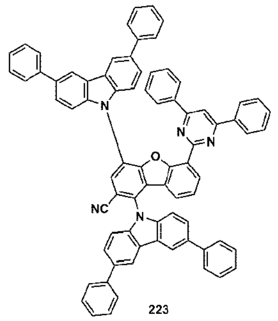
223

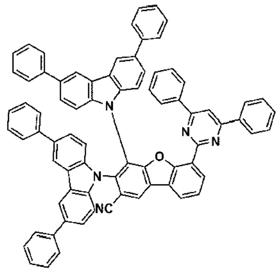
224

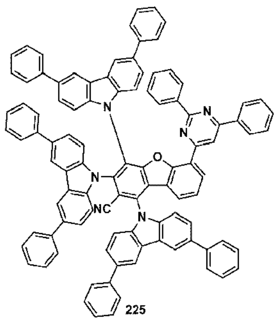
225

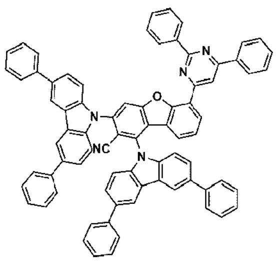
226

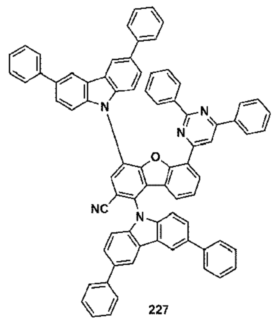
227

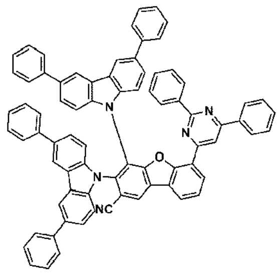
228

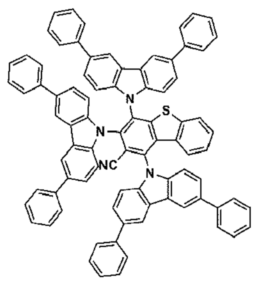

229

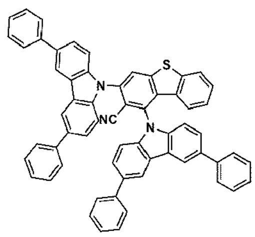

230

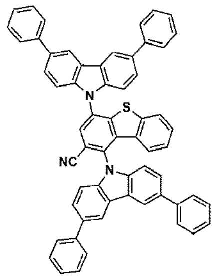

231

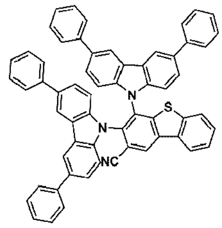

232

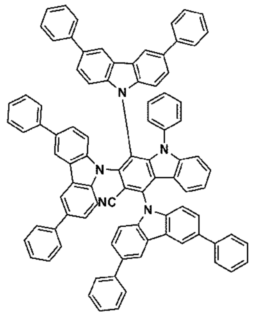

233

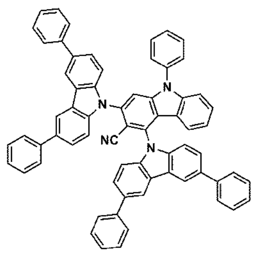

234

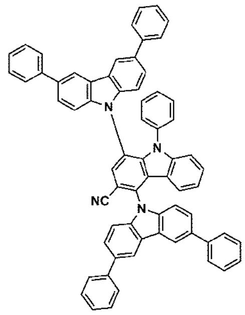

235

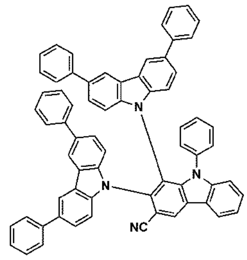

236

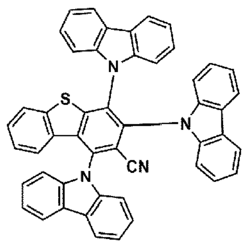

237

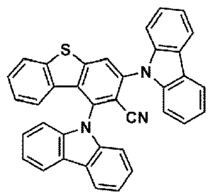

238

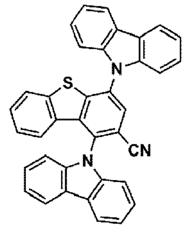

239

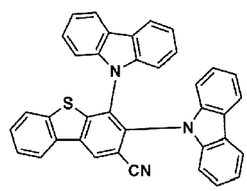

240

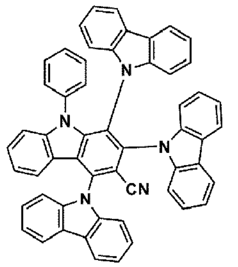

241

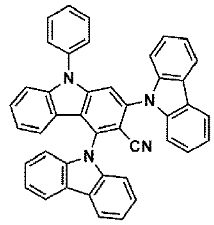

242

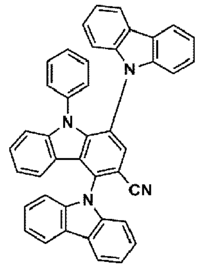

243

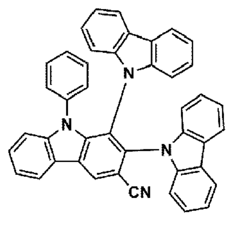

244

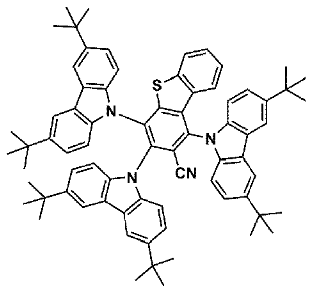

245

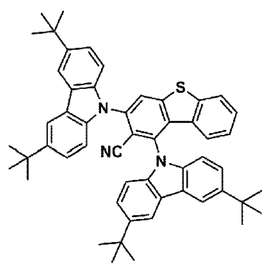

246

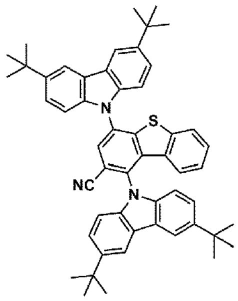

247

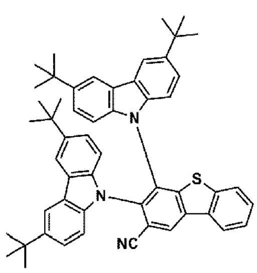

248

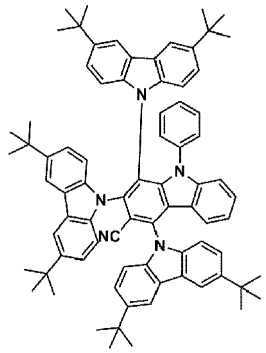

249

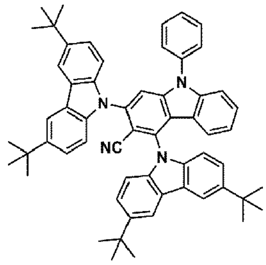

250

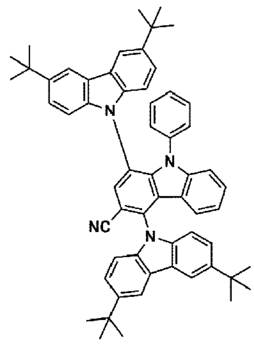

251

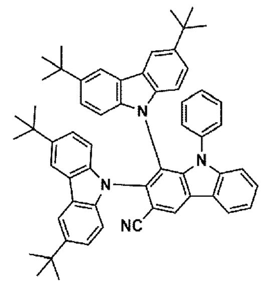

252

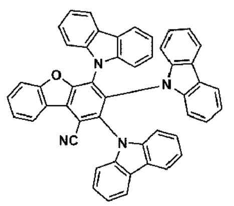

253

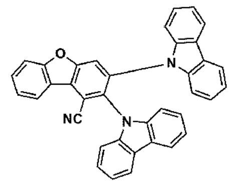

254

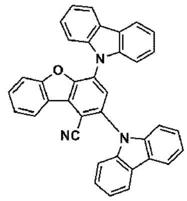

255

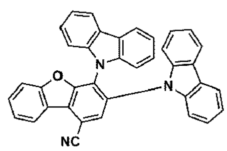

256

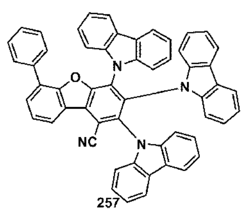

257

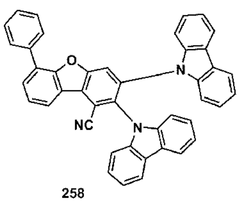

258

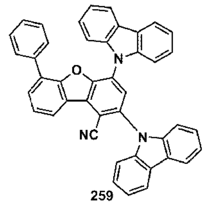

259

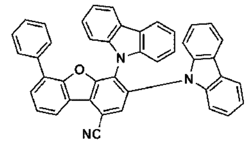

260

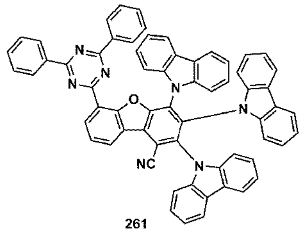

261

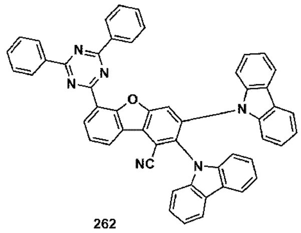

262

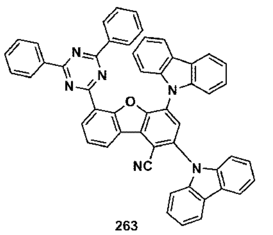

263

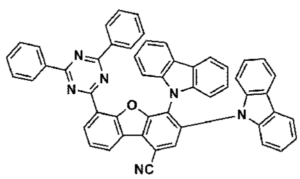

264

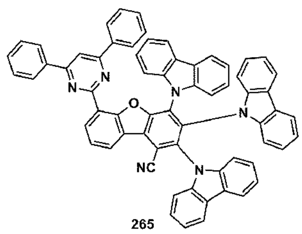

265

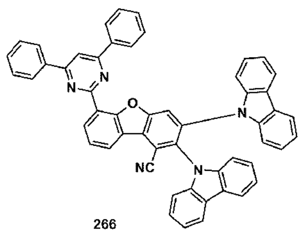

266

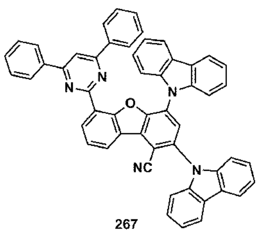

267

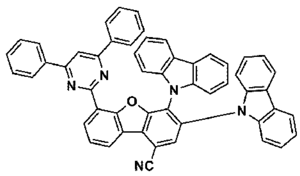

268

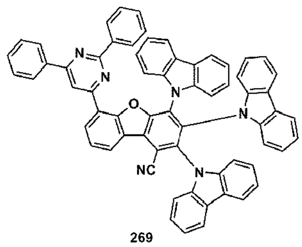

269

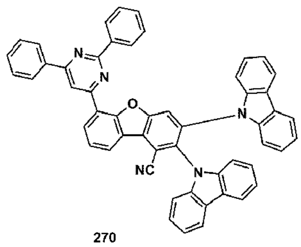

270

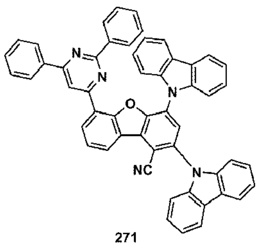

271

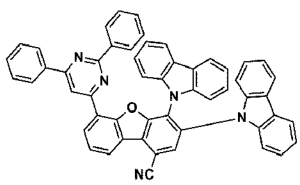

272

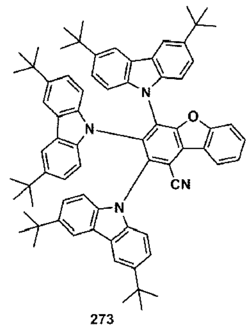
273

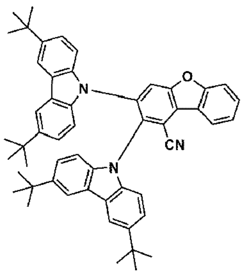
274

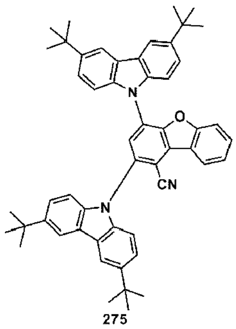
275

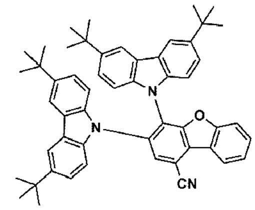
276

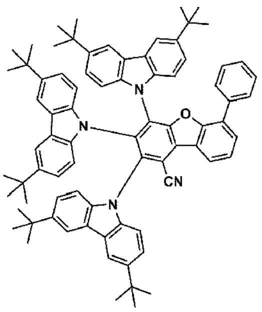
277

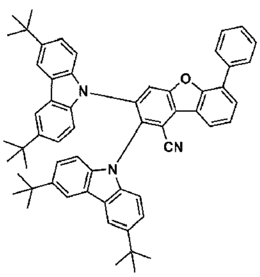
278

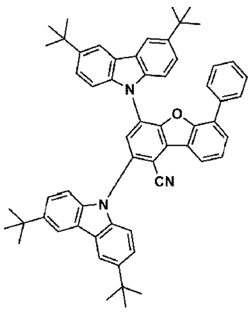
279

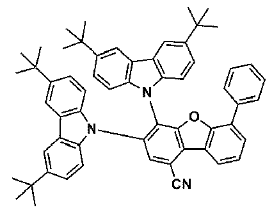
280

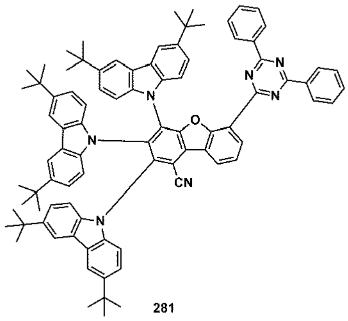
281

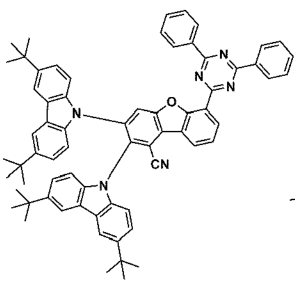
282

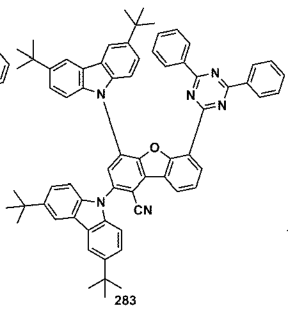
283

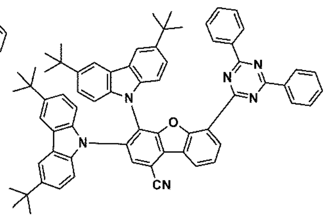
284

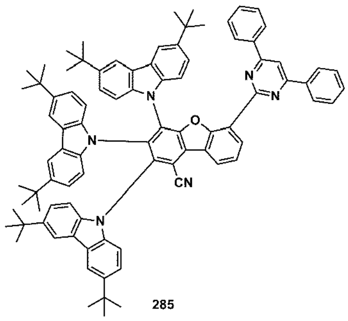
285

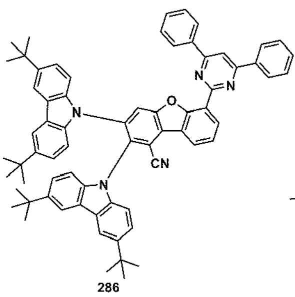
286

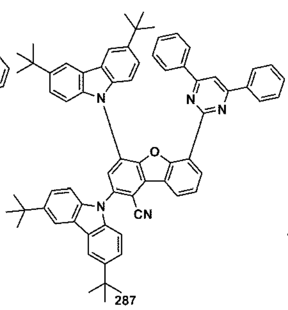
287

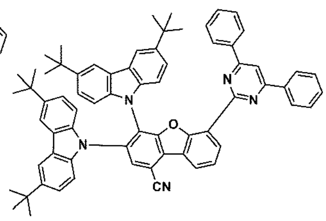
288

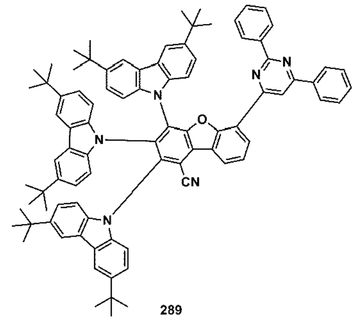
289

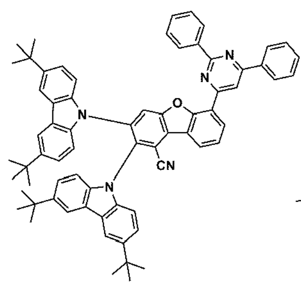
290

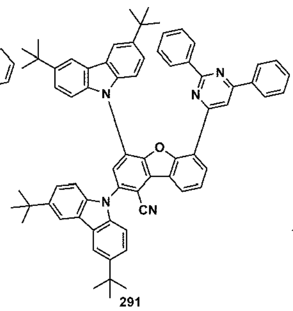
291

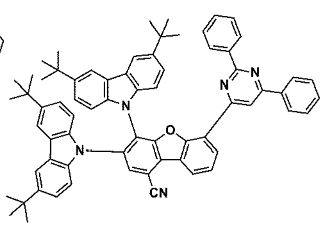
292

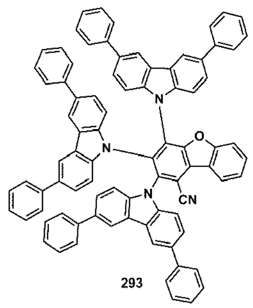
293

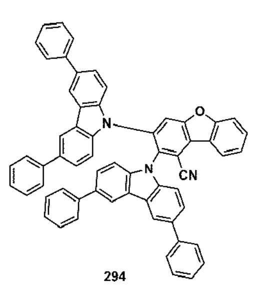
294

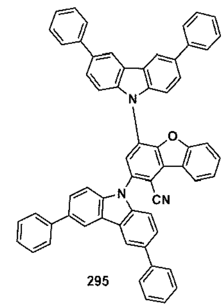
295

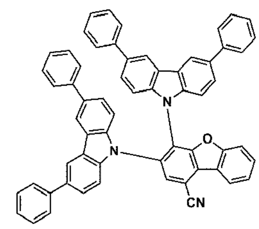
296

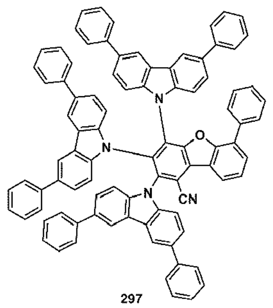
297

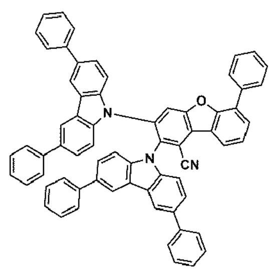
298

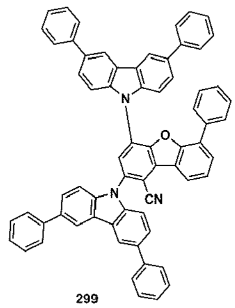
299

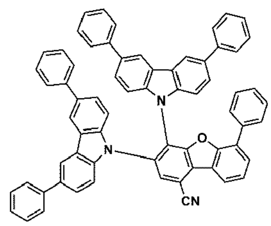
300

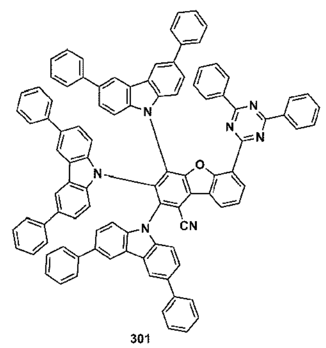
301

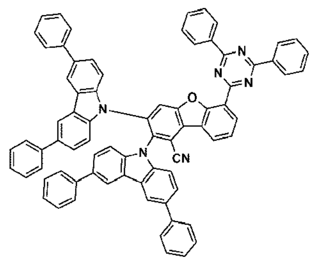
302

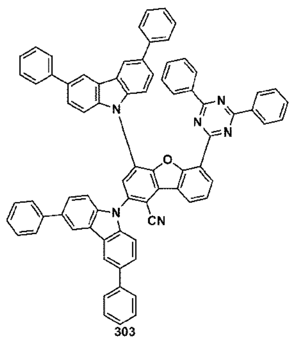
303

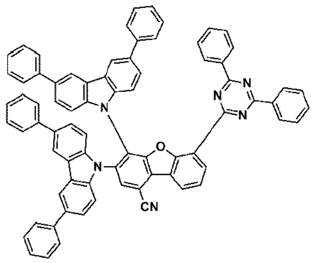
304

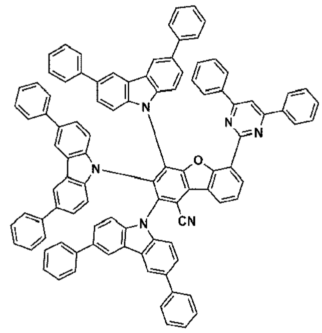

305

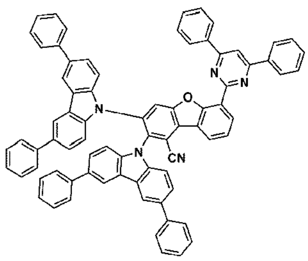

306

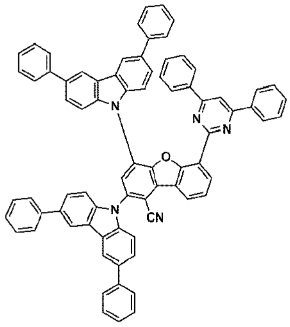

307

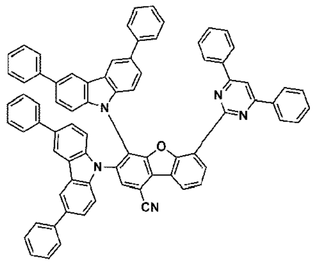

308

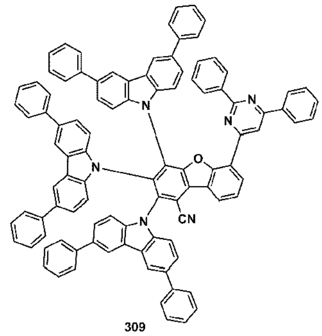

309

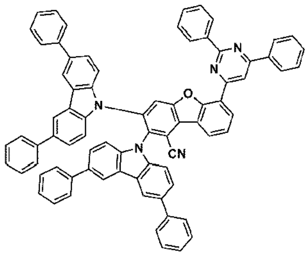

310

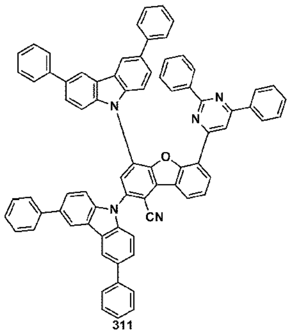

311

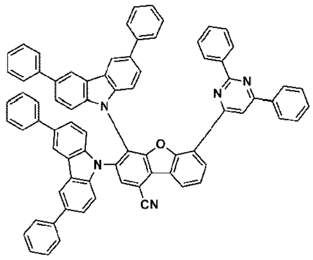

312

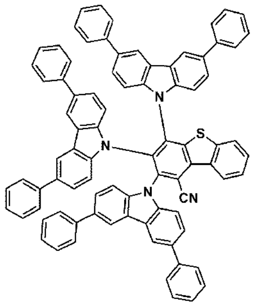

313

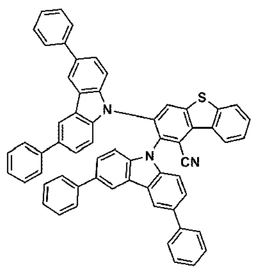

314

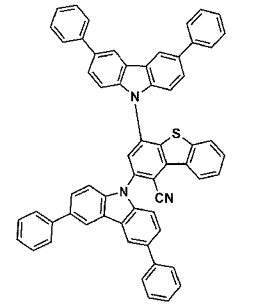

315

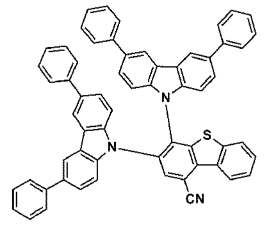

316

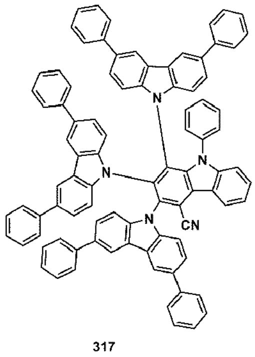

317

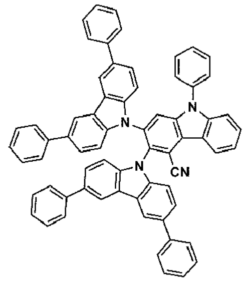

318

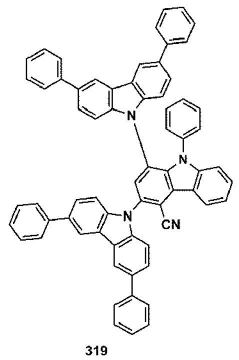

319

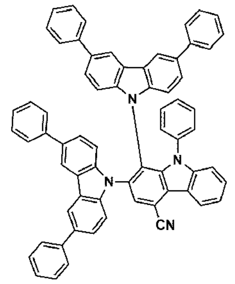

320

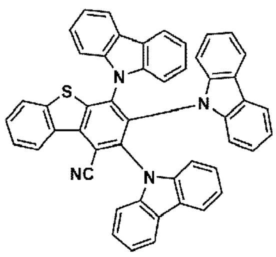

321

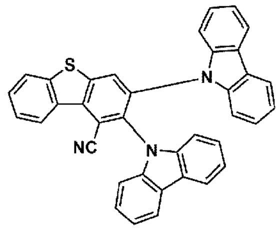

322

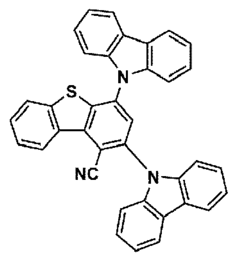

323

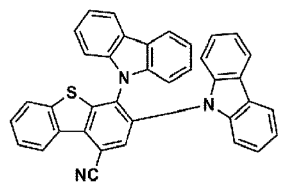

324

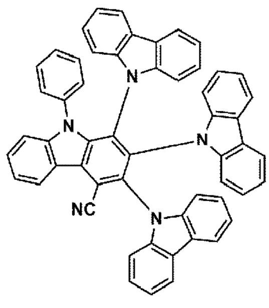

325

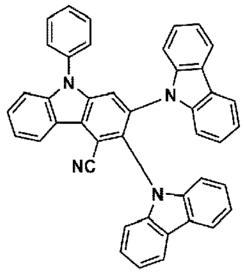

326

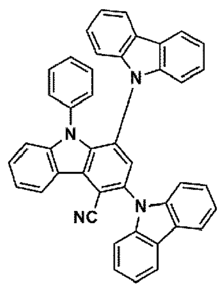

327

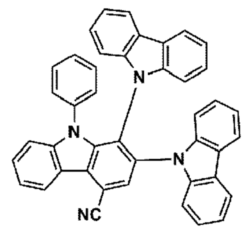

328

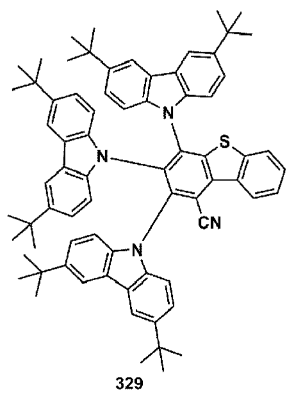

329

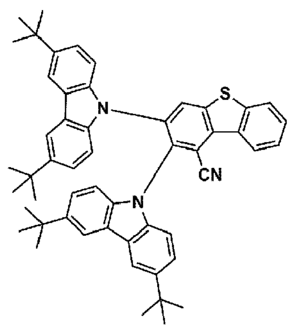

330

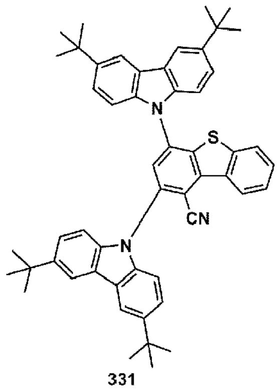

331

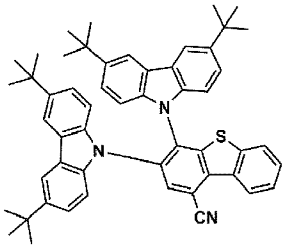

332

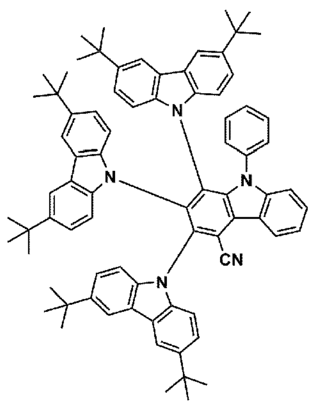

333

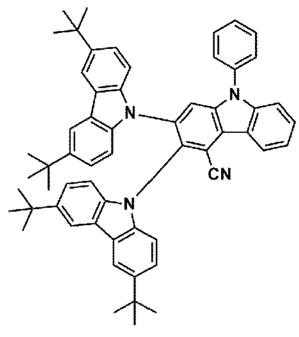

334

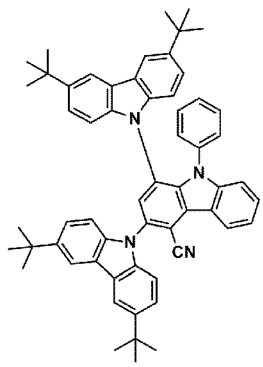

335

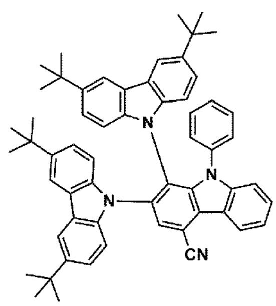

336

The condensed-cyclic compound represented by Formula 1 has CN, which acts as an electron-withdrawing group, and two or more groups represented by one of Formulae 2-1 and 2-2, which act as electron-donating groups in the same ring. Thus, since the electron-donating groups electronically shield the electron-withdrawing group, the stability of the condensed-cyclic compound may be improved. Accordingly, an organic light-emitting device with high efficiency and/ or a long lifespan may be provided.

In detail, when two electron-donating groups are not located adjacent to an electron-withdrawing group, optical

characteristics may be degraded. However, since the condensed-cyclic compound represented by Formula 1 has two electron-donating groups and an electron withdrawing group located adjacent thereto, CN is electrically shielded, which leads to an increase in delayed fluorescent characteristics.

The condensed-cyclic compound represented by Formula 1 uses a cyano group as an electron-withdrawing group. Accordingly, the condensed-cyclic compound may have increased thermal stability. Thus, an organic light-emitting device including the condensed-cyclic compound may have a longer lifespan.

In the case of the condensed-cyclic compound represented by Formula 1, an emission wavelength and an energy level thereof may be easily controlled by changing the number of the electron-donating groups and the electron withdrawing groups or the structure of the electron-donating group.

The condensed-cyclic compound represented by Formula 1 may have a narrow full width half-width. Accordingly, an organic light-emitting device including the condensed-cyclic compound may have high efficiency and high color purity.

Regarding the condensed-cyclic compound represented by Formula 1, a highest occupied molecular orbital (HOMO) and a lowest unoccupied molecular orbital (LUMO) may be spatially separated, and therefore, $\triangle E_{ST}$ (herein, $\triangle E_{ST}$ is the difference between a lowest excited singlet energy level ($E_{S1}$) and a lowest excited triplet energy level ($E_{T1}$)) is reduced. Therefore, the condensed-cyclic compound represented by Formula 1 may experience reverse intersystem crossing even at low temperatures (for example, room temperature). Electronic devices, for example, organic light-emitting devices, including the condensed-cyclic compound may have high efficiency and/or a long lifespan.

The condensed-cyclic compound represented by Formula 1 may satisfy Equation 1:

Equation 1

$$0 \text{ eV} < \triangle E_{ST} \leq 0.5 \text{ eV}$$

In Equation 1,

$\triangle E_{ST}$ is the difference between the lowest excited singlet energy level ($E_{S1}$) of the condensed-cyclic compound represented by Formula 1 and the lowest excited triplet energy level ($E_{T1}$) of the condensed-cyclic compound represented by Formula 1. $E_{T1}$ and $E_{S1}$ are evaluated by using a DFT method of Gaussian program that is structurally optimized at the level of B3LYP/6-31 G(d,p).

In detail, the condensed-cyclic compound represented by Formula 1 may satisfy the following Equation 1-1, but is not limited to:

Equation 1-1

$$\text{eV} < \triangle E_{ST} \leq 0.3 \text{ eV}$$

The lowest excited singlet energy level of the condensed-cyclic compound represented by Formula 1 may be greater than 2.5 eV and less than 3.0 eV, but embodiments are not limited thereto.

For example, the HOMO, LUMO, $T_1$ energy level, $S_1$ energy level, photoluminescence (PL) maximum emission wavelength and oscillator intensity of some of the compounds were evaluated by using a DFT method of Gaussian program (optimized at the level of B3LYP, 6-31G(d,p)), and the obtained results are shown in Table 1.

Table 1

| Compound No. | HOMO (eV) | LUMO (eV) | $S_1$ (eV) | $T_1$ (eV) | $S_1$-$T_1$ (eV) | The maximum emission wavelength (nm) of the PL spectrum | Oscillator strength (f) |
|---|---|---|---|---|---|---|---|
| 5 | -5.5 | -2.2 | 2.711 | 2.593 | 0.118 | 456 | 0.0579 |
| 6 | -5.588 | -2.056 | 2.95 | 2.607 | 0.343 | 429 | 0.1221 |
| 7 | -5.576 | -2.057 | 2.94 | 2.723 | 0.217 | 430 | 0.0755 |
| 85 | -5.532 | -2.22 | 2.725 | 2.613 | 0.112 | 453 | 0.0053 |
| 87 | -5.565 | -2.146 | 2.822 | 2.692 | 0.131 | 436 | 0.0033 |
| 89 | -5.5 | -2.2 | 2.711 | 2.593 | 0.118 | 456 | 0.0467 |
| 91 | -5.533 | -2.137 | 2.801 | 2.688 | 0.114 | 435 | 0.0350 |

(continued)

| Compound No. | HOMO (eV) | LUMO (eV) | $S_1$ (eV) | $T_1$ (eV) | $S_1$-$T_1$ (eV) | The maximum emission wavelength (nm) of the PL spectrum | Oscillator strength (f) |
|---|---|---|---|---|---|---|---|
| 95 | -5.54 | -2.316 | 2.694 | 2.602 | 0.093 | 446 | 0.0327 |
| 127 | -5.373 | -2.225 | 2.619 | 2.526 | 0.093 | 466 | 0.0054 |
| 131 | -5.358 | -2.2 | 2.624 | 2.539 | 0.085 | 461 | 0.0523 |
| A | -5.569 | -2.128 | 2.941 | 2.747 | 0.194 | 416 | 0.00125 |
| B | -5.572 | -1.856 | 3.165 | 2.946 | 0.22 | 379 | 0.0285 |

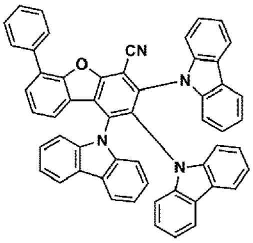
5

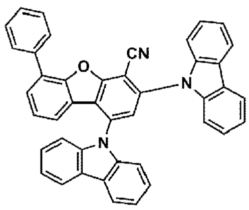
6

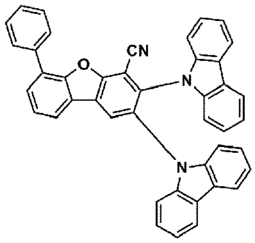
7

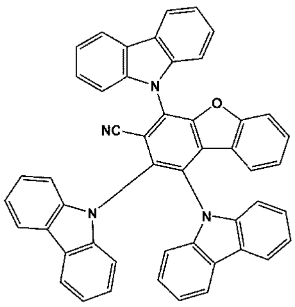
85

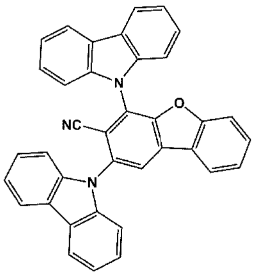
87

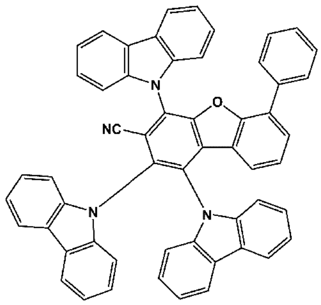
89

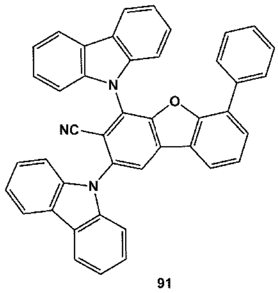
91

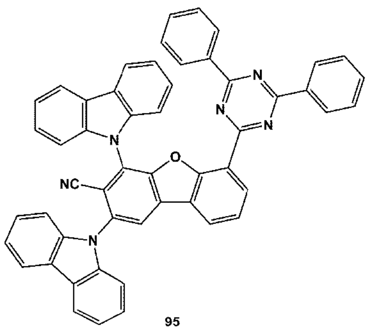
95

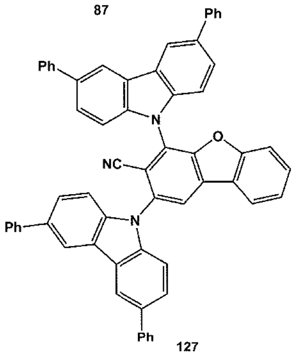
127

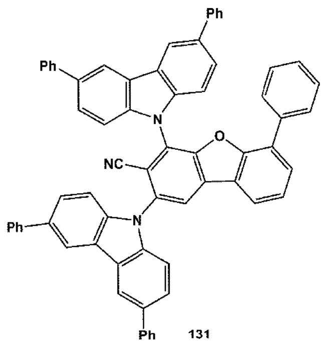
131

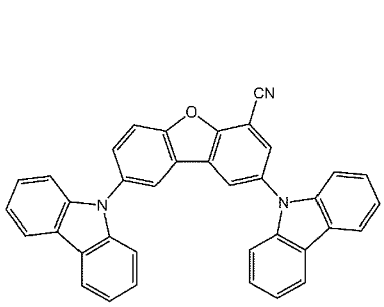
A

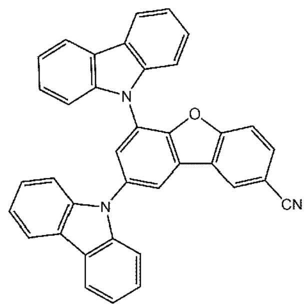
B

From Table 1, it can be seen that the compound represented by Formula 1 has a relatively small difference between a singlet energy level and a triplet energy level and large oscillator intensities. Therefore, it can be seen that electronic devices, for example, organic light-emitting devices, employing the compound represented by Formula 1 have high luminous efficiency.

Synthesis methods of the condensed-cyclic compound represented by Formula 1 may be understood by one of ordinary skill in the art by referring to Synthesis Examples provided below.

The condensed-cyclic compound represented by Formula 1 may be used as a material for electronic devices, for example, organic light-emitting devices. According to one or more embodiments, an organic light-emitting device includes a first electrode; a second electrode; and an organic layer including an emission layer between the first electrode and the second electrode, wherein the organic layer includes the condensed-cyclic compound represented by Formula 1.

When an organic layer including the condensed-cyclic compound represented by Formula 1 is included in an organic light-emitting device, the obtained organic light-emitting device may have low driving voltage, high efficiency, high luminance, high quantum luminous efficiency, and/or long lifespan.

The condensed-cyclic compound of Formula 1 may be used between a pair of electrodes of an organic light-emitting device. For example, the condensed-cyclic compound may be included in an emission layer, a hole transport region between a first electrode and an emission layer (for example, the hole transport region includes a hole injection layer, a hole transport layer, an electron blocking layer, or a combination thereof), an electron transport region between the emission layer and the second electrode (for example, the electron transport region includes a hole blocking layer, an electron transport layer, an electron injection layer, or a combination thereof), or a combination thereof.

Depending on the use of the condensed-cyclic compound represented by Formula 1, the emission layer of the organic light-emitting device may be embodied according to a first embodiment, a second embodiment, or a third embodiment.

First Embodiment

The first embodiment is an embodiment in which the condensed-cyclic compound included in the emission layer is used as a fluorescent emitter, that is, the condensed-cyclic compound is a fluorescent emitter.

According to the first embodiment, the emission layer may include the condensed-cyclic compound alone; or the emission layer may further include a host (hereinafter referred to as 'host A', wherein the host A is not the same as the condensed-cyclic compound).

Accordingly, according to the first embodiment, the ratio of an emission component emitted from the condensed-cyclic compound to the total emission components emitted from the emission layer may be 80% or more, for example, 90% or more. For example, the ratio of the emission component emitted from the condensed-cyclic compound to the total emission components emitted from the emission layer may be 95% or more. Herein, the condensed-cyclic compound emits fluorescence and/or delayed fluorescent light, and the emission component of the condensed-cyclic compound may be the sum of prompt emission component of the condensed-cyclic compound and delayed fluorescence component due to reverse intersystem crossing of the condensed-cyclic compound.

In the first embodiment, when the emission layer further includes host A in addition to the condensed-cyclic compound, the amount of the condensed-cyclic compound may be, based on 100 parts by weight of the emission layer, 50 parts by weight or less, for example, 30 parts by weight or less, and the amount of host A in the emission layer may be, based on 100 parts by weight of the emission layer, 50 parts by weight or more, for example, 70 parts by weight or more, but embodiments are not limited thereto.

In the first embodiment, when the emission layer further includes the host A in addition to the condensed-cyclic compound, the host A and the condensed-cyclic compound represented by Formula 1 may satisfy Equation 2:

Equation 2

$$E(H_A)_{S1} > E_{S1}$$

wherein, in Equation 2,

$E(H_A)_{S1}$ is the lowest excitation singlet energy level of the host A;

$E_{S1}$ is the lowest excitation singlet energy level of the condensed-cyclic compound represented by Formula 1. $E(H_A)_{S1}$ and $E_{S1}$ are evaluated by using a DFT method of Gaussian program that is structurally optimized at the level of B3LYP/6-31G(d,p).

When the condensed-cyclic compound represented by Formula 1 satisfies Equation 1 and the condensed-cyclic compound represented by Formula 1 and the host A satisfy Equation 2, the condensed-cyclic compound represented by Formula 1 may emit fluorescent light and/or delayed fluorescent light. Therefore, the luminous efficiency of an organic light-emitting device including the condensed-cyclic compound represented by Formula 1 and the host A may be increased.

For example, the host A may be a host material to be described later, but embodiments are not limited thereto.

Second Embodiment

The second embodiment is an embodiment in which the condensed-cyclic compound contained in the emission layer is used as a host.

According to the second embodiment, the emission layer includes a host and a dopant, and the host may include the condensed-cyclic compound represented by Formula 1. That is, the host may consist of the condensed-cyclic compound represented by Formula 1 alone, or may include other known hosts. The dopant may be, for example, a fluorescent dopant, a phosphorescent dopant, or a thermal activation delayed fluorescent dopant.

Therefore, according to the second embodiment, the ratio of the light of the dopant in the total emission components emitted from the emission layer may be 80% or more, for example, 90% or more (as another example, 95% or more).

In the second embodiment, the amount of dopant in the emission layer may be, based on 100 parts by weight of the emission layer, 50 parts by weight or less, for example, 30 parts by weight or less, and the amount of the host in the emission layer may be, based on 100 parts by weight of the emission layer, 50 parts by weight or more, for example, 70 parts by weight or more, but embodiments are not limited thereto.

For example, in the second embodiment, when the dopant is a fluorescent dopant (hereinafter 'fluorescent dopant A'), the condensed-cyclic compound represented by Formula 1 and the fluorescent dopant A may satisfy Equation 3:

Equation 3

$$E_{S1} > E(F_A)_{S1}$$

wherein, in Equation 3,

$E_{S1}$ is the lowest excitation singlet energy level of the condensed-cyclic compound represented by Formula 1; and $E(F_A)_{S1}$ is the lowest excitation singlet energy level of the fluorescent dopant A.

$E_{S1}$ and $E(F_A)_{S1}$ are evaluated by using a DFT method of Gaussian program that is structurally optimized at the level of B3LYP/6-31G(d,p).

When the condensed-cyclic compound represented by Formula 1 and the fluorescent dopant A satisfy Equation 3, Forster energy transfer from the condensed-cyclic compound represented by Formula 1 to the fluorescent dopant A may be promoted. Therefore, the luminous efficiency of an organic light-emitting device including the condensed-cyclic compound represented by Formula 1 and the fluorescent dopant A may be increased.

For example, the dopant may be a dopant material to be described later, but embodiments are not limited thereto.

When the host contains other known hosts, the other known hosts may be the host materials described below, but not necessarily limited.

Third Embodiment

The third embodiment is an embodiment in which the condensed-cyclic compound contained in the emission layer is used as an auxiliary dopant.

According to the third embodiment, the emission layer includes a host, an auxiliary dopant, and a dopant, and the auxiliary dopant may include the condensed-cyclic compound. The dopant may be, for example, a fluorescent dopant, a phosphorescent dopant, or a thermal activation delayed fluorescent dopant.

Therefore, according to the third embodiment, the ratio of the light emission component of dopant in the total light emission components emitted from the emission layer may be 80% or more, for example, 90% or more (as another example, 95% or more).

In the third embodiment, the amount of dopant in the emission layer may be, based on 100 parts by weight of the emission layer, 50 parts by weight or less, for example, 30 parts by weight or less, the amount of the host in the emission layer may be, based on 100 parts by weight of the emission layer, 50 parts by weight or more, for example,

70 parts by weight or more, and the amount of the auxiliary dopant may be, based on 100 parts by weight of the emission layer, 30 parts by weight or less, for example, 20 parts by weight or less, but embodiments are not limited thereto.

For example, in the third embodiment, when the dopant is a fluorescent dopant (hereinafter, referred to as 'fluorescent dopant B'), the host (hereinafter referred to as 'host B), the condensed-cyclic compound represented by Formula 1 and the fluorescent dopant B may satisfy Equation 4:

Equation 4

$$E(H_B)_{S1} > E_{S1} > E(F_B)_{S1}$$

wherein, in Equation 4,

$E(H_B)_{S1}$ is the lowest excitation singlet energy level of the host B;
$E_{S1}$ is the lowest excitation singlet energy level of the condensed-cyclic compound represented by Formula 1; and
$E(F_B)_{S1}$ is the lowest excitation singlet energy level of the fluorescent dopant B.
$E(H_B)_{S1}$, $E_{S1}$, and $E(F_B)_{S1}$ are evaluated by using a DFT method of Gaussian program that is structurally optimized at the level of B3LYP/6-31G(d,p).

When the host B, the condensed-cyclic compound represented by Formula 1 and the fluorescent dopant B satisfy Equation 4, Forster energy transfer from the condensed-cyclic compound represented by Formula 1 to the fluorescent dopant B may be promoted. Therefore, the luminous efficiency of an organic light-emitting device including host B, the condensed-cyclic compound represented by Formula 1 and the fluorescent dopant B may be increased.

The host B and the condensed-cyclic compound represented by Formula 1 may satisfy Equation 5:

Equation 5

$$E(H_B)_{T1} - E_{T1} > 0.05 \text{ eV}$$

wherein, in Equation 5,

$E(H_B)_{S1}$ is the lowest excitation triplet energy level of the host B; and
$E_{T1}$ is the lowest excitation triplet energy level of the condensed-cyclic compound represented by Formula 1.
$E(H_B)_{T1}$ and $E_{T1}$ are evaluated by using a DFT method of Gaussian program that is structurally optimized at the level of B3LYP/6-31G(d,p).

In the third embodiment, due to the satisfaction of Equation 5 (for example, $E(H_B)_{T1}$ - $E_{T1}$ is 0.10 eV or more and 0.65 eV or less), the energy of the triplet exciton generated in the auxiliary dopant in the emission layer may not be transferred to the host B in the emission layer. Accordingly, the probability of the triplet exciton being lost in a path other than the light emission path is reduced. As a result, the organic light-emitting device may have high efficiency.

The condensed-cyclic compound represented by Formula 1 and the fluorescent dopant B may satisfy Equation 6:

Equation 6

$$E(F_B)_{S1} - E_{S1} < 0 \text{ eV}$$

wherein, in Equation 6,

$E(F_B)_{S1}$ is the lowest excitation singlet energy level of the fluorescent dopant, and
$E_{S1}$ is the lowest excitation triplet energy level of the condensed-cyclic compound represented by Formula 1.
$E(F_B)_{S1}$ and $E_{S1}$ are evaluated by using a DFT method of Gaussian program that is structurally optimized at the level of B3LYP/6-31G(d,p).

In the third embodiment, due to the satisfaction of Equation 6 (for example, $E_{S1(FD)}$ - $E_{S1(AD)}$ is -0.4 eV or more and -0.05 eV or less), the energy of singlet exciton generated in the auxiliary dopant in the emission layer may quickly move toward the fluorescent dopant B. Thus, substantially, only the fluorescent dopant B in the emission layer of the

organic light-emitting device emits light, thereby realizing a fluorescence emission spectrum of excellent color purity based on the fluorescent dopant B. In addition, a fluorescence emission having a relatively short exciton lifespan may occur. Accordingly, the efficiency transfer phenomenon under high luminosity (that is, a roll-off phenomenon), which may occur due to the interaction among a plurality of excitons (exciton-exciton interaction) or exciton-charge (hole or electron) interaction (exciton-polaron interaction) may be suppressed, thereby leading to the embodiment of an organic light-emitting device having high efficiency. Furthermore, since the auxiliary dopant has a short exciton lifespan, the probability in which the auxiliary dopant experiences chemical or physical deterioration when in the exciton state may be reduced. Thus, an organic light-emitting device satisfying Equation 6 has increased durability.

In the third embodiment, the host may be a host material to be described later, but embodiments are not limited thereto.

In the third embodiment, the dopant may be a dopant material to be described later, but embodiments are not limited thereto.

For example, the host may have 2.9 eV or more of triplet energy level, for example, 2.9 eV or more and 4.5 eV or less than the triplet energy level. As a result, energy transfer from the host to a fluorescent dopant, a phosphorescent dopant and/or a delayed fluorescent dopant may be effectively performed, and the organic light-emitting device may have high efficiency.

For example, the host may include a fluorene-containing compound, a carbazole-containing compound, a dibenzothiophene-containing compound, a dibenzothiophene-containing compound, an indenocarbazole-containing compound, an indolocarbazole-containing compound, a benzofurocarbazole-containing compound, a benzothienocarbazole-containing compound, an acridine-containing compound, a dihydroacridine-containing compound, a triindolobenzene-containing compound, a pyridine-containing compound, a pyrimidine-containing compound, a triazine-containing compound, a silicon-containing compound, a cyano-containing compound, a phosphine oxide-containing compound, a sulfoxide-containing compound, or a combination thereof, but embodiments are not limited thereto.

In one embodiment, the host may include a compound including a carbazole ring and a cyano group.

For example, the host may include compounds represented by Formulae 11-1 to 11-3, but embodiments are not limited thereto:

Formula 11-1 $Ar_{11}\text{-}(L_{21})_{a21}\text{-}(Ar_{12})_{c12}$

Formula 11-2

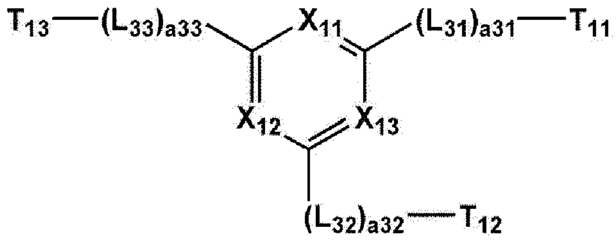

Formula 11-3 $T_{21}\text{-}(L_{21})_{a21}\text{-}(T_{22})_{c12}$

Formula 13

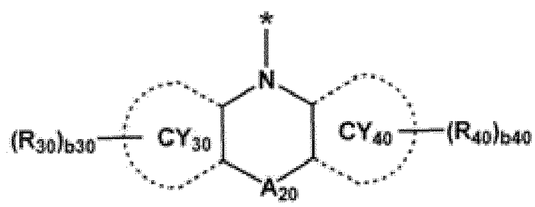

Formula 14

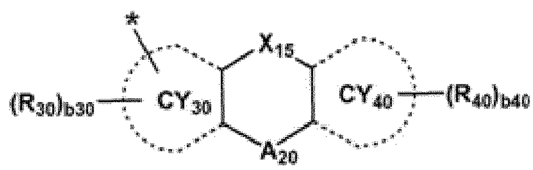

wherein, in Formulae 11-1 to 11-3, 13, and 14,

$Ar_{11}$ and $Ar_{12}$ may each independently be a group represented by one of Formulae 13 and 14,
$X_{15}$ may be $N(R_{200})$, O, or S,

$X_{11}$ may be N or $C(T_{14})$, $X_{12}$ may be N or $C(T_{15})$, and $X_{13}$ may be N or $C(T_{16})$, and $X_{11}$ to $X_{13}$ may be N,
$T_{21}$ and $T_{22}$ may each independently be $*-(L_{21})_{a21}-Si(Q_{41})(Q_{42})(Q_{43})$ or $*-(L_{21})_{a21}-P(=O)(Q_{51})(Q_{52})$,
$L_{21}$ and $L_{31}$ to $L_{33}$ may each independently be
a single bond, O, S, $Si(Q_{61})(Q_{62})$, a phenylene group, a pyridinylene group, a pyrimidinylene group, a pyrazinylene group, a pyridazinylene group, a triazinylene group, a naphthylene group, a fluorenylene group, a carbazolylene group, a dibenzofuranylene group, or a dibenzothiophenylene group; or
a phenylene group, a pyridinylene group, a pyrimidinylene group, a pyrazinylene group, a pyridazinylene group, a triazinylene group, a naphthylene group, a fluorenylene group, a carbazolylene group, a dibenzofuranylene group, or a dibenzothiophenylene group, each substituted with deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group, a $C_1$-$C_{10}$ alkoxy group, $-CF_3$, $-CF_2H$, $-CFH_2$, a phenyl group, a phenyl group substituted with a cyano group, a biphenyl group, a terphenyl group, a naphthyl group, a pyridinyl group, a pyrimidinyl group, a pyrazinyl group, a pyridazinyl group, a triazinyl group, a fluorenyl group, a carbazolyl group, a dibenzofuranyl group, a dibenzothiophenyl group, $-Si(Q_{71})(Q_{72})(Q_{73})$, or a combination thereof;
a21 and a31 to a33 may each independently be an integer from 0 to 5; when a21 is two or more, two or more $L_{21}$(s) may be identical to or different from each other, when a31 is two or more, two or more $L_{31}$(s) may be identical to or different from each other, when a32 is two or more two or more $L_{32}$(s) may be identical to or different from each other, and when a33 is two or more two or more $L_{33}$(s) may be identical to or different from each other,
$CY_{30}$ and $CY_{40}$ may each independently be a benzene group, a naphthalene group, a fluorene group, a carbazole group, a benzocarbazole group, an indolocarbazole group, a dibenzofuran group, or a dibenzothiophene group,
$A_{20}$ may be:

a single bond, a $C_1$-$C_4$ alkylene group, or a $C_2$-$C_4$ alkenylene group; or
a $C_1$-$C_4$ alkylene group or a $C_2$-$C_4$ alkenylene group, each substituted with deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group, a $C_1$-$C_{10}$ alkoxy group, a phenyl group, a biphenyl group, a terphenyl group, a naphthyl group, a pyridinyl group, a pyrimidinyl group, a pyrazinyl group, a pyridazinyl group, a triazinyl group, a fluorenyl group, a carbazolyl group, a dibenzofuranyl group, a dibenzothiophenyl group, $-Si(Q_{81})(Q_{82})(Q_{83})$, or a combination thereof;
$T_{11}$ to $T_{16}$, $R_{200}$, $R_{30}$, and $R_{40}$ may each independently be hydrogen, deuterium, -F, -Cl, -Br, -I, a hydroxyl group, a cyano group(CN), a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted monovalent non-aromatic condensed polycyclic group, a substituted or unsubstituted monovalent non-aromatic condensed heteropolycyclic group, or-$Si(Q_{91})(Q_{92})(Q_{93})$,
b30 and b40 may each independently be an integer from 0 to 10,
c12 may be 0, 1, 2, or 3,
* may be a binding site to a neighboring atom,
a substituent of the substituted $C_1$-$C_{60}$ alkyl group, substituted $C_2$-$C_{60}$ alkenyl group, substituted $C_2$-$C_{60}$ alkynyl group, substituted $C_3$-$C_{10}$ cycloalkyl group, substituted $C_1$-$C_{10}$ heterocycloalkyl group, substituted $C_3$-$C_{10}$ cycloalkenyl group, substituted $C_1$-$C_{10}$ heterocycloalkenyl group, substituted $C_6$-$C_{60}$ aryl group, substituted $C_6$-$C_{60}$ aryloxy group, substituted $C_6$-$C_{60}$ arylthio group, substituted $C_1$-$C_{60}$ heteroaryl group, substituted monovalent non-aromatic condensed polycyclic group, and substituted monovalent non-aromatic condensed heteropolycyclic group may be deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, $-Si(Q_{101})(Q_{102})(Q_{103})$, or a combination thereof, and

$Q_{41}$ to $Q_{43}$, $Q_{51}$ to $Q_{52}$, $Q_{61}$ to $Q_{62}$, $Q_{71}$ to $Q_{73}$, $Q_{81}$ to $Q_{83}$, $Q_{91}$ to $Q_{93}$ and $Q_{101}$ to $Q_{103}$ may each independently be hydrogen, deuterium, a $C_1$-$C_{60}$ alkyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_1$-$C_{60}$ heteroaryl group, a monovalent non-aromatic condensed polycyclic group, or a monovalent non-aromatic condensed heteropolycyclic group.

For example, the host may include Compounds H-1 to H-27, but embodiments are not limited thereto:

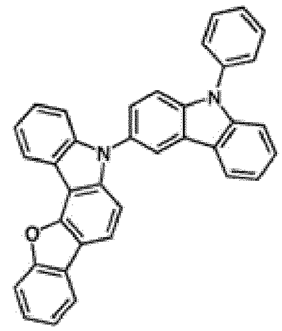
H-1

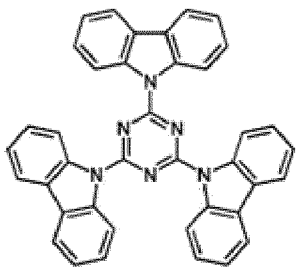
H-2

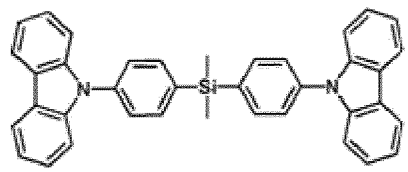
H-3

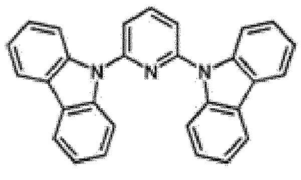
H-4

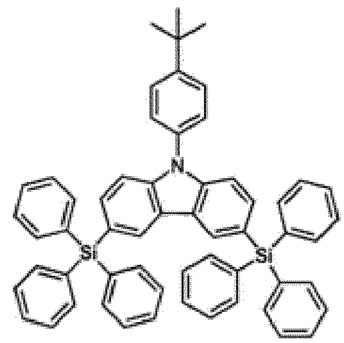
H-5

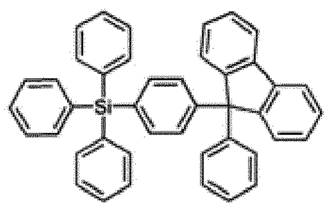
H-6

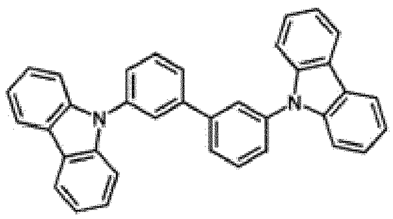
H-7

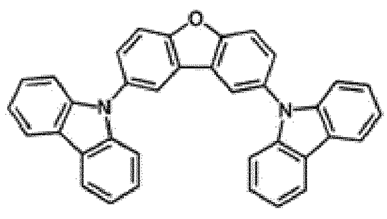
H-8

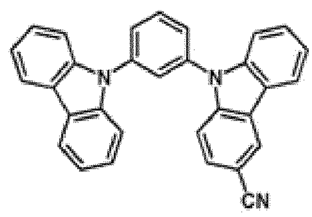
H-9

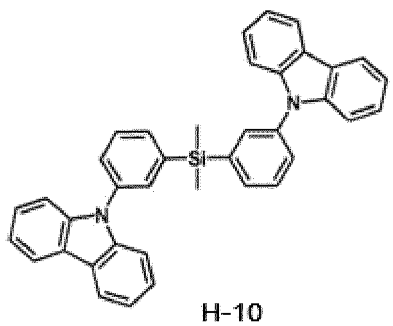
H-10

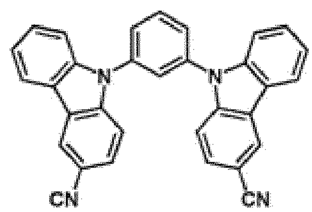
H-11

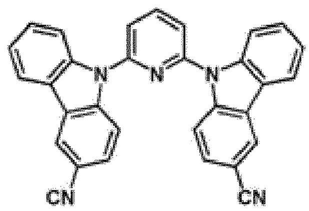
H-12

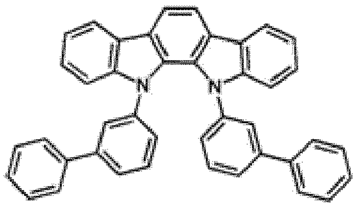
H-13

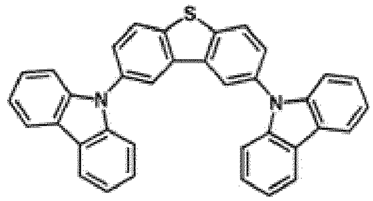
H-14

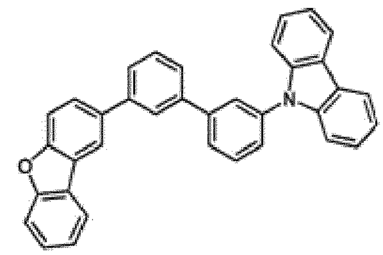
H-15

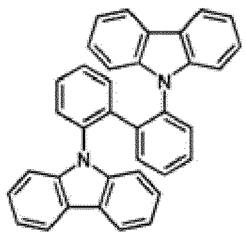
H-16

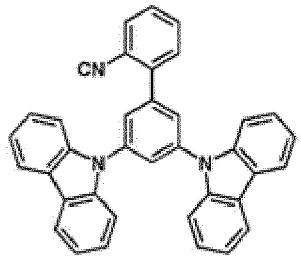
H-17

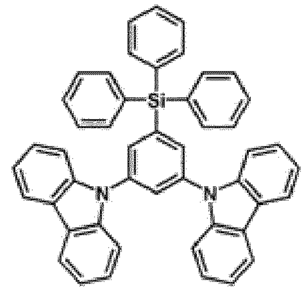
H-18

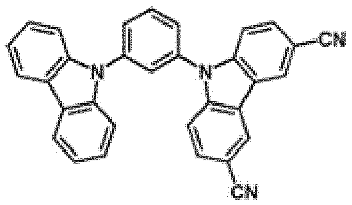
H-19

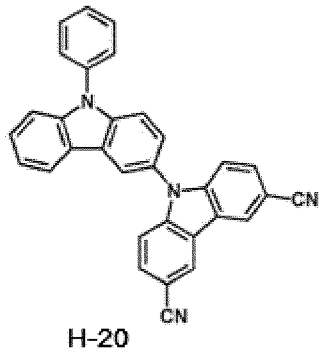
H-20

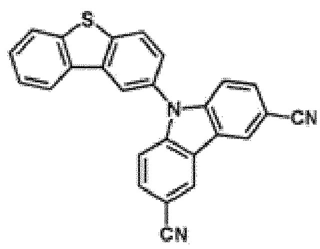
H-21

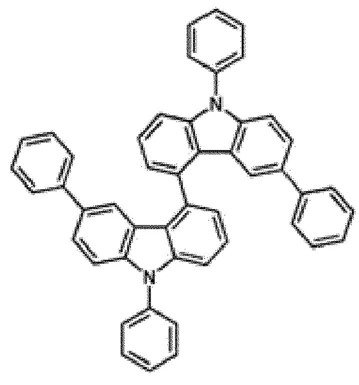
H-22

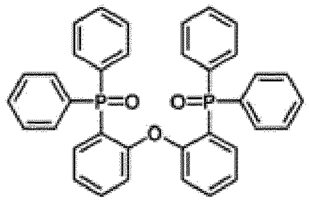
H-23

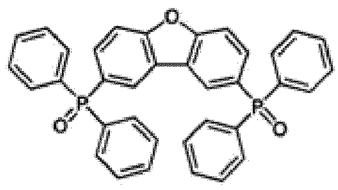
H-24

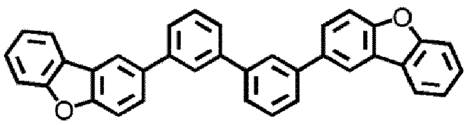

H-25

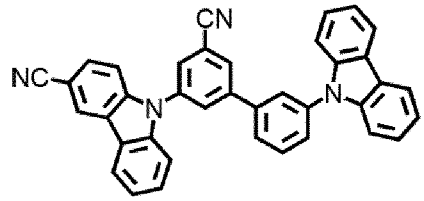

H-26

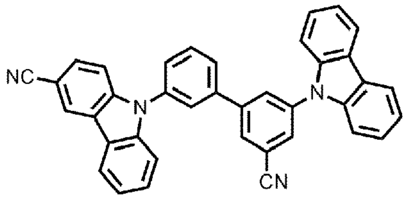

H-27

The fluorescent dopant may be a condensed polycyclic compound or a styryl compound.

For example, the fluorescent dopant may include a naphthalene-containing core, a fluorene-containing core, a spiro-bifluorene-containing core, a benzofluorene-containing core, a dibenzofluorene-containing core, a phenanthrene-containing core, an anthracene-containing core, a fluoranthene-containing core, a triphenylene-containing core, a pyrene-containing core, a chrysene-containing core, a naphthacene-containing core, a picene-containing core, a perylene-containing core, a pentaphene-containing core, an indenoanthracene-containing core, a tetracene-containing core, a bisanthracene-containing core, or a core represented by one of Formulae 501-1 to 501-18, but embodiments of the present disclosure are not limited thereto:

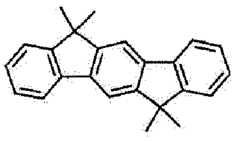

501-1

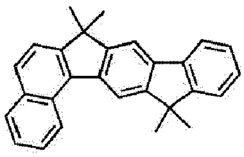

501-2

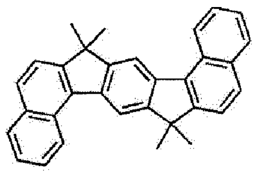

501-3

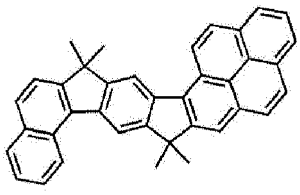

501-4

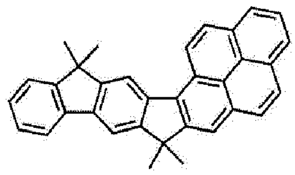

501-5

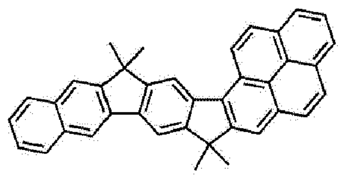

501-6

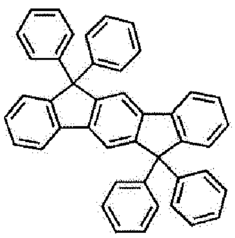

501-7

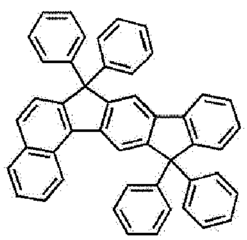

501-8

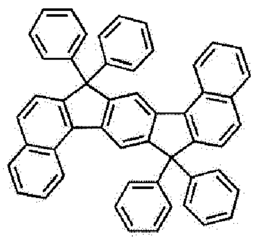

501-9

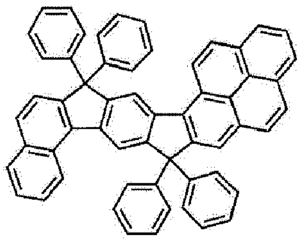

501-10

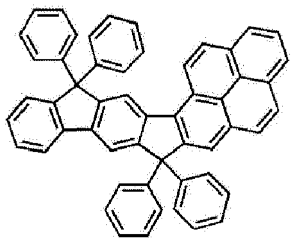

501-11

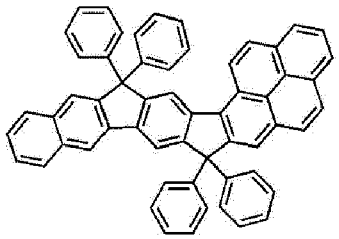

501-12

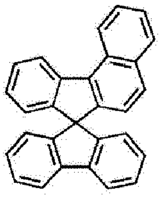

501-13

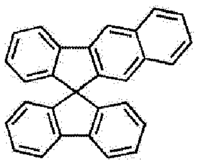

501-14

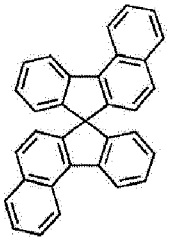

501-15

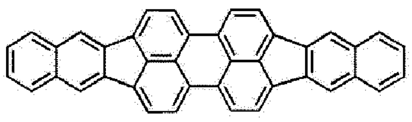

501-16

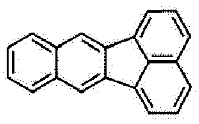

501-17

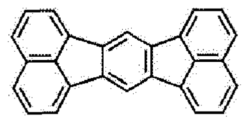

501-18 .

In one or more embodiments, the fluorescent dopant may be a styryl-amine compound or a styryl-carbazole compound, but embodiments of the present disclosure are not limited thereto.

In one embodiment, the fluorescent dopant may be a group represented by Formula 501:

Formula 501

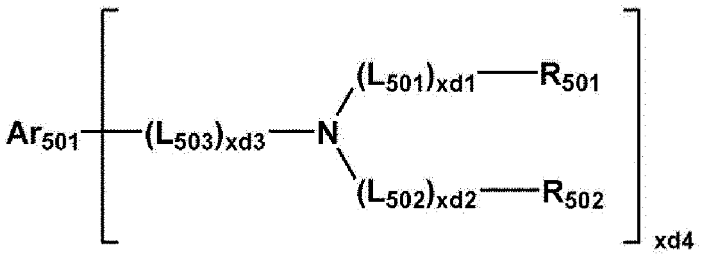

wherein, in Formula 501,

$Ar_{501}$ may be:

a naphthalene group, a fluorene group, a spiro-bifluorene group, a benzofluorene group, a dibenzofluorene group, a phenanthrene group, an anthracene group, a fluoranthene group, a triphenylene group, a pyrene group, a chrysene group, a naphthacene group, a picene group, a perylene group, a pentaphene group, an indenoanthracene group, a tetracene group, a bisanthracene group, or a group represented by one of Formulae 501-1 to 501-18; or

a naphthalene group, a fluorene group, a spiro-bifluorene group, a benzofluorene group, a dibenzofluorene group, a phenanthrene group, an anthracene group, a fluoranthene group, a triphenylene group, a pyrene group, a chrysene group, a naphthacene group, a picene group, a perylene group, a pentaphene an indenoanthracene group, a tetracene group, a bisanthracene, or a group represented by one of Formula 501-1 to 501-18, each substituted with deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof,

a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group,-Si($Q_{501}$)($Q_{502}$)($Q_{503}$) (wherein $Q_{501}$ to $Q_{503}$ may each independently be hydrogen, a $C_1$-$C_{60}$ alkyl group, a $C_1$-$C_{60}$ alkoxy group, a $C_6$-$C_{60}$ aryl group, a $C_1$-$C_{60}$ heteroaryl group, a monovalent non-aromatic condensed polycyclic group, or a monovalent non-aromatic condensed heteropolycyclic group), or a combination thereof;

$L_{501}$ to $L_{503}$ may each independently be a substituted or unsubstituted $C_3$-$C_{10}$ cycloalkylene group, a substituted or unsubstituted $C_1$-$C_{10}$ heterocycloalkylene group, a substituted or unsubstituted $C_3$-$C_{10}$ cycloalkenylene group, a substituted or unsubstituted $C_1$-$C_{10}$ heterocycloalkenylene group, a substituted or unsubstituted $C_6$-$C_{60}$ arylene group, a substituted or unsubstituted $C_1$-$C_{60}$ heteroarylene group, a substituted or unsubstituted divalent non-aromatic condensed polycyclic group, or a substituted or unsubstituted divalent non-aromatic condensed heteropolycyclic group,

$R_{501}$ and $R_{502}$ may each independently be:

a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazole group, a triazinyl group, a dibenzofuranyl group, or a dibenzothiophenyl group; or

a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, a triazinyl group, a dibenzofuranyl group, or a dibenzothiophenyl group, each substituted with deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, a triazinyl group, a dibenzofuranyl group, a dibenzothiophenyl group, or a combination thereof;

xd1 to xd3 may each independently be 0, 1, 2, or 3, and

xd4 may be 0, 1, 2, 3, 4, 5, or 6.

For example, in Formula 501,

$Ar_{501}$ may be:

a naphthalene group, a fluorene group, a spiro-bifluorene group, a benzofluorene group, a dibenzofluorene group, a phenanthrene group, an anthracene group, a fluoranthene group, a triphenylene group, a pyrene group, a chrysene group, a naphthacene group, a picene group, a perylene group, a pentaphene group, an indenoanthracene group, a tetracene group, a bisanthracene group, or a group represented by one of Formulae 501-1 to 501-18; or

a naphthalene group, a fluorene group, a spiro-bifluorene group, a benzofluorene group, a dibenzofluorene group, a phenanthrene group, an anthracene group, a fluoranthene group, a triphenylene group, a pyrene group, a chrysene group, a naphthacene group, a picene group, a perylene group, a pentaphene group, an indenoanthracene group, a tetracene group, a bisanthracene group, or a group represented by one of Formulae 501-1 to 501-18, each substituted with deuterium, -F,-Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a phenyl group, a biphenyl group, a terphenyl group, a naphthyl group, a fluorenyl group, a dibenzofuranyl group, a dibenzothiophenyl group, a carbazolyl group, a pyridinyl group, a pyrimidinyl group, a triazinyl group, a quinolinyl group, an isoquinolinyl group, and -Si($Q_{501}$)($Q_{502}$)($Q_{503}$) (wherein $Q_{501}$ to $Q_{503}$ may each independently be hydrogen, a $C_1$-$C_{20}$ alkyl group, a $C_1$-$C_{20}$ alkoxy group, a phenyl group, a biphenyl group, a terphenyl group, or a naphthyl group), or a combination thereof;

$L_{501}$ to $L_{503}$ are the same as described in connection with $L_{21}$,

xd1 to xd3 may each independently be 0, 1, or 2,

xd4 may be 0, 1, 2, or 3, but embodiments of the present disclosure are not limited thereto.

In one or more embodiments, the fluorescent dopant may include a compound represented by one of Formulae 502-1 to 502-5:

Formula 502-1

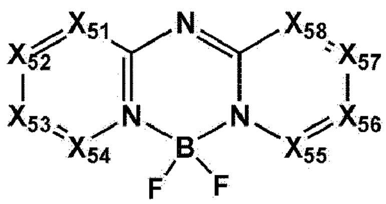

Formula 502-2

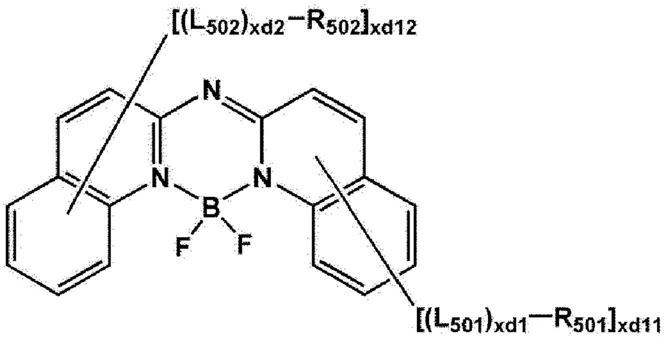

Formula 502-3

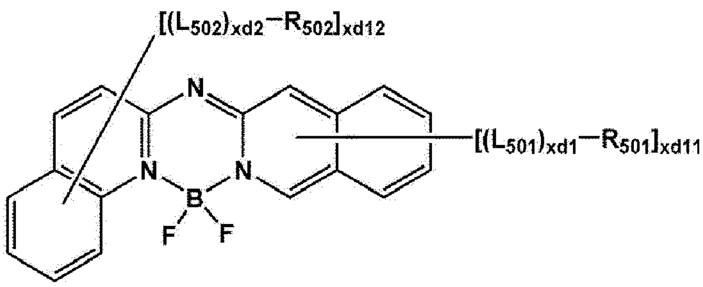

Formula 502-4

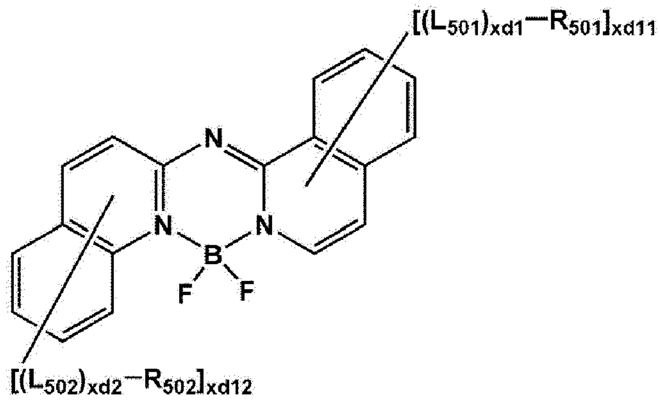

Formula 502-5

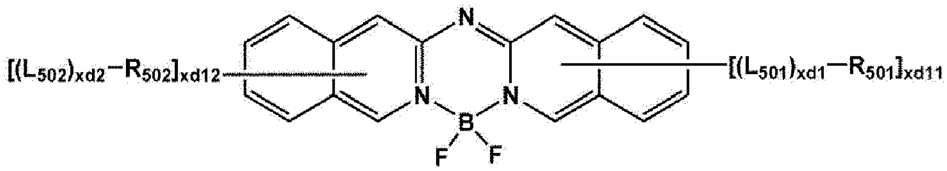

In Formulae 502-1 to 502-5,

$X_{51}$ may be N or C-[$(L_{501})_{xd1}$-$R_{501}$], $X_{52}$ may be N or C-[$(L_{50}2)_{xd2}$-$R_{502}$], $X_{53}$ may be N or C-[$(L_{503})_{xd3}$-$R_{503}$], $X_{54}$ may be N or C-[$(L_{504})_{xd4}$-$R_{504}$], $X_{55}$ may be N or C-[$(L_{505})_{xd5}$-$R_{505}$], X56 may be N or C-[$(L_{506})_{xd6}$-$R_{506}$], $X_{57}$ may be N or C-[$(L_{507})_{xd7}$-$R_{507}$], and $X_{58}$ may be N or C-[$(L_{508})_{xd8}$-$R_{508}$],

$L_{501}$ to $L_{508}$ may each be understood by referring to the description provided in connection with $L_{501}$ in Formula 501,

xd1 to xd8 may each be understood by referring to the description provided in connection with xd1 in Formula 501,

$R_{501}$ to $R_{508}$ may each independently be

hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, or a $C_1$-$C_{20}$ alkoxy group,

a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazole group, a triazinyl group, a dibenzofuranyl group, or a dibenzothiophenyl group; or

a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, a triazinyl group, a dibenzofuranyl group, or a dibenzothiophenyl group, each substituted with deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, a triazinyl group, a dibenzofuranyl group, a dibenzothiophenyl group, or a combination thereof;

xd11 and xd12 may each independently be an integer from 0 to 5,

two of $R_{501}$ to $R_{504}$ may optionally be combined with each other to form a saturated or unsaturated ring, and

two of $R_{505}$ to $R_{508}$ may optionally be combined with each other to form a saturated or unsaturated ring.

The fluorescent dopant may include, for example, Compounds FD(1) to FD(16) and FD1 to FD13:

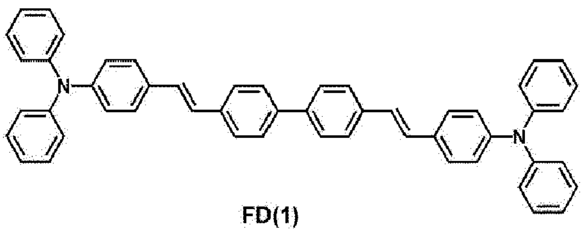

FD(1)

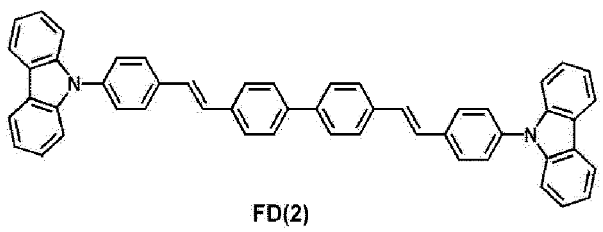

FD(2)

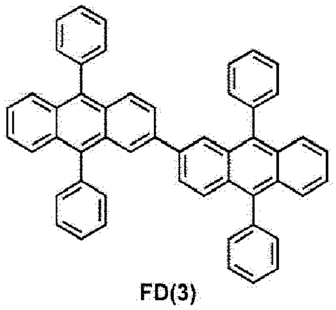

FD(3)

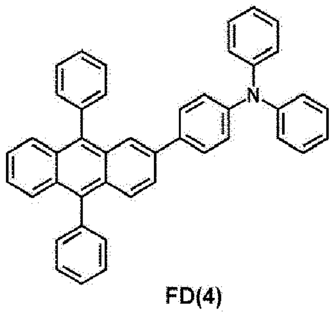

FD(4)

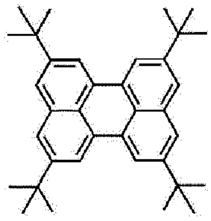

FD(5)

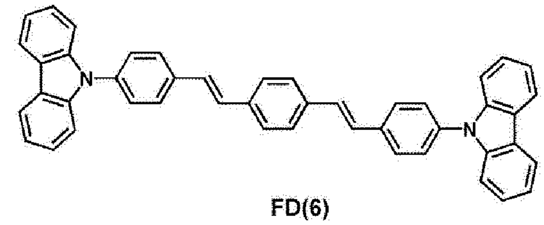

FD(6)

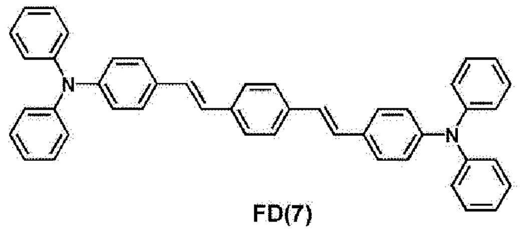

FD(7)

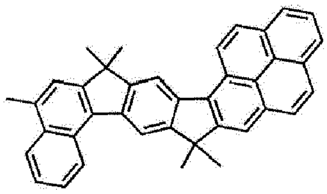

FD(8)

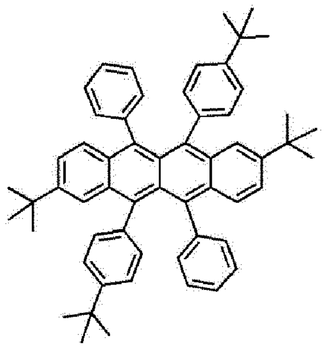

FD(9)

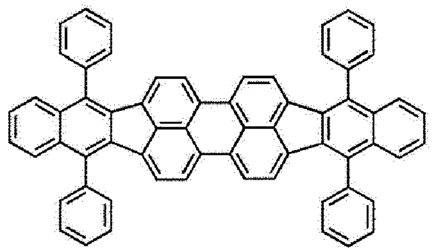

FD(10)

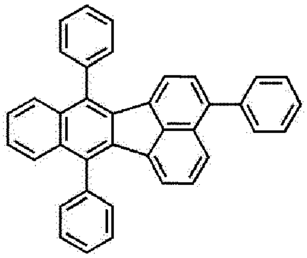

FD(11)

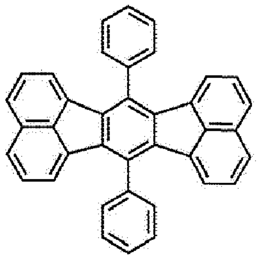

FD(12)

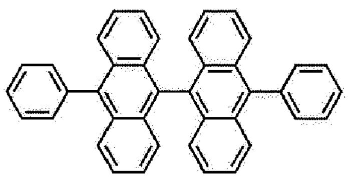

FD(13)

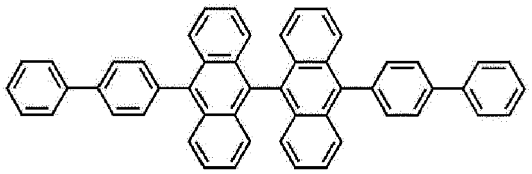

FD(14)

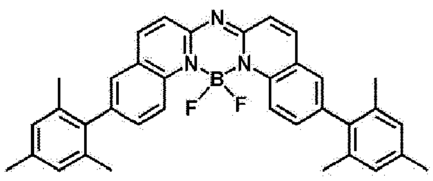

FD(15)

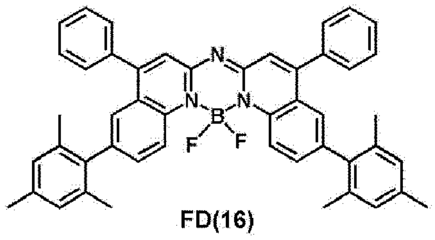

FD(16)

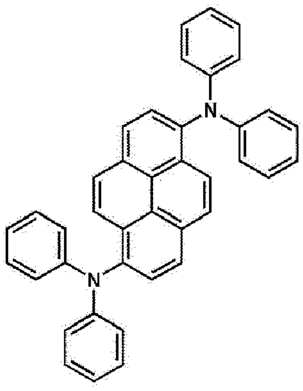

FD1

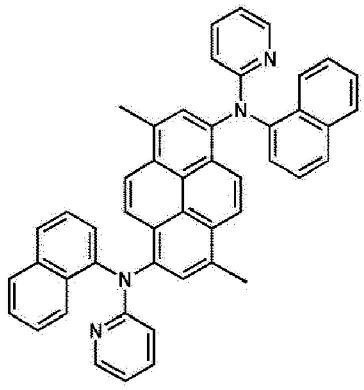

FD2

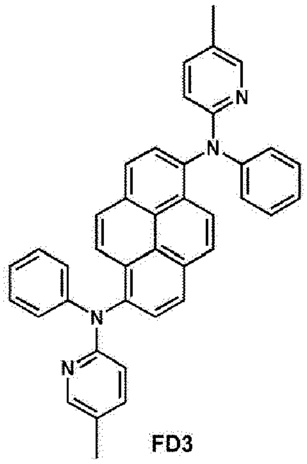

FD3

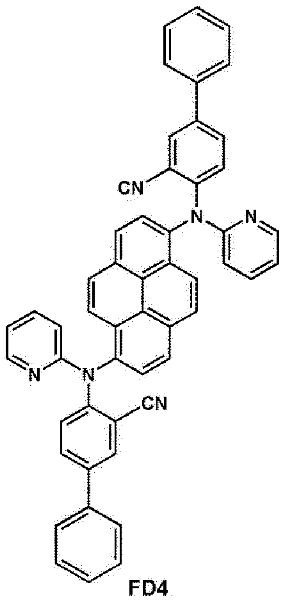

FD4

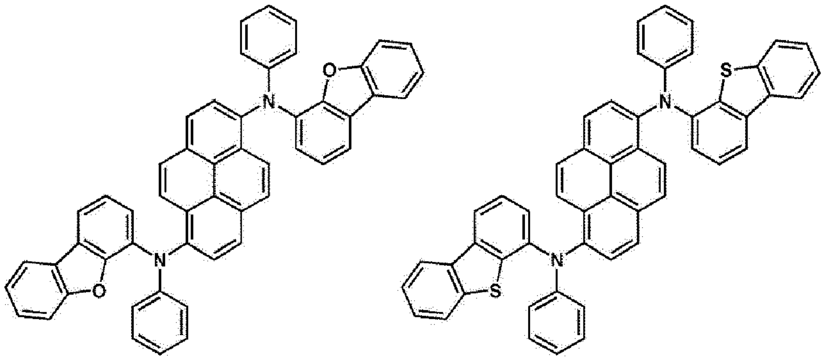

FD5

FD6

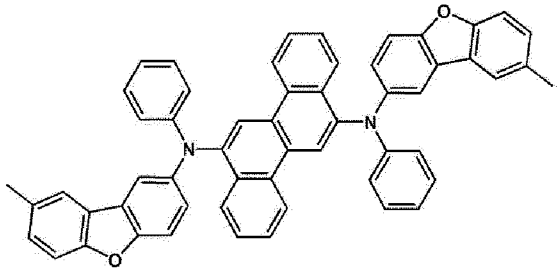

FD7

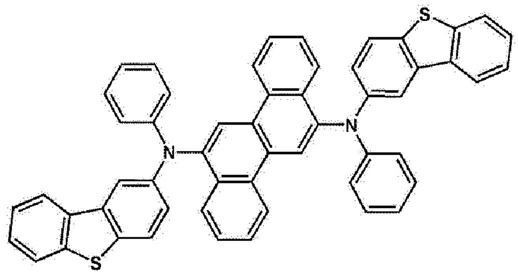

FD8

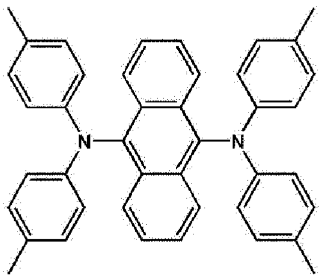

FD9

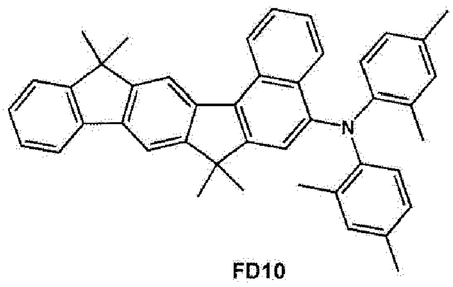

FD10

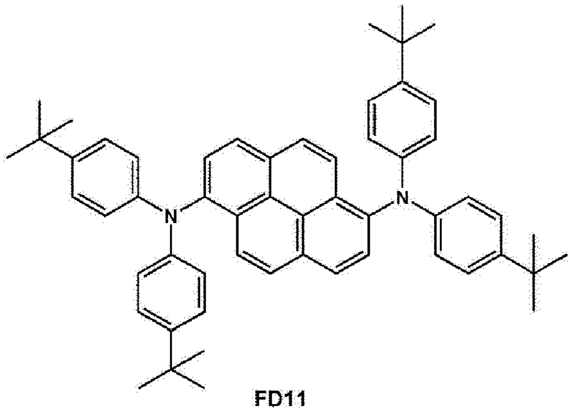

FD11

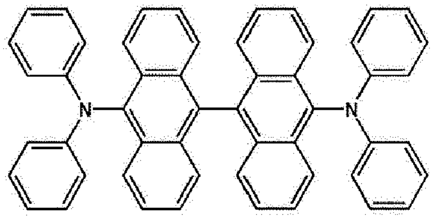

FD12

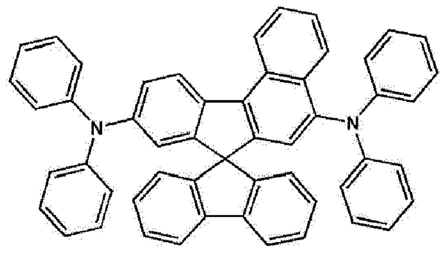

FD13

FIGURE is a schematic view of an organic light-emitting device 10 according to one embodiment. Hereinafter, the structure of an organic light-emitting device according to an embodiment and a method of manufacturing an organic light-emitting device according to an embodiment will be described in connection with FIGURE. The organic light-emitting device 10 includes a first electrode 11, an organic layer 15, and a second electrode 19, which are sequentially stacked.

A substrate may be additionally located under the first electrode 11 or above the second electrode 19. For use as the substrate, any substrate that is used in organic light-emitting devices available in the art may be used, and the substrate may be a glass substrate or a transparent plastic substrate, each having excellent mechanical strength, thermal stability, transparency, surface smoothness, ease of handling, and water resistance.

In one or more embodiments, the first electrode 11 may be formed by depositing or sputtering a material for forming the first electrode 11 on the substrate. The first electrode 11 may be an anode. The material for forming the first electrode 11 may be a material with a high work function to facilitate hole injection. The first electrode 11 may be a reflective electrode, a semi-transmissive electrode, or a transmissive electrode. The material for forming the first electrode 11 may be indium tin oxide (ITO), indium zinc oxide (IZO), tin oxide ($SnO_2$), or zinc oxide (ZnO). In one or more embodiments, the material for forming the first electrode 11 may be metal, such as magnesium (Mg), aluminum (Al), aluminum-lithium (Al-Li), calcium (Ca), magnesium-indium (Mg-In), or magnesium-silver (Mg-Ag).

The first electrode 11 may have a single-layered structure or a multi-layered structure including two or more layers. For example, the first electrode 11 may have a three-layered structure of ITO/Ag/ITO, but the structure of the first electrode 11 is not limited thereto.

The organic layer 15 is located on the first electrode 11.

The organic layer 15 may include a hole transport region, an emission layer, and an electron transport region.

The hole transport region may be between the first electrode 11 and the emission layer.

The hole transport region may include a hole injection layer, a hole transport layer, an electron blocking layer, a buffer layer, or a combination thereof.

The hole transport region may include only either a hole injection layer or a hole transport layer. In one or more embodiments, the hole transport region may have a hole injection layer/hole transport layer structure or a hole injection layer/hole transport layer/electron blocking layer structure, which are sequentially stacked in this stated order from the first electrode 11.

When the hole transport region includes a hole injection layer (HIL), the hole injection layer may be formed on the first electrode 11 by using one or more suitable methods, for example, vacuum deposition, spin coating, casting, and/or Langmuir-Blodgett (LB) deposition.

When a hole injection layer is formed by vacuum deposition, the deposition conditions may vary according to a material that is used to form the hole injection layer, and the structure and thermal characteristics of the hole injection layer. For example, the deposition conditions may include a deposition temperature of about 100 °C to about 500 °C, a vacuum pressure of about $10^{-8}$ torr to about $10^{-3}$ torr, and a deposition rate of about 0.01 Å/sec to about 100 Å/sec. However, the deposition conditions are not limited thereto.

When the hole injection layer is formed using spin coating, coating conditions may vary according to the material used to form the hole injection layer, and the structure and thermal properties of the hole injection layer. For example, a coating speed may be from about 2,000 rpm to about 5,000 rpm, and a temperature at which a heat treatment is performed to remove a solvent after coating may be from about 80 °C to about 200 °C. However, the coating conditions are not limited thereto.

Conditions for forming a hole transport layer and an electron blocking layer may be understood by referring to conditions for forming the hole injection layer.

The hole transport region may include m-MTDATA, TDATA, 2-TNATA, NPB, β-NPB, TPD, Spiro-TPD, Spiro-NPB, methylated-NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonic acid (PANI/CSA), polyaniline/poly(4-styrenesulfonate) (PANI/PSS), a compound represented by Formula 201 below, a compound represented by Formula 202 below, or a combination thereof:

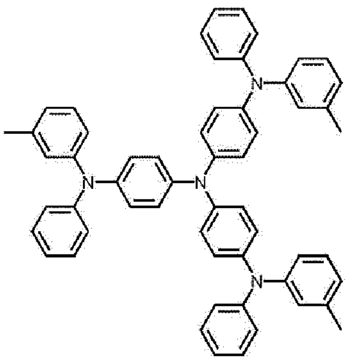

m-MTDATA

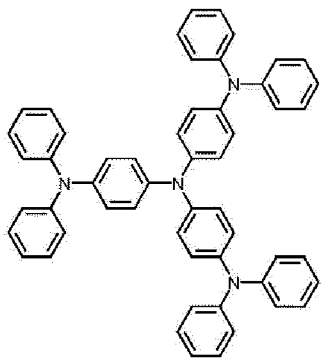

TDATA

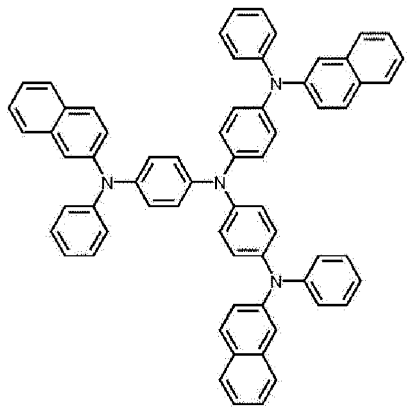

2-TNATA

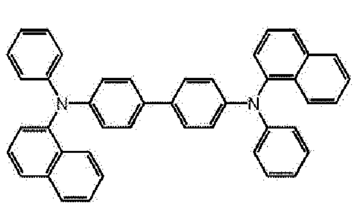

NPB

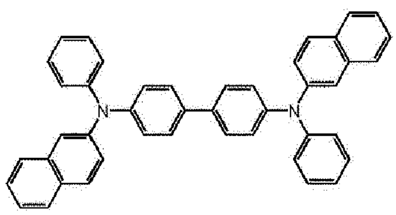

β-NPB

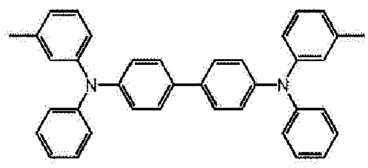

TPD

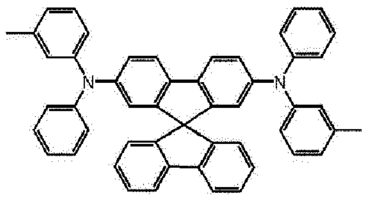

Spiro-TPD

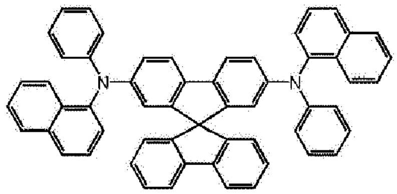

Spiro-NPB

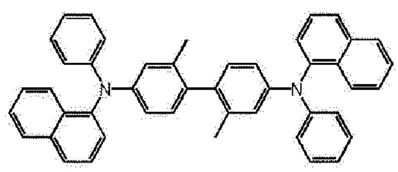

methylated NPB

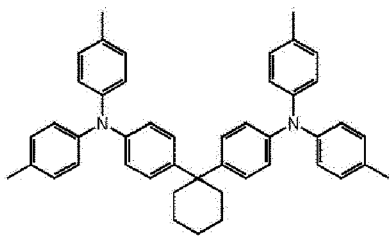

TAPC

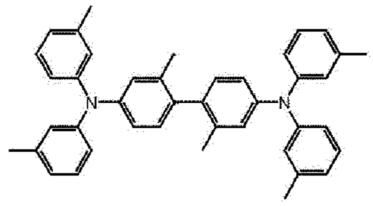

HMTPD

Formula 201

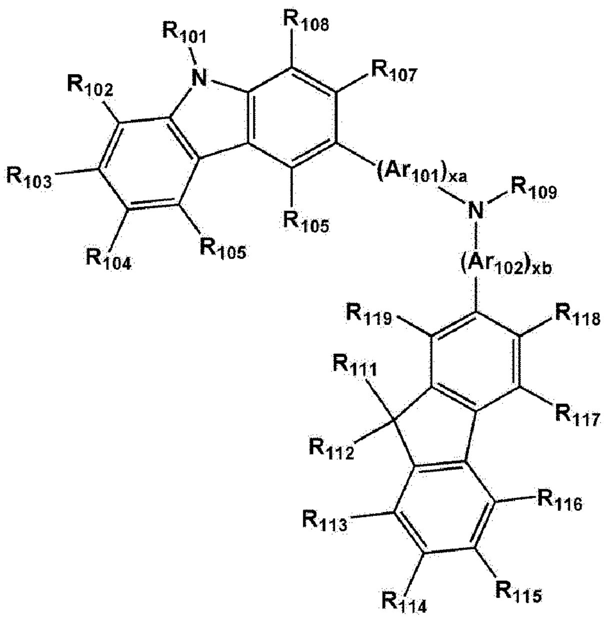

Formula 202

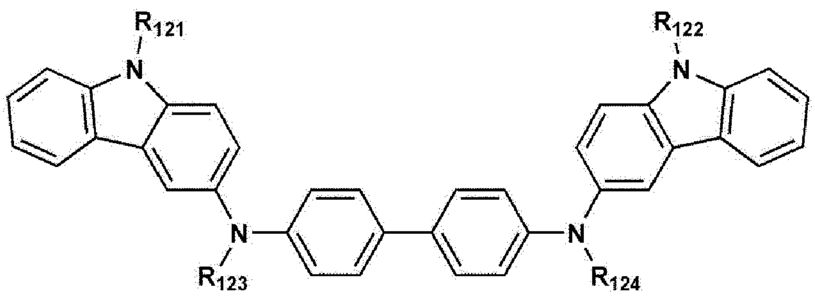

$Ar_{101}$ to $Ar_{102}$ in Formula 201 may each independently be:

a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, or a pentacenylene group; or a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, or a pentacenylene group, each substituted with deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_3$-$C_{10}$ cycloalkenyl group, a $C_1$-$C_{10}$ heterocycloalkyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, or a combination thereof.

xa and xb in Formula 201 may each independently be an integer from 0 to 5, or 0, 1, or 2. For example, xa may be 1 and xb may be 0, but xa and xb are not limited thereto.

$R_{101}$ to $R_{108}$, $R_{111}$ to $R_{119}$ and $R_{121}$ to $R_{124}$ in Formulae 201 and 202 may each independently be:

hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group (for example, a methyl group, an ethyl group, a propyl group, a butyl group, pentyl group, a hexyl group, etc.) or a $C_1$-$C_{10}$ alkoxy group (for example, a methoxy group, an ethoxy group, a propoxy group, a butoxy group, a pentoxy group, etc.);

a $C_1$-$C_{10}$ alkyl group, or a $C_1$-$C_{10}$ alkoxy group, each substituted with deuterium,-F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof and a phosphoric acid group or a salt thereof, or a combination thereof;

a phenyl group, a naphthyl group, an anthracenyl group, a fluorenyl group, or a pyrenyl group; or

a phenyl group, a naphthyl group, an anthracenyl group, a fluorenyl group, or a pyrenyl group, each substituted with deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group, a $C_1$-$C_{10}$ alkoxy group, or a combination thereof,

but embodiments of the present disclosure are not limited thereto.

$R_{109}$ in Formula 201 may be:

a phenyl group, a naphthyl group, an anthracenyl group, or a pyridinyl group; or

a phenyl group, a naphthyl group, an anthracenyl group, or a pyridinyl group, each substituted with deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a phenyl group, a naphthyl group, an anthracenyl group, a pyridinyl group, or a combination thereof.

According to an embodiment, the compound represented by Formula 201 may be represented by Formula 201A below, but embodiments of the present disclosure are not limited thereto:

Formula 201A

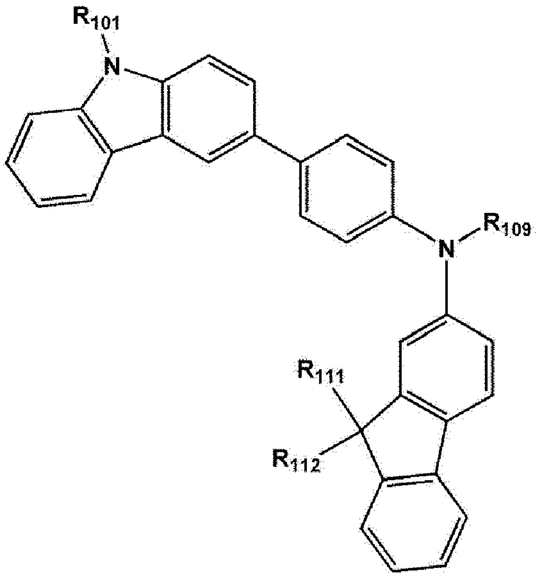

$R_{101}$, $R_{111}$, $R_{112}$, and $R_{109}$ in Formula 201A may be understood by referring to the description provided herein.

For example, the compound represented by Formula 201, and the compound represented by Formula 202 may include compounds HT1 to HT20 illustrated below, but are not limited thereto:

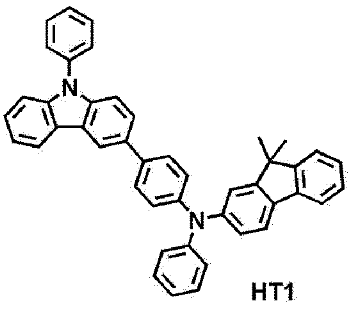

HT1

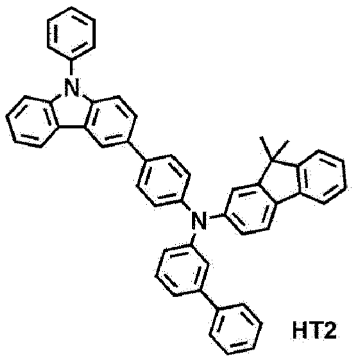

HT2

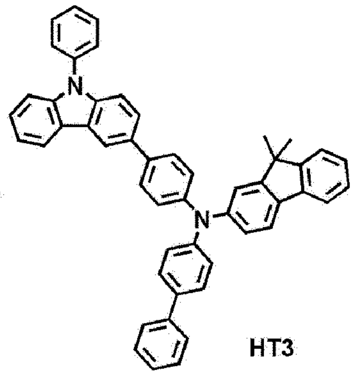

HT3

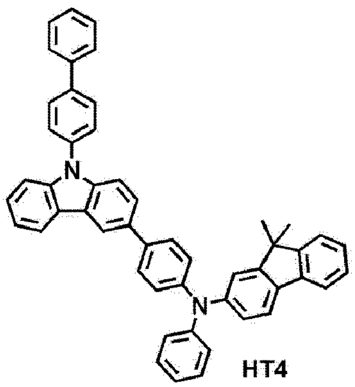

HT4

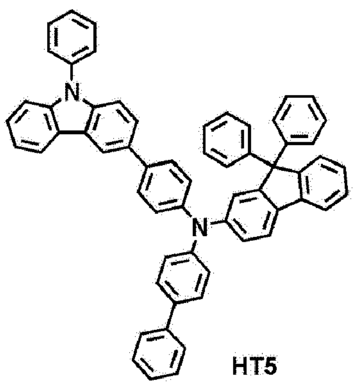

HT5

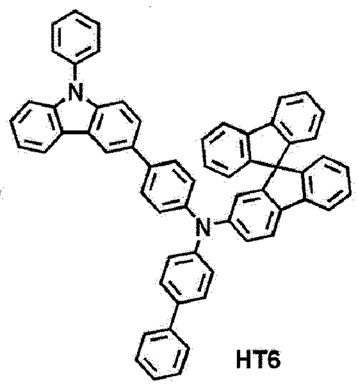

HT6

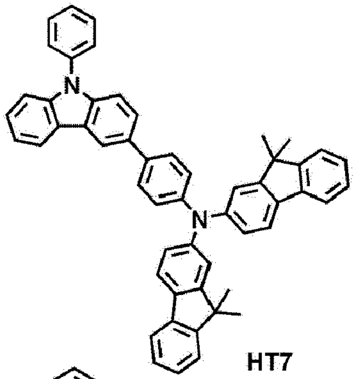

HT7

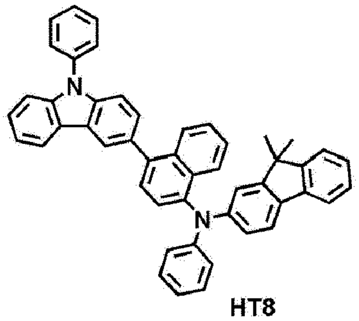

HT8

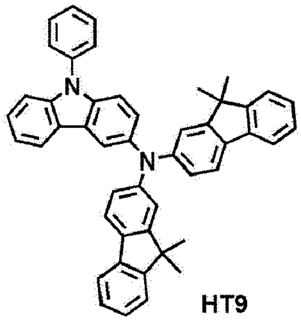

HT9

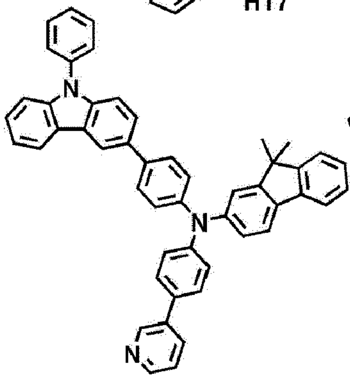

HT10

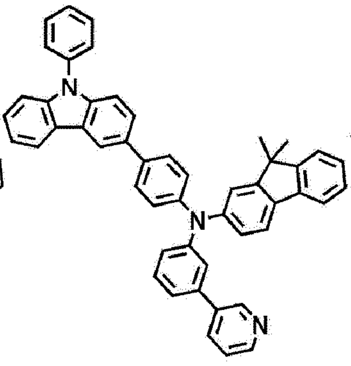

HT11

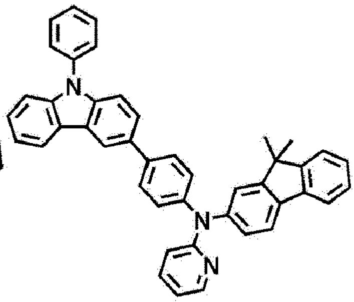

HT12

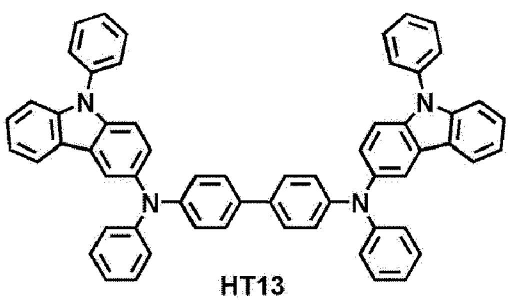

HT13

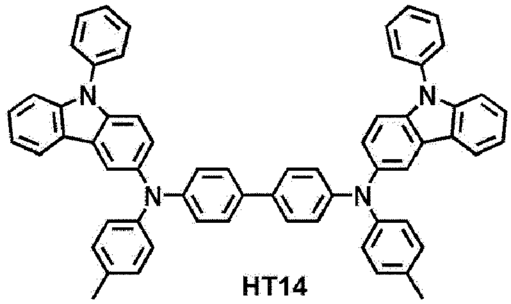

HT14

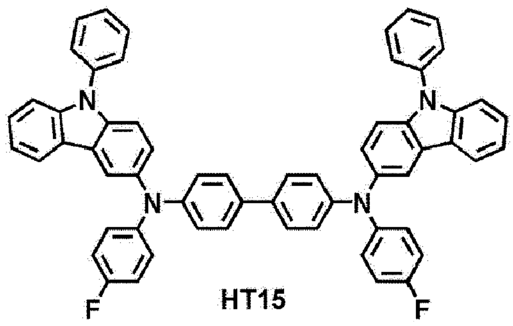

HT15

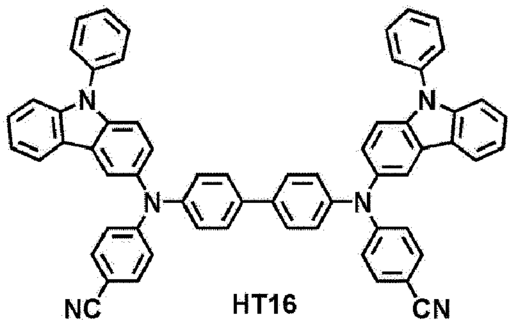

HT16

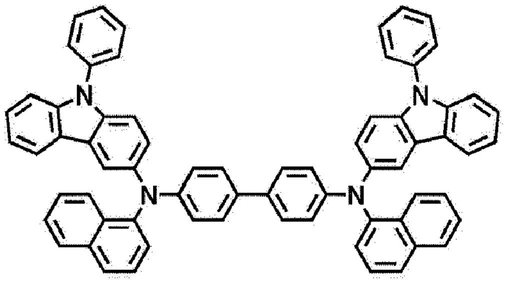

HT17

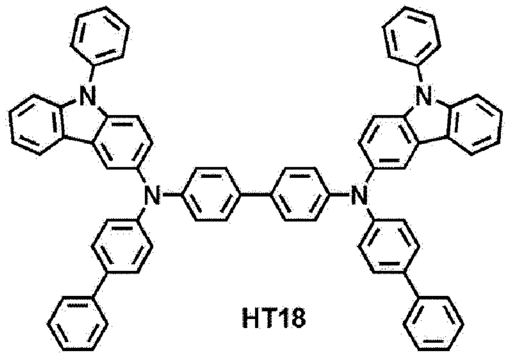

HT18

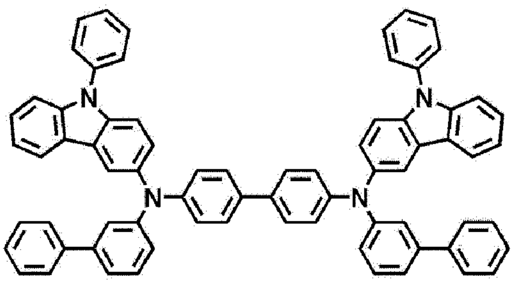

HT19

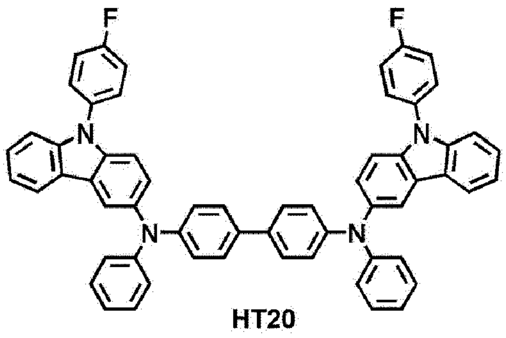

HT20

A thickness of the hole transport region may be in a range of about 100 Å to about 10,000 Å, for example, about 100 Å to about 1,000 Å. When the hole transport region includes a hole injection layer and a hole transport layer at the same time, a thickness of the hole injection layer may be in a range of about 100 Å to about 10,000 Å, for example, about 100 Å to about 1,000 Å, and a thickness of the hole transport layer may be in a range of about 50 Å to about 2,000 Å, for example, about 100 Å to about 1,500 Å. When the thicknesses of the hole transport region, the hole injection layer and the hole transport layer are within these ranges, satisfactory hole transporting characteristics may be obtained without a substantial increase in driving voltage.

The hole transport region may further include, in addition to these materials, a charge-generation material for the improvement of conductive properties. The charge-generation material may be homogeneously or non-homogeneously dispersed in the hole transport region.

The charge-generation material may be, for example, a p-dopant. The p-dopant may be one a quinone derivative, a metal oxide, or a cyano group-containing compound, but embodiments of the present disclosure are not limited thereto. Non-limiting examples of the p-dopant are a quinone derivative, such as tetracyanoquinonedimethane (TCNQ) or 2,3,5,6-tetrafluoro-tetracyano-1,4-benzoquinonedimethane (F4-TCNQ); a metal oxide, such as a tungsten oxide or a molybdenum oxide; and a cyano group-containing compound, such as Compound HT-D1 or Compound HP-1 below, but are not limited thereto.

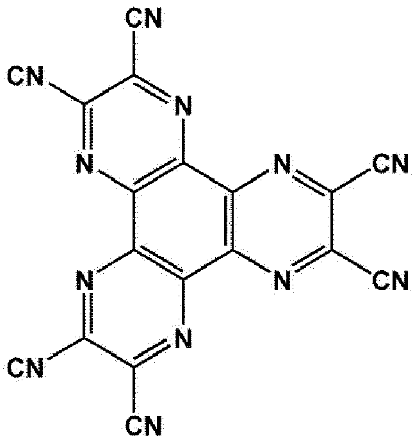

HT-D1

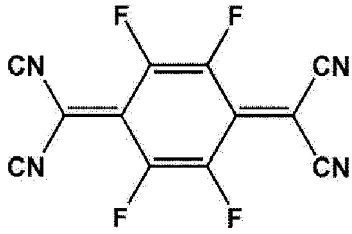

F4-TCNQ

HP-1

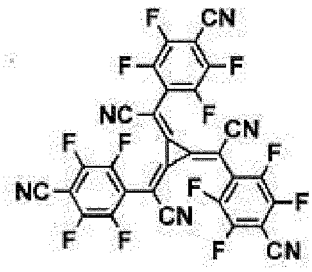

The hole transport region may include a buffer layer.

Also, the buffer layer may compensate for an optical resonance distance according to a wavelength of light emitted from the emission layer, and thus, efficiency of a formed organic light-emitting device may be improved.

The hole transport region may further include an electron blocking layer. The electron blocking layer may include a material available in the art, for example, mCP, but embodiments of the present disclosure are not limited.

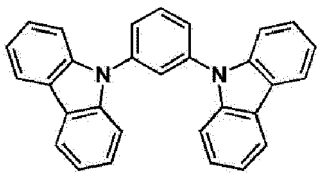

mCP

An emission layer (EML) may be formed on the hole transport region by vacuum deposition, spin coating, casting, LB deposition, or the like. When the emission layer is formed by vacuum deposition or spin coating, the deposition or coating conditions may be similar to those applied in forming the hole injection layer although the deposition or coating conditions may vary according to a material that is used to form the hole transport layer.

When the organic light-emitting device is a full-color organic light-emitting device, the emission layer may be patterned into a red emission layer, a green emission layer, and a blue emission layer. In one or more embodiments, due to a stacked structure including a red emission layer, a green emission layer, and/or a blue emission layer, the emission layer may emit white light.

The description of the emission layer is given in this specification.

A thickness of the emission layer may be in a range of about 100 Å to about 1,000 Å, for example, about 200 Å to about 600 Å. When the thickness of the emission layer is within this range, excellent light-emission characteristics may be obtained without a substantial increase in driving voltage.

Then, an electron transport region may be located on the emission layer.

The electron transport region may include a hole blocking layer, an electron transport layer, an electron injection layer, or a combination thereof.

For example, the electron transport region may have a hole blocking layer/electron transport layer/electron injection layer structure or an electron transport layer/electron injection layer structure, but the structure of the electron transport region is not limited thereto. The electron transport layer may have a single-layered structure or a multi-layered structure including two or more different materials.

Conditions for forming the hole blocking layer, the electron transport layer, and the electron injection layer which constitute the electron transport region may be understood by referring to the conditions for forming the hole injection layer.

When the electron transport region includes a hole blocking layer, the hole blocking layer may include, for example, BCP, Bphen, or a combination thereof, but embodiments of the present disclosure are not limited thereto.

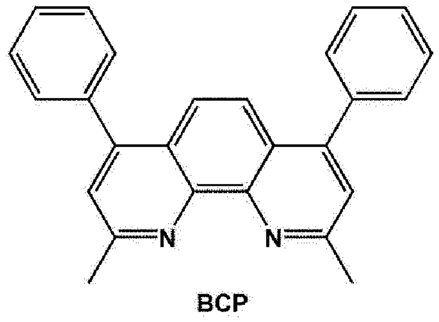

BCP

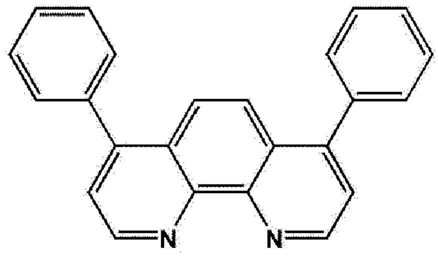

Bphen

In one or more embodiments, the hole blocking layer may include a host. For example, the hole blocking layer may include Compound H19, but embodiments are not limited thereto.

A thickness of the hole blocking layer may be in a range of about 20 Å to about 1,000 Å, for example, about 30 Å to about 300 Å. When the thickness of the hole blocking layer is within these ranges, the hole blocking layer may have excellent hole blocking characteristics without a substantial increase in driving voltage.

The electron transport layer may further include BCP, Bphen, $Alq_3$, BAlq, TAZ, NTAZ, or a combination thereof.

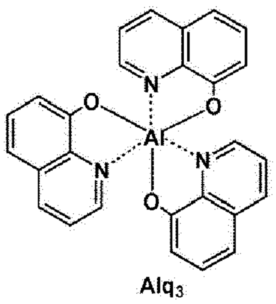

$Alq_3$

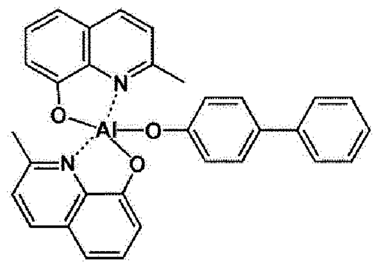

BAlq

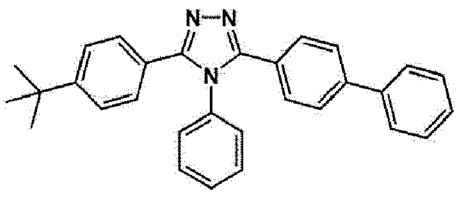

TAZ

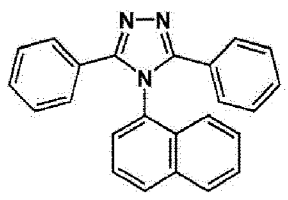

NTAZ

In one or more embodiments, the electron transport layer may include ET1, ET2, and ET3, or a combination thereof, but is not limited thereto:

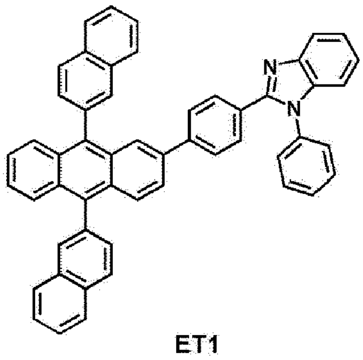

ET1

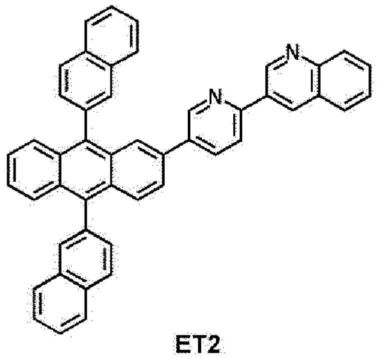

ET2

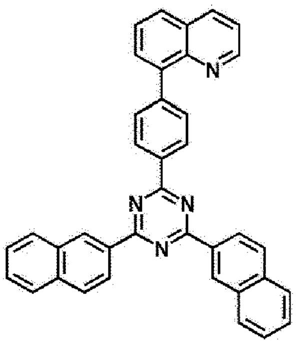

ET3

A thickness of the electron transport layer may be in a range of about 100 Å to about 1,000 Å, for example, about 150 Å to about 500 Å. When the thickness of the electron transport layer is within the range described above, the electron transport layer may have satisfactory electron transport characteristics without a substantial increase in driving voltage.
Also, the electron transport layer may further include, in addition to the materials described above, a metal-containing material.
The metal-containing material may include a Li complex. The Li complex may include, for example, Compound ET-D1 (lithium 8-hydroxyquinolate, LiQ) or ET-D2.

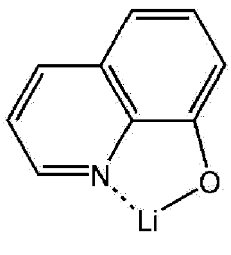

ET-D1

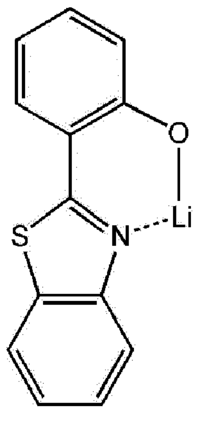

ET-D2

The electron transport region may include an electron injection layer (EIL) that promotes flow of electrons from the second electrode 19 thereinto.
The electron injection layer may include LiF, NaCl, CsF, $Li_2O$, BaO, or a combination thereof.
A thickness of the electron injection layer may be in a range of about 1 Å to about 100 Å, for example, about 3 Å to about 90 Å. When the thickness of the electron injection layer is within the range described above, the electron injection layer may have satisfactory electron injection characteristics without a substantial increase in driving voltage.

The second electrode 19 is located on the organic layer 15. The second electrode 19 may be a cathode. A material for forming the second electrode 19 may be metal, an alloy, an electrically conductive compound, or a combination thereof, which have a relatively low work function. For example, lithium (Li), magnesium (Mg), aluminum (Al), aluminum-lithium (Al-Li), calcium (Ca), magnesium-indium (Mg-In), or magnesium-silver (Mg-Ag) may be formed as the material for forming the second electrode 19. To manufacture a top-emission type light-emitting device, a transmissive electrode formed using ITO or IZO may be used as the second electrode 19.

Hereinbefore, the organic light-emitting device has been described with reference to FIGURE, but embodiments of the present disclosure are not limited thereto.

The term "$C_1$-$C_{60}$ alkyl group" as used herein refers to a linear or branched saturated aliphatic hydrocarbon monovalent group having 1 to 60 carbon atoms, and non-limiting examples thereof include a methyl group, an ethyl group, a propyl group, an isobutyl group, a sec-butyl group, a tert-butyl group, a pentyl group, an isoamyl group, and a hexyl group. The term "$C_1$-$C_{60}$ alkylene group" used herein refers to a divalent group having the same structure as that of the $C_1$-$C_{60}$ alkyl group.

The term "$C_1$-$C_{60}$ alkoxy group" used herein refers to a monovalent group represented by $-OA_{101}$ (wherein $A_{101}$ is the $C_1$-$C_{60}$ alkyl group), and examples thereof include a methoxy group, an ethoxy group, and an isopropyloxy group.

The term "$C_2$-$C_{60}$ alkenyl group" as used herein refers to a hydrocarbon group formed by substituting a carbon-carbon double bond in the middle or at the terminus of the $C_2$-$C_{60}$ alkyl group, and examples thereof include an ethenyl group, a propenyl group, and a butenyl group. The term "$C_2$-$C_{60}$ alkenylene group" used herein refers to a divalent group having the same structure as that of the $C_2$-$C_{60}$ alkenyl group.

The term "$C_2$-$C_{60}$ alkynyl group" as used herein refers to a hydrocarbon group formed by substituting a carbon-carbon triple bond in the middle or at the terminus of the $C_2$-$C_{60}$ alkyl group, and examples thereof include an ethynyl group, and a propynyl group. The term "$C_2$-$C_{60}$ alkynylene group" as used herein refers to a divalent group having the same structure as that of the $C_2$-$C_{60}$ alkynyl group.

The term "$C_3$-$C_{10}$ cycloalkyl group" as used herein refers to a monovalent saturated hydrocarbon monocyclic group having 3 to 10 carbon atoms, and examples thereof include a cyclopropyl group, a cyclobutyl group, a cyclopentyl group, a cyclohexyl group, and a cycloheptyl group. The term "$C_3$-$C_{10}$ cycloalkylene group" as used herein refers to a divalent group having the same structure as that of the $C_3$-$C_{10}$ cycloalkyl group.

The term "$C_1$-$C_{10}$ heterocycloalkyl group" as used herein refers to a monovalent saturated monocyclic group having N, O, P, Si, Se, S, or a combination thereof as ring-forming atoms and 1 to 10 carbon atoms, and non-limiting examples thereof include a tetrahydrofuranyl group, and a tetrahydrothiophenyl group. The term "$C_1$-$C_{10}$ heterocycloalkylene group" as used herein refers to a divalent group having the same structure as the $C_1$-$C_{10}$ heterocycloalkyl group.

The term "$C_3$-$C_{10}$ cycloalkenyl group" as used herein refers to a monovalent monocyclic group that has 3 to 10 carbon atoms and a carbon-carbon double bond in the ring thereof and no aromaticity, and non-limiting examples thereof include a cyclopentenyl group, a cyclohexenyl group, and a cycloheptenyl group. The term "$C_3$-$C_{10}$ cycloalkenylene group" as used herein refers to a divalent group having the same structure as the $C_3$-$C_{10}$ cycloalkenyl group.

The term "$C_1$-$C_{10}$ heterocycloalkenyl group" as used herein refers to a monovalent monocyclic group that has N, O, P, Si, Se, S, or a combination thereof as ring-forming atoms, 1 to 10 carbon atoms, and a carbon-carbon double bond in its ring. Examples of the $C_1$-$C_{10}$ heterocycloalkenyl group are a 2,3-dihydrofuranyl group, and a 2,3-dihydrothiophenyl group. The term "$C_1$-$C_{10}$ heterocycloalkenylene group" as used herein refers to a divalent group having the same structure as the $C_1$-$C_{10}$ heterocycloalkenyl group.

The term "$C_6$-$C_{60}$ aryl group" as used herein refers to a monovalent group having a carbocyclic aromatic system having 6 to 60 carbon atoms, and the term "$C_6$-$C_{60}$ arylene group" as used herein refers to a divalent group having a carbocyclic aromatic system having 6 to 60 carbon atoms. Non-limiting examples of the $C_6$-$C_{60}$ aryl group include a phenyl group, a naphthyl group, an anthracenyl group, a phenanthrenyl group, a pyrenyl group, and a chrysenyl group. When the $C_6$-$C_{60}$ aryl group and the $C_6$-$C_{60}$ arylene group each include two or more rings, the rings may be fused to each other.

The term "$C_1$-$C_{60}$ heteroaryl group" as used herein refers to a monovalent group having a heterocyclic aromatic system that has N, O, P, Si, Se, S, or a combination thereof as ring-forming atoms, and 1 to 60 carbon atoms. The term "$C_1$-$C_{60}$ heteroarylene group" as used herein refers to a divalent group having a heterocyclic aromatic system that has N, O, P, Si, Se, S, or a combination thereof as ring-forming atoms, and 1 to 60 carbon atoms. Examples of the $C_1$-$C_{60}$ heteroaryl group include a pyridinyl group, a pyrimidinyl group, a pyrazinyl group, a pyridazinyl group, a triazinyl group, a quinolinyl group, and an isoquinolinyl group. When the $C_1$-$C_{60}$ heteroaryl group and the $C_1$-$C_{60}$ heteroarylene group each include two or more rings, the rings may be fused to each other.

The term "$C_6$-$C_{60}$ aryloxy group" as used herein refers to $-OA_{102}$ (wherein $A_{102}$ is the $C_6$-$C_{60}$ aryl group), and the term "$C_6$-$C_{60}$ arylthio group" as used herein indicates-$SA_{103}$ (wherein $A_{103}$ is the $C_6$-$C_{60}$ aryl group).

The term "$C_1$-$C_{60}$ heteroaryloxy group" as used herein refers to $-OA_{104}$ (wherein $A_{104}$ is the $C_1$-$C_{60}$ heteroaryl

group), and the term "$C_6$-$C_{60}$ arylthio group" as used herein indicates -$SA_{105}$ (wherein $A_{105}$ is the $C_1$-$C_{60}$ heteroaryl group).

The term "$C_7$-$C_{60}$ arylalkyl group" as used herein refers to an aryl group linked to an alkyl group. An example of a $C_7$ arylalkyl group is a benzyl group.

The term "$C_2$-$C_{60}$ heteroarylalkyl group" as used herein refers to a heteroaryl group linked to an alkyl group. An example of a $C_7$ heteroarylalkyl group is a -$CH_2$-pyridine group.

The term "monovalent non-aromatic condensed polycyclic group" used herein refers to a monovalent group in which two or more rings are condensed with each other, only carbon is used as a ring-forming atom (for example, the number of carbon atoms may be 8 to 60) and the entire group is non-aromatic. Examples of the monovalent non-aromatic condensed polycyclic group include a fluorenyl group. The term "divalent non-aromatic condensed polycyclic group" as used herein refers to a divalent group having the same structure as the monovalent non-aromatic condensed polycyclic group.

The term "monovalent non-aromatic condensed heteropolycyclic group" as used herein refers to a monovalent group having two or more rings condensed to each other, N, O, P, Si, Se, S, or a combination thereof as ring forming atoms in addition to carbon atoms (for example, having 2 to 60 carbon atoms), as ring-forming atoms, and the entire group is not aromatic. Non-limiting examples of the monovalent non-aromatic condensed heteropolycyclic group include a carbazolyl group. The term "divalent non-aromatic condensed heteropolycyclic group" as used herein refers to a divalent group having the same structure as the monovalent non-aromatic condensed heteropolycyclic group.

The term "$C_5$-$C_{60}$ carbocyclic group" as used herein refers to a saturated or unsaturated cyclic group having, as a ring-forming atom, 5 to 60 carbon atoms only. The $C_5$-$C_{60}$ carbocyclic group may be a monocyclic group or a polycyclic group, and depending on the structure of formula, may be a monovalent, bivalent, trivalent, tetravalent, pentavalent, or hexavalent group.

The term "$C_2$-$C_{60}$ heterocyclic group" as used herein refers to a saturated or unsaturated cyclic group having, as ring-forming atoms, N, O, Si, P, Se, S, or a combination thereof other than 2 to 60 carbon atoms. The $C_2$-$C_{60}$ heterocyclic group may be a monocyclic group or a polycyclic group, and depending on the structure of formula, may be a monovalent, bivalent, trivalent, tetravalent, pentavalent, or hexavalent group.

Substituents of the substituted $C_5$-$C_{60}$ carbocyclic group, the substituted $C_2$-$C_{60}$ heterocyclic group, the substituted $\pi$ electron-deficient nitrogen-containing $C_2$-$C_{60}$ heterocyclic group, the substituted $C_1$-$C_{60}$alkyl group, the substituted $C_2$-$C_{60}$ heterocyclic group, the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ aryl group, the substituted $C_6$-$C_{60}$ aryloxy group, the substituted $C_6$-$C_{60}$ arylthio group, the substituted $C_1$-$C_{60}$ heteroaryl group, the substituted monovalent non-aromatic condensed polycyclic group, and the substituted monovalent non-aromatic condensed heteropolycyclic group may be:

deuterium, -F, -Cl, -Br, -I, -$CD_3$, -$CD_2H$, -$CDH_2$, -$CF_3$, -$CF_2H$, -$CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, or a $C_1$-$C_{60}$ alkoxy group;

a $C_1$-$C_{60}$ alkyl group, a $C_2$-$C_{60}$ alkenyl group, a $C_2$-$C_{60}$ alkynyl group, or a $C_1$-$C_{60}$ alkoxy group, each substituted with deuterium, -F, -Cl, -Br, -I, -$CD_3$, -$CD_2H$, -$CDH_2$, -$CF_3$, -$CF_2H$, -$CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group,-$N(Q_{11})(Q_{12})$, -$Si(Q_{13})(Q_{14})(Q_{15})$, -$B(Q_{16})(Q_{17})$, -$P(=O)(Q_{18})(Q_{19})$, or a combination thereof;

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, or a monovalent non-aromatic condensed heteropolycyclic group;

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, or a monovalent non-aromatic condensed heteropolycyclic group, each substituted with deuterium, -F, -Cl, -Br, -I, -$CD_3$, -$CD_2H$, -$CDH_2$, -$CF_3$, -$CF_2H$, -$CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed

polycyclic group, a monovalent non-aromatic condensed heteropolycyclic group, -N($Q_{21}$)($Q_{22}$),-Si($Q_{23}$)($Q_{24}$)($Q_{25}$), -B($Q_{26}$)($Q_{27}$), -P(=O)($Q_{28}$)($Q_{29}$), or a combination thereof; or
-N($Q_{31}$)($Q_{32}$), -Si($Q_{33}$)($Q_{34}$)($Q_{35}$), -B($Q_{36}$)($Q_{37}$), or -P(=O)($Q_{38}$)($Q_{39}$),

wherein $Q_1$ to $Q_9$, $Q_{11}$ to $Q_{19}$, $Q_{21}$ to $Q_{29}$, and $Q_{31}$ to $Q_{39}$ are each independently hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, an unsubstituted $C_6$-$C_{60}$ aryl group, a $C_6$-$C_{60}$ aryl group substituted with a $C_1$-$C_{60}$ alkyl group, a $C_6$-$C_{60}$ aryl group, or a combination thereof, a $C_6$-$C_{60}$ aryloxy group, a $C_6$-$C_{60}$ arylthio group, a $C_1$-$C_{60}$ heteroaryl group, a monovalent non-aromatic condensed polycyclic group, or a monovalent non-aromatic condensed heteropolycyclic group.

The "room temperature" used herein refers to the temperature of about 25°C.

Hereinafter, a compound and an organic light-emitting device according to embodiments are described in detail with reference to the Synthesis Example and Examples. However, the organic light-emitting device is not limited thereto. The wording "B was used instead of A" used in describing Synthesis Examples means that an amount of A used was identical to an amount of B used, in terms of a molar equivalent.

[Examples]

Synthesis Example 1: Synthesis of Compound 5

Compound 5 was synthesized according to the following reaction scheme.

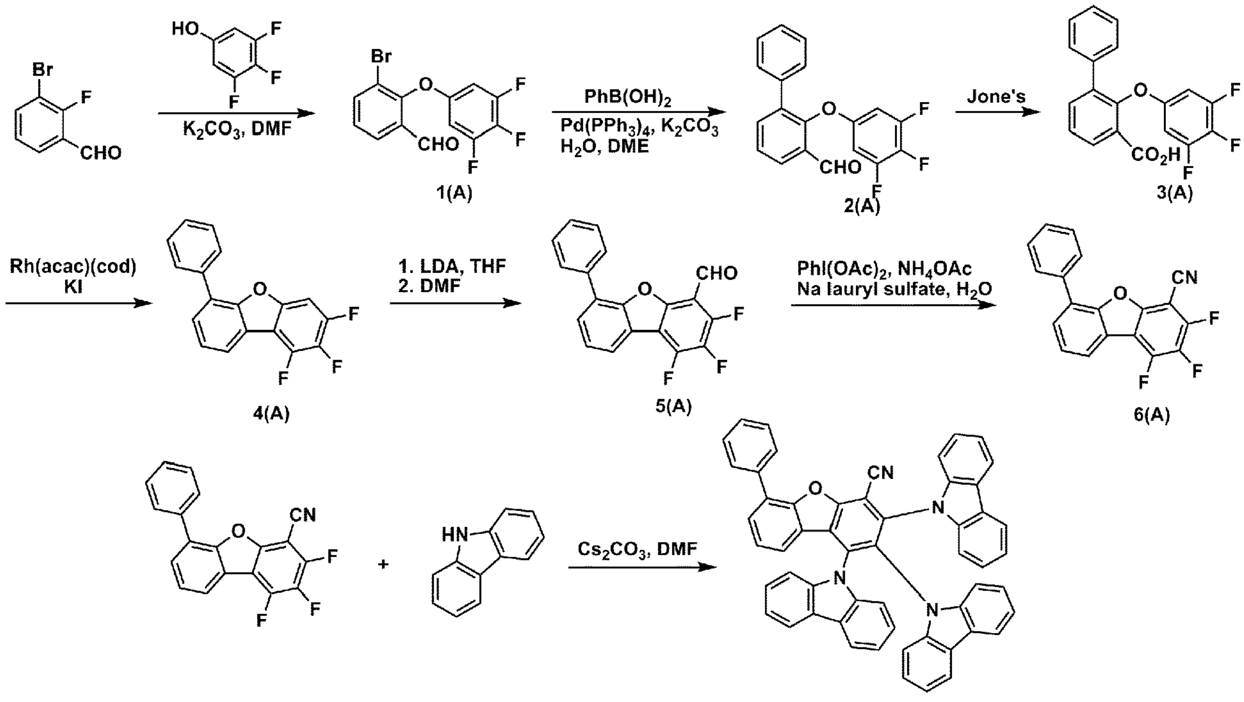


5

(1) Synthesis of Intermediate 1(A)

3-bromo-2-fluorobenzaldehyde (4 g, 24.59 mmol), 3,4,5-trifluorophenol (4.00 g, 27.05 mmol), potassium carbonate ($K_2CO_3$) (3.74 g, 27.05 mmol), and N-methyl-2-pyrrolidinone (NMP) were mixed at a temperature of 200°C for 4 hours. After completion of the reaction, the reaction mixture was cooled to room temperature and then purified by silica gel column chromatography to obtain 8.1 g (yield: 99%) of Intermediate 1(A).

(2) Synthesis of Intermediate 2(A)

3-bromo-2-(3,4,5-trifluorophenoxy)benzaldehyde (8 g, 73.64 mmol), phenylboronic acid (3.24 g, 26.58 mmol), palladium (0) tetrakis(triphenylphosphine) [Pd($PPh_3$)$_4$)] (1.396 g, 1.21 mmol), and potassium carbonate ($K_2CO_3$) (4 g, 29.00 mmol) were added to 60 ml of tetrahydrofuran and 60 ml of distilled water, and the resulting mixture was refluxed. After completion of the reaction, the reaction mixture was cooled to room temperature, and then methanol was added thereto, followed by filtration through silica gel. The resulting organic layer was concentrated, and then methanol was added thereto to perform a precipitation process, thereby obtaining 7.35 g (93%) of Intermediate 2(A) as a white solid.

(3) Synthesis of Intermediate 3(A)

2-(3,4,5-trifluorophenoxy)-[1,1'-biphenyl]-3-carbaldehyde (1 g, 3.05 mmol), Jones reagent (0.54 mL/mmol of reagents), and acetone were mixed at 0°C for 20 minutes. Then, 10 mL of distilled water was added thereto and stirred. After completion of the reaction, the reaction mixture was heated to room temperature and then purified by silica gel column chromatography to obtain 0.87 g (yield: 83%) of Intermediate 3(A).

(4) Synthesis of Intermediate 4(A)

2-(3,4,5-trifluorophenoxy)-[1,1'-biphenyl]-3-carboxylic acid (3 g, 8.71 mmol), potassium iodide (KI) (1.59 g, 9.58 mmol), (acetylacetonato) (1,5-cyclooctadiene)rhodium (I) [RH(acac)(cod)] (0.14 g, 0.44 mmol), and 50 mL of ethyl acetate were mixed and stirred at 100 °C for 1 hour. Then, 100 mL of distilled water was added thereto and stirred. After completion of the reaction, the reaction mixture was cooled to room temperature and then purified by silica gel column chromatography to obtain 2.35 (yield: 90%) of Intermediate 4(A).

(5) Synthesis of Intermediate 5(A)

1,2,3-trifluoro-6-phenyldibenzo[b,d]furan (2 g, 6.71 mmol) was dissolved in tetrahydrofuran, and then, cooled by using dry ice bath to -78°C. Lithium diisopropylamide (LDA) (0.862 g, 8.05 mmol) was slowly added dropwise thereto, and the reaction mixture was stirred at -78°C of 30 minutes. N,N-dimethylformamide (DMF) was added to the reaction container, and then, the temperature was slowly raised to room temperature, followed by 6 hours of stirring. After completion of the reaction, the solvent was removed therefrom, and the reaction mixture was acidified at room temperature for 2 hours by using aqueous sulfuric acid. After completion of the reaction, the reaction mixture was neutralized with aqueous sodium hydroxide and extracted with dichloromethane. The obtained organic layer was concentrated and purified by silica gel column chromatography to obtain 1.68 g (yield: 77%) of Intermediate 5 (A).

(6) Synthesis of Intermediate 6(A)

(Diacetoxyiodo)benzene (0.025 g, 0.08 mmol), ammonium acetate (0.142 g, 1.84 mmol), sodium lauryl sulfate (0.486 g, 1.69 mmol), and 10 mL of DMF were added to 1,2,3-trifluoro-6-phenyldibenzo[b,d]furan-4-carbaldehyde (0.5 g, 1.53 mmol), and then, the resultant mixture was stirred 150°C for 4 hours. After completion of the reaction, the reaction mixture was cooled to room temperature and then purified by silica gel column chromatography to obtain 0.2 g (yield: 40%) of Intermediate 6(A).

(6) Synthesis of Compound 5

Intermediate 6(A) (5.0 g, 15.47 mmol), 9H-carbazole (3.103 g, 18.56 mmol), and cesium carbonate ($Cs_2CO_3$) (7.055 g, 21.65 mmol) were added to 77 ml of DMF, and then, the resultant mixture was stirred at 165°C for 20 hours. After completion of the reaction, the reaction mixture was cooled to room temperature, and then methanol was added thereto, followed by filtration through silica gel. The resulting organic layer was concentrated, the crude residue was dissolved in toluene, filtered through silica gel, and concentrated. Recrystallization (ethyl acetate/ ethanol) was performed thereon to obtain 2.7 g (yield: 23%) of Compound 5 as a yellow solid.
LC- Mass (calculated: 764.26 g/mol, found: 764.36 g/mol (M+1))

Synthesis Example 2: Synthesis of Compound 6

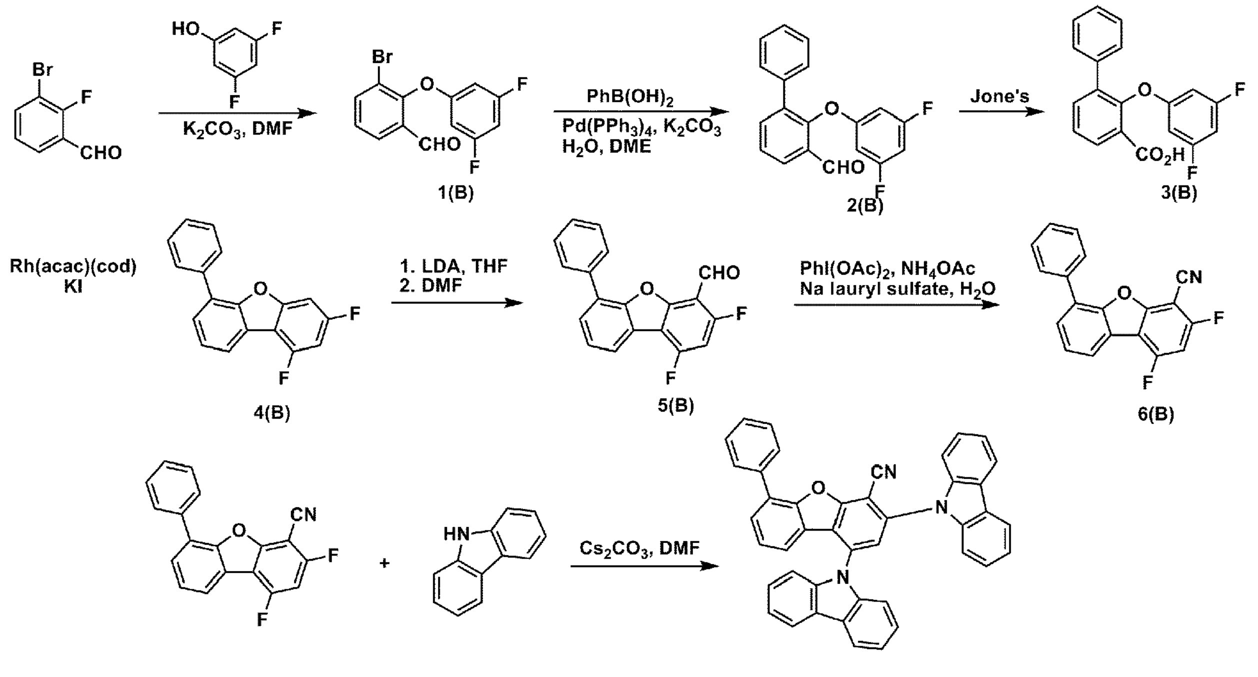


6

Compound 6 was obtained in an amount of 3.5 g (yield: 36%) in the same manner as used to synthesize Compound 5, except that 3,5-difluorophenol was used instead of 3,4,5-trifluorophenol.

LC- Mass (calculated: 599.20 g/mol, found: 599.3 g/mol (M+1))

Synthesis Example 3: Synthesis of Compound 7

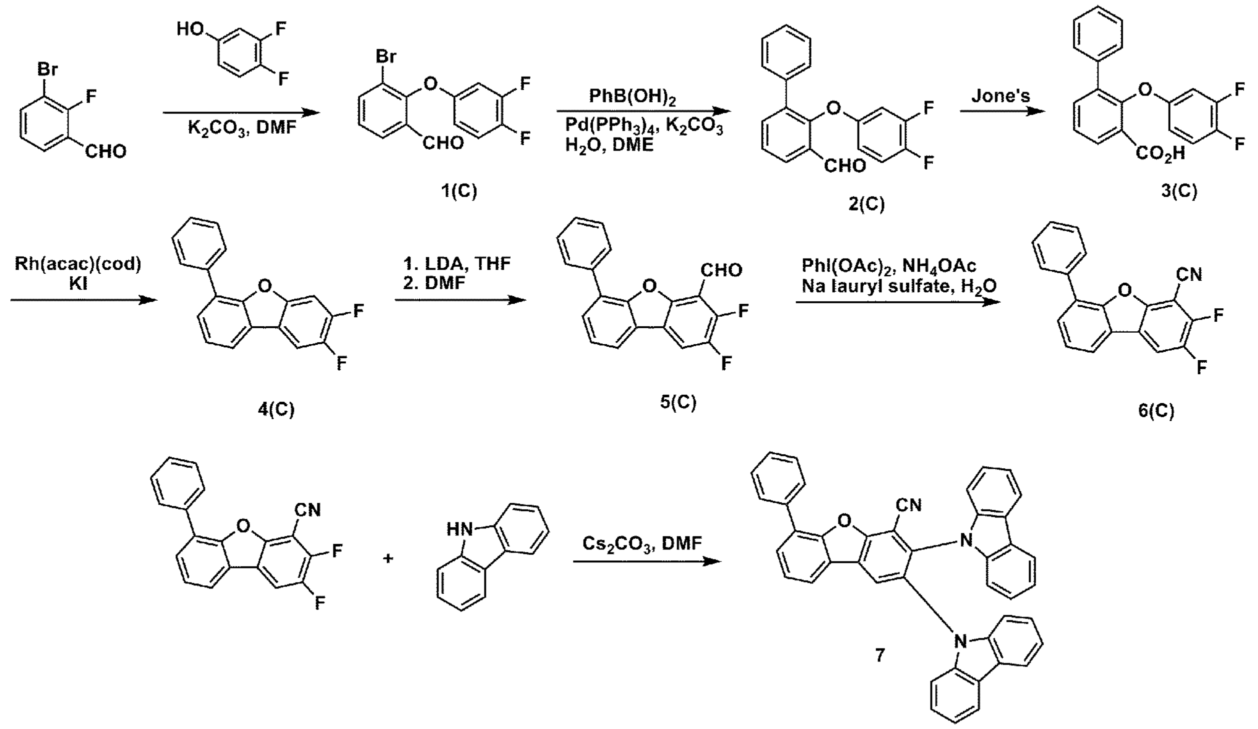


Compound 7 was obtained in an amount of 2.9 g (yield: 30%) in the same manner as used to synthesize

Compound 5, except that 3,4-difluorophenol was used instead of 3,4,5-triflurorophenol.

LC- Mass (calculated: 599.20 g/mol, found: 599.3 g/mol (M+1))

Evaluation Example 1

According to the methods shown in Table 2, the emission spectrum, HOMO, LUMO, singlet ($S_1$) energy level, triplet ($T_1$) energy level, and $\Delta E_{ST}$ of the compounds shown in Table 3 were evaluated. The results are shown in Table 3.

Table 2

| | |
|---|---|
| photoluminescence (PL) spectrum | Each compound was diluted in toluene at the concentration of $10^{-5}$ M, and the PL spectrum was measured (at 298K) by using the Xenon lamp-equipped Hitachi Model No. F7000 Spectrofluorometer |
| $S_1$ energy level evaluation method | The PL spectrum of a mixture of toluene and each compound (diluted to a concentration of $1 \times 10^{-4}$ M) was measured at room temperature by using a PL spectrometer and identified peaks were analyzed to calculate on-set $S_1$ energy level |
| $T_1$ energy level evaluation method | A mixture of toluene and each compound (diluted to a concentration of $1 \times 10^{-4}$ M) was placed in a quartz cell and placed in liquid nitrogen (77 K). The PL spectrum thereof was measured by using a PL spectrometer, and the PL spectrometer was compared with a PL spectrometer measured at room temperature to identify peaks that appeared only at room temperature to calculate on-set $T_1$ energy level. |
| $\Delta E_{ST}$ | Calculated the difference between $S_1$ energy level and $T_1$ energy level. |

Table 3

| Compound No. | HOMO (eV) | LUMO (eV) | $S_1$ energy level (eV) | $T_1$ energy Level (eV) | $\Delta E_{ST}$ (eV) | The maximum emission wavelength (nm) of the PL spectrum |
|---|---|---|---|---|---|---|
| Compound 5 | -6.016 | -2.837 | 3.01 | 2.76 | 0.153 | 412 |
| Compound 6 | -5.77 | -2.092 | 2.883 | 2.653 | 0.32 | 430 |
| Compound 7 | -5.74 | -2.18 | 2.917 | 2.795 | 0.122 | 425 |
| A | -5.66 | -2.14 | 3.19 | 3.0 | 0.19 | 389 |
| B | -5.78 | -2.35 | 3.3 | 2.92 | 0.38 | 376 |

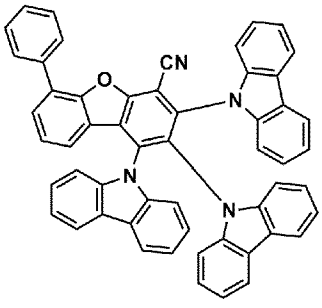

5

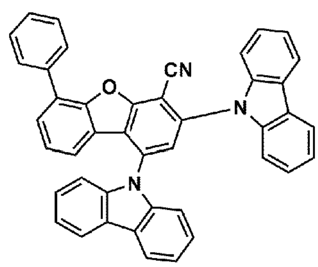

6

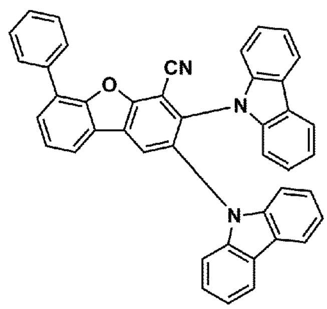

7

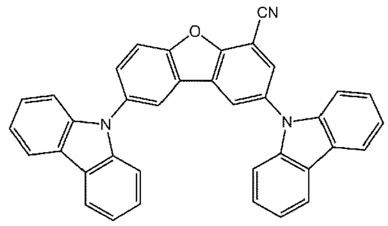

A

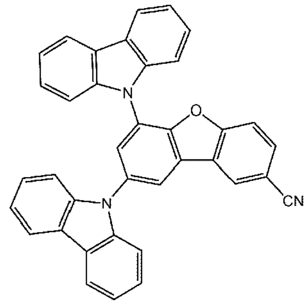

B

From Table 3, it can be seen that Compounds 5 to 7 can emit deep blue light, have a small $\Delta E_{ST}$ and emit thermal activation delayed fluorescence light. Light of Comparative Examples A and B had extremely short wavelengths in the wavelength range of purple, and it would be difficult to utilize them as actual light emitting materials due to their low luminous efficiency.

Evaluation Example 2

Compound H19 and Compound 5 (15% by weight) were co-deposited on a quartz cell to prepare Film 1 having a thickness of 100 Å. Films 2, 3, A, and B were fabricated in the same manner as described above by using Compound 6, 7, A, and B, respectively, instead of Compound 5. Then, Films 1 to 3, A, and B were excited with excitation light having a wavelength of 340 nm under a nitrogen atmosphere by using C9920-02 and PMA-11 of Hamamatsu photonics to measure luminous efficiency (PL yield) of each film, and the results are shown in Table 4 below.

Table 4

| Film No. | Film component | Luminous efficiency (%) |
|---|---|---|
| 1 | Compound 5 + H19 | 17.4 |
| 2 | Compound 6 + H19 | 58.1 |
| 3 | Compound 7 + H19 | 68 |
| A | Compound A + H19 | <1 |
| B | Compound B + H19 | <1 |

From Table 4, it can be seen that Films 1 to 3 have higher luminous efficiencies than Films A and B.

Example 1

A glass substrate, on which a 1500 Å- thick indium tin oxide (ITO) electrode (first electrode, anode) was formed, was cleaned by distilled water ultrasonication. After the distilled water ultrasonication was completed, ultrasonic cleaning was performed sequentially with isopropyl alcohol, acetone, and methanol, once each, and the glass substrate was dried and transferred to a plasma cleaner. The glass substrate was cleaned by using oxygen plasma for 5 minutes, and then transferred to a vacuum laminator.

Compound HT3 was co-deposited on the ITO electrode on the glass substrate to form a first hole injection layer having a thickness of 100 Å, Compound HT-D1 was deposited on the first hole injection layer to form a second hole injection layer having a thickness of 100 Å, and mCP was deposited on the second hole injection layer to form an electron blocking layer having a thickness of 150 Å, thereby completing the formation of a hole transport region.

Compound H19 (host) and Compound 5 (dopant) were co-deposited on the hole transport region at a volumetric ratio of 85:15 to form an emission layer having a thickness of 300 Å.

Compound ET3 was vacuum deposited on the emission layer to form an electron transport layer having a thickness of 300 Å, and then, ET-D1 (Liq) was deposited on the electron transport layer to form an electron injection layer having a thickness of 10 Å, and an Al second electrode (cathode) having a thickness of 1200 Å was formed on the electron injection layer, thereby completing the manufacture of an organic liht-emittin device.

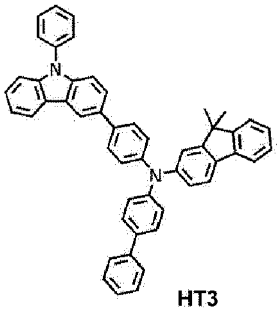
HT3

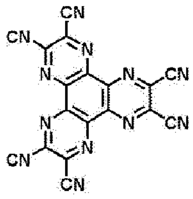
HT-D1

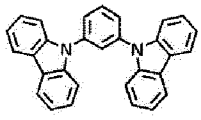
mCP

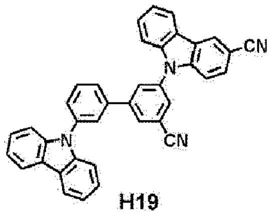
H19

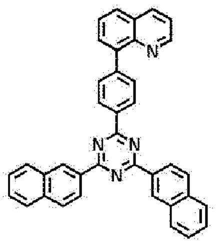
ET3

Examples 2 and 3 and Comparative Examples A and B

Organic light-emitting devices were manufactured in the same manner as in Example 1, except that in forming an emission layer, for use as a dopant, corresponding compounds shown in Table 5 were used instead of Compound 1.

Evaluation example 3

The driving voltage and external quantum efficiency of the organic light-emitting devices manufactured according to Examples 1 to 3 and Comparative Examples A and B were measured by using a current-voltage meter (Keithley 2400) and a luminance meter (Minolta Cs-1000A) at 500 cd/m$^2$. Results thereof are shown in Table 5.

Table 5

| Example No. | Host | Dopant | Driving voltage (V) | External quantum efficiency (Relative value (%)) |
|---|---|---|---|---|
| Example 1 | Compound H19 | Compound 5 | 5.85 | 100 |
| Example 2 | Compound H19 | Compound 6 | 6.99 | 143 |
| Example 3 | Compound H19 | Compound 7 | 8.87 | 170 |
| Comparat ive Example A | Compound H19 | Compound A | 10.61 | 9.8 |
| Comparat ive Example B | Compound H19 | Compound B | 9.65 | 10.5 |

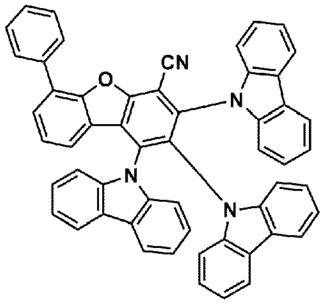

5

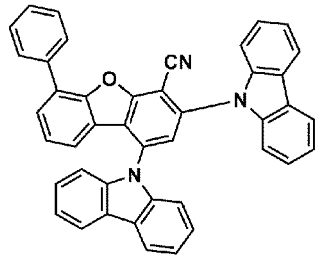

6

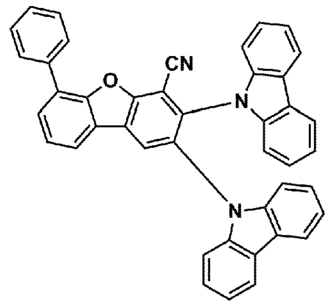

7

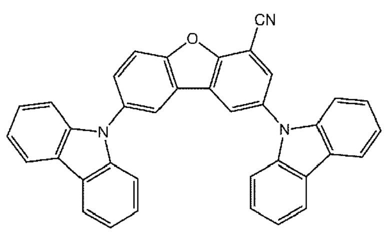

A

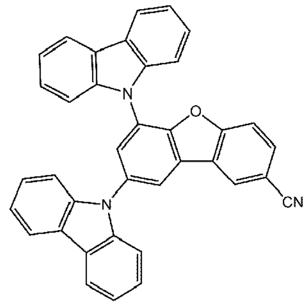

B

From Table 5, it can be seen that the organic light-emitting devices of Examples 1 to 3 have better driving voltage and/or external quantum efficiency characteristics than the organic light-emitting devices of Comparative Examples A and B.

The condensed-cyclic compound has excellent delayed fluorescent emission characteristics, and an organic light-emitting device employing the condensed-cyclic compound may have high efficiency and/or long lifespan.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the scope as defined by the following claims.

## Claims

1. A condensed-cyclic compound represented by Formula 1:

Formula 1

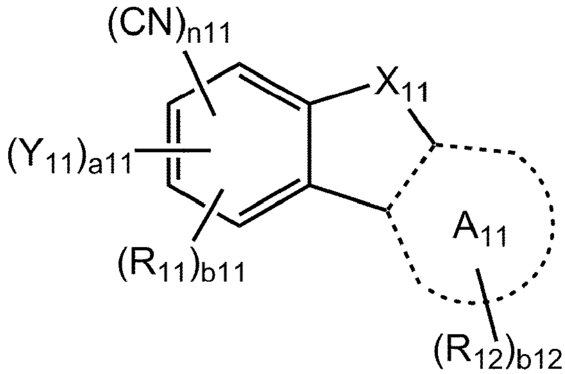

Formula 2-1

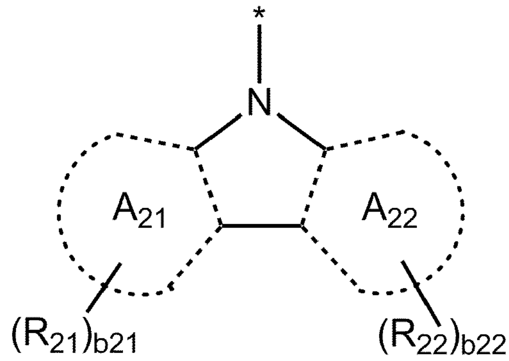

Formula 2-2

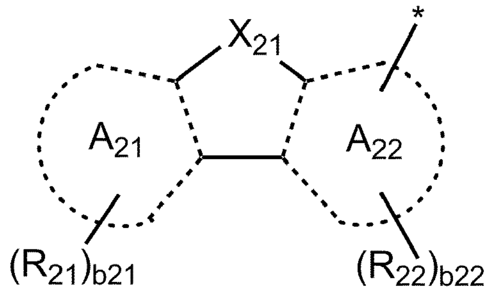

wherein, in Formulae 1, 2-1, and 2-2,

n11 is 1 or 2;
$Y_{11}$ is a group represented by Formulae 2-1 or 2-2;
a11 is 2 or 3;
$X_{11}$ is O, S, $N(R_{16})$, or $C(R_{16})(R_{17})$;
$A_{11}$, $A_{21}$, and $A_{22}$ are each independently a $C_5$-$C_{60}$ carbocyclic group or a $C_1$-$C_{60}$ heterocyclic group;
$X_{21}$ is O, S, $N(R_{23})$, or $C(R_{23})(R_{24})$;
$R_{11}$, $R_{12}$, and $R_{21}$ to $R_{24}$ are each independently hydrogen, deuterium, -F, -Cl, -Br, -I, a hydroxyl group, a cyano group, a nitro group, an amidino group, a hydrazino group, a hydrazono group, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkylaryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkylheteroaryl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, $-Si(Q_1)(Q_2)(Q_3)$, $-B(Q_1)(Q_2)$, $-N(Q_1)(Q_2)$, $-P(Q_1)(Q_2)$, $-C(=O)(Q_1)$, $-S(=O)(Q_1)$, $-S(=O)_2(Q_1)$, $-P(=O)(Q_1)(Q_2)$, or$-P(=S)(Q_1)(Q_2)$,
b11 is 0 or 1;
b12, b21, and b22 are each independently 1, 2, 3, 4, 5, 6, 7, 8, 9, or 10;
the sum of n11, a11, and b11 is 4;
$Q_1$ to $Q_3$ are each independently hydrogen, deuterium, -F, -Cl, -Br, -I, a hydroxyl group, a cyano group, a nitro group, an amidino group, a hydrazino group, a hydrazono group, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_7$-$C_{60}$ alkylaryl group, a $C_6$-$C_{60}$ aryloxy group, a $C_6$-$C_{60}$ arylthio group, a $C_1$-$C_{60}$ heteroaryl group, a $C_2$-$C_{60}$ alkylheteroaryl group, a $C_1$-$C_{60}$ heteroaryloxy group, a $C_1$-$C_{60}$ heteroarylthio group, a monovalent non-aromatic condensed polycyclic group, a monovalent non-aromatic condensed heteropolycyclic group, a $C_1$-$C_{60}$ alkyl group which is substituted with deuterium, -F, a cyano group, a $C_1$-$C_{60}$ alkyl group, a $C_6$-$C_{60}$ aryl group, or a combination thereof, or a $C_6$-$C_{60}$ aryl group which is substituted with deuterium, -F, a cyano group, a $C_1$-$C_{60}$ alkyl group, a $C_6$-$C_{60}$ aryl group, or a combination thereof; and
* indicates a binding site to a neighboring atom.

**2.** The condensed-cyclic compound of claim 1, wherein n11 is 1; and/or
wherein $X_{11}$ is O, S, or $N(R_{16})$; and/or
wherein
$A_{11}$, $A_{21}$, and $A_{22}$ are each independently a benzene group, a naphthalene group, an anthracene group, a phenanthrene group, a phenalene group, a triphenylene group, a pyridine group, a pyrimidine group, a pyrazine group, a pyridazine group, a triazine group, a quinoline group, an isoquinoline group, 2,6-naphthyridine group, 1,8-naphthyridine group, 1,5-naphthyridine group, 1,6-naphthyridine group, 1,7-naphthyridine group, 2,7-naphthyridine group,

a quinoxaline group, a phthalazine group, a quinazoline group, a phenanthroline group, a benzoquinoline group, a benzoisoquinoline group, a benzoquinoxaline group, a benzoquinazoline group, a furan group, a thiophene group, a silole group, an indene group, a fluorene group, an indole group, a carbazole group, a benzofuran group, a dibenzofuran group, a benzothiophene group, a dibenzothiophene group, a benzosilole group, a dibenzosilole group, an indenopyridine group, an indolopyridine group, a benzofuropyridine group, a benzothienopyridine group, a benzosilolopyridine group, an indenopyrimidine group, an indolopyrimidine group, a benzofuropyrimidine group, a benzothienopyrimidine group, or a benzosilolopyrimidine group.

**3.** The condensed-cyclic compound of claims 1 or 2, wherein
$A_{11}$ is a benzene group or a naphthalene group;
$A_{21}$ and $A_{22}$ are each independently a benzene group, a naphthalene group, a fluorene group, a carbazole group, a dibenzofuran group, or a dibenzothiophene group.

**4.** The condensed-cyclic compound of any of claims 1-3, wherein
$R_{11}$, $R_{12}$, and $R_{21}$ to $R_{24}$ are each independently hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, or a $C_1$-$C_{20}$ alkoxy group;
a $C_1$-$C_{20}$ alkyl group or a $C_1$-$C_{20}$ alkoxy group, each substituted with deuterium, - F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group, a cyclopentyl group, a cyclohexyl group, a cycloheptyl group, a cycloctyl group, a bicyclo[2.2.1]heptyl group, an adamantyl group, a norbornyl group, a norbornenyl group, a cyclopentenyl group, a cyclohexenyl group, a cycloheptenyl group, a phenyl group, a biphenyl group, a $C_7$-$C_{20}$ alkylphenyl group, a naphthyl group, a pyridinyl group, a pyrimidinyl group, or a combination thereof;
a cyclopentyl group, a cyclohexyl group, a cycloheptyl group, a cycloctyl group, a bicyclo[2.2.1]heptyl group, an adamantyl group, a norbornyl group, a norbornenyl group, a cyclopentenyl group, a cyclohexenyl group, a cycloheptenyl group, a phenyl group, a biphenyl group, a $C_7$-$C_{20}$ alkylphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a dibenzosilolyl group, a benzocarbazolyl group, a dibenzocarbazolyl group, an imidazopyridinyl group, or an imidazopyrimidinyl group;
a cyclopentyl group, a cyclohexyl group, a cycloheptyl group, a cycloctyl group, a bicyclo[2.2.1]heptyl group, an adamantyl group, a norbornyl group, a norbornenyl group, a cyclopentenyl group, a cyclohexenyl group, a cycloheptenyl group, a phenyl group, a biphenyl group, a $C_7$-$C_{20}$ alkylphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a dibenzosilolyl group, a benzocarbazolyl group, a dibenzocarbazolyl group, an imidazopyridinyl group, or an imidazopyrimidinyl group, each substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a cyclopentyl group, a cyclohexyl group, a cycloheptyl group, a cycloctyl group, a bicyclo[2.2.1]heptyl group, an adamantyl group, a norbornyl group, a norbornenyl group, a cyclopentenyl group, a cyclohexenyl group, a cycloheptenyl group, a phenyl group, a biphenyl group, a $C_7$-$C_{20}$ alkylphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group,

a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a dibenzosilolyl group, a benzocarbazolyl group, a dibenzocarbazolyl group, an imidazopyridinyl group, an imidazopyrimidinyl group, -Si($Q_{11}$)($Q_{12}$)($Q_{13}$), -B($Q_{11}$)($Q_{12}$), -N($Q_{11}$)($Q_{12}$), or a combination thereof; or
-Si($Q_1$)($Q_2$)($Q_3$), -B($Q_1$)($Q_2$), -or N($Q_1$)($Q_2$),
wherein $Q_1$ to $Q_3$ and $Q_{11}$ to $Q_{13}$ are each independently
a methyl group, an ethyl group, an n-propyl group, an isopropyl group, an n-butyl group, an isobutyl group, a sec-butyl group, a tert-butyl group, an n-pentyl group, an isopentyl group, a 2-methylbutyl group, a sec-pentyl group, a tert-pentyl group, a neopentyl group, 3-pentyl group, 3-methyl-2-butyl group, a phenyl group, a biphenyl group, a $C_7$-$C_{20}$ alkylphenyl group, or a naphthyl group; or
a methyl group, an ethyl group, an n-propyl group, an isopropyl group, an n-butyl group, an isobutyl group, a sec-butyl group, a tert-butyl group, an n-pentyl group, an isopentyl group, a 2-methylbutyl group, a sec-pentyl group, a tert-pentyl group, a neopentyl group, 3-pentyl group, 3-methyl-2-butyl group, a phenyl group, or a naphthyl group, each substituted with deuterium, a phenyl group, or a combination thereof.

**5.** The condensed-cyclic compound of any of claims 1-4, wherein
$Y_{11}$ is a group represented by one of Formulae 2-11 to 2-17:

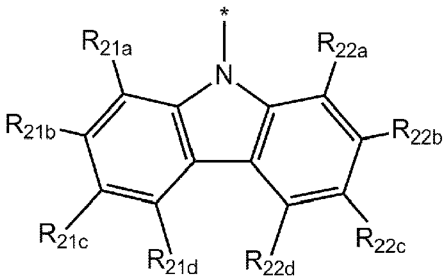

2-11

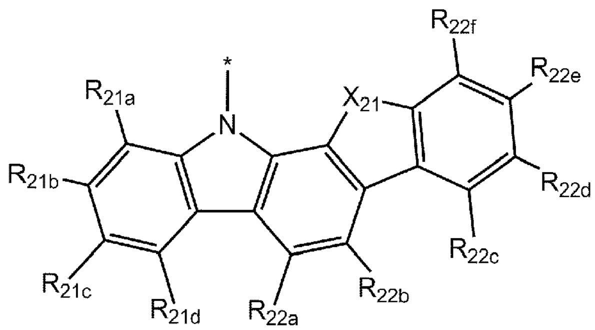

2-12

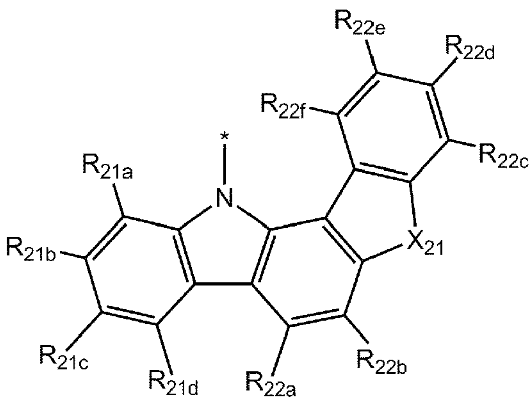

2-13

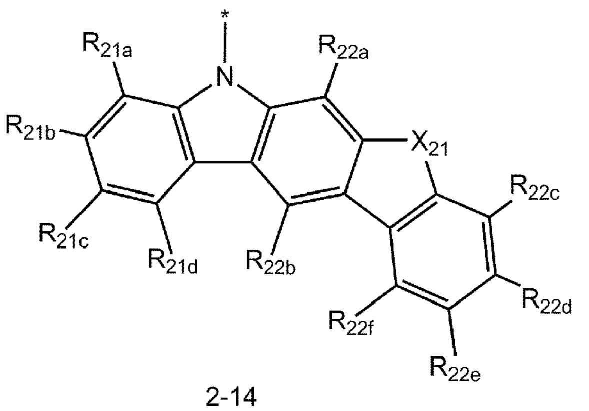

2-14

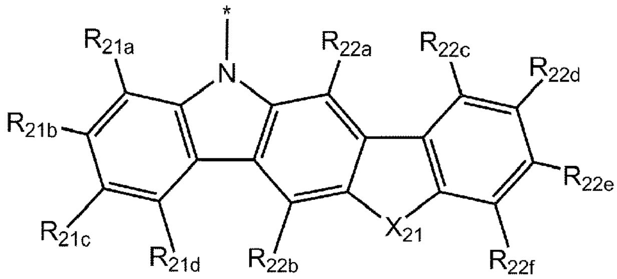

2-15

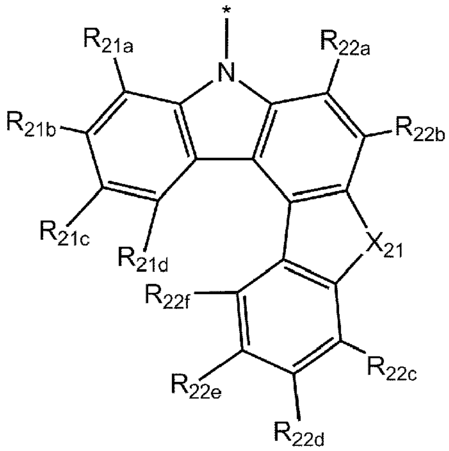

2-16

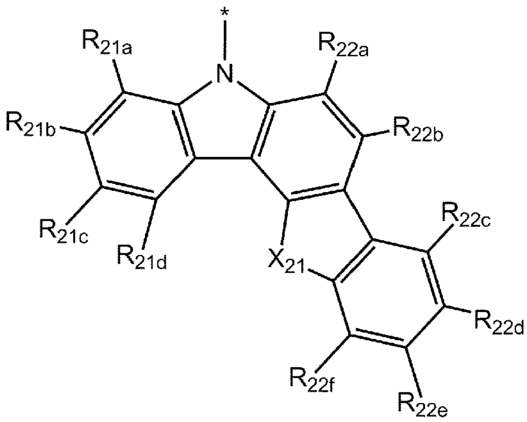

2-17

wherein, in Formulae 2-11 to 2-17,

$X_{21}$ is O, S, $N(R_{22g})$, or $C(R_{22g})(R_{22h})$;
$R_{21a}$ to $R_{21d}$ are understood by referring to the definition of $R_{21}$ in claim 1;
$R_{22a}$ to $R_{22h}$ are understood by referring to the definition of $R_{22}$ in claim 1; and
* indicates a binding site to a neighboring atom.

**6.** The condensed-cyclic compound of any of claims 1-5, wherein the condensed-cyclic compound is represented by one of Formulae 1-1 to 1-6:

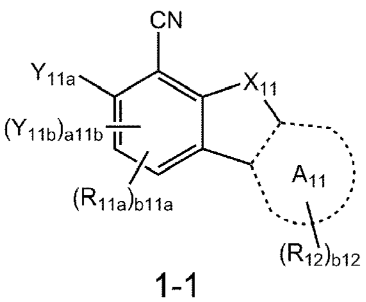

1-1

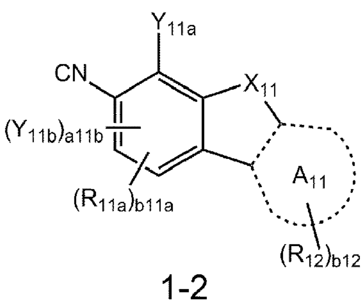

1-2

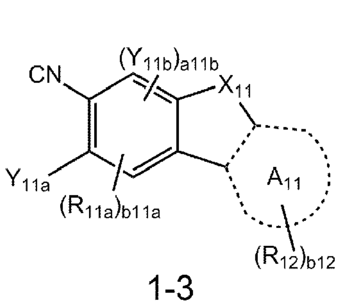

1-3

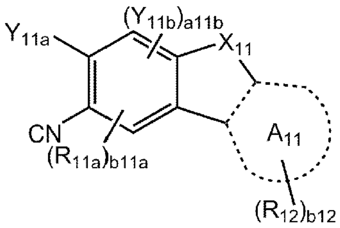

1-4

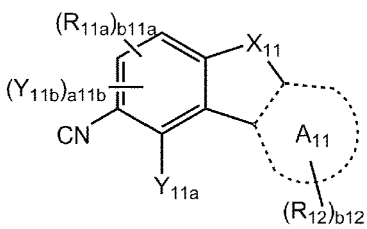

1-5

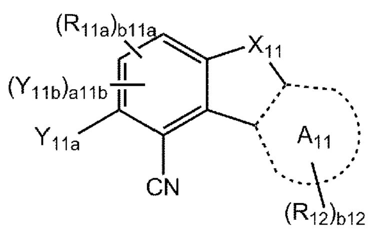

1-6

wherein, in Formulae 1-1 to 1-6,

$Y_{11a}$ and $Y_{11b}$ are each understood by referring to the definition of $Y_{11}$ in claim 1, $R_{11a}$ is understood by referring to the definition of $R_{11}$ in claim 1,
a11b is 1 or 2;
$X_{11}$, $A_{11}$, $R_{12}$, and b12 are each understood by referring to claim 1,
$A_{21}$, $A_{22}$, $X_{21}$, $R_{21}$ to $R_{24}$, b21, and b22 are each understood by referring to claim 1, and
the sum of a11 b and b11a is 3.

**7.** The condensed-cyclic compound of claim 6, wherein
$A_{11}$ is a benzene group.

**8.** The condensed-cyclic compound of claims 6 or 7, wherein
$Y_{11}$ is represented by one of Formulae 2-11 to 2-17:

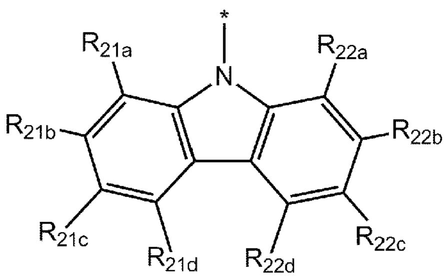

2-11

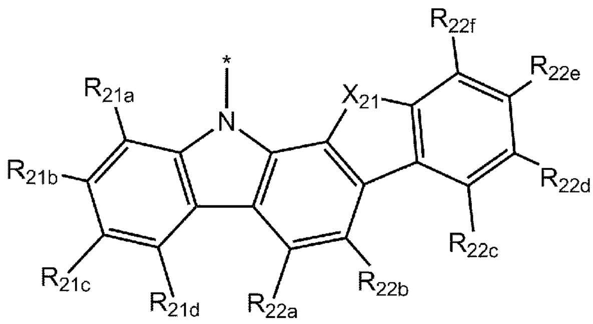

2-12

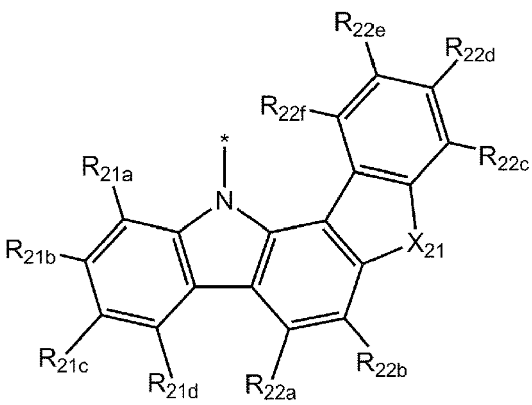

2-13

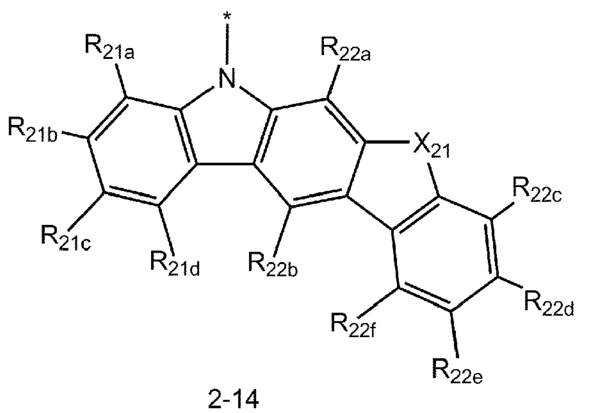

2-14

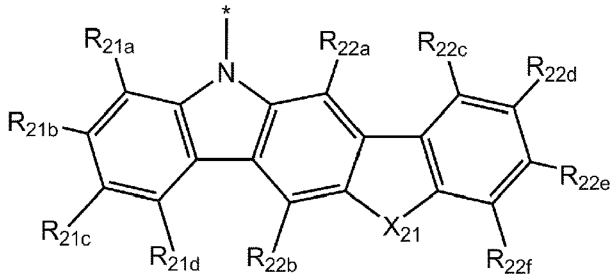

2-15

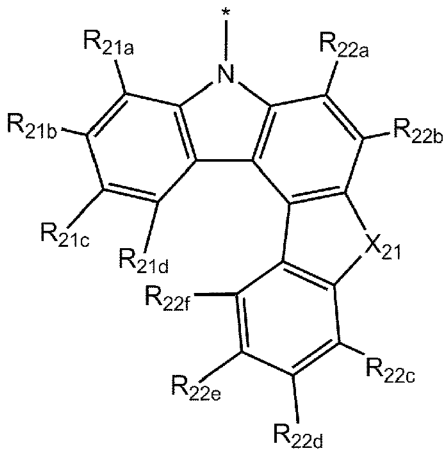

2-16

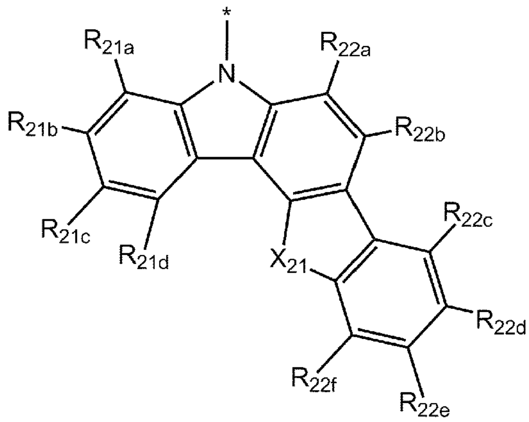

2-17

wherein, in Formulae 2-11 to 2-17,

$X_{21}$ is O, S, N($R_{22g}$), or C($R_{22g}$)($R_{22h}$);
$R_{21a}$ to $R_{21d}$ are understood by referring to the definition of $R_{21}$ in claim 1;
$R_{22a}$ to $R_{22h}$ are understood by referring to the definition of $R_{22}$ in claim 1; and
* indicates a binding site to a neighboring atom.

**9.** The condensed-cyclic compound of any of claims 1-8, wherein the condensed-cyclic compound is represented by one of Formulae 1-11 to 1-23:

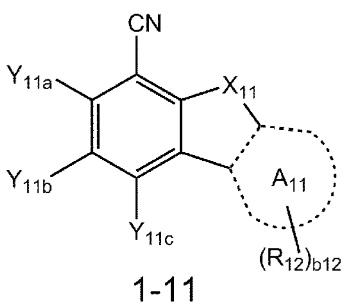

1-11

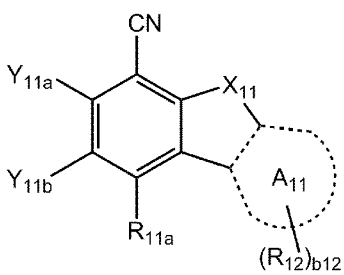

1-12

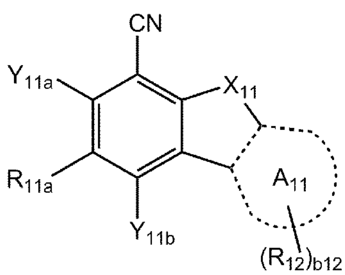

1-13

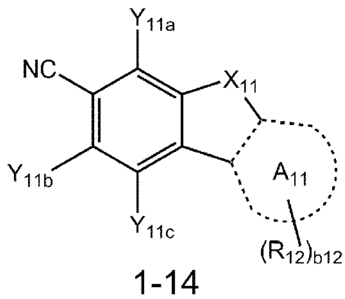

1-14

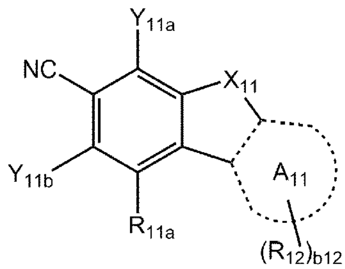

1-15

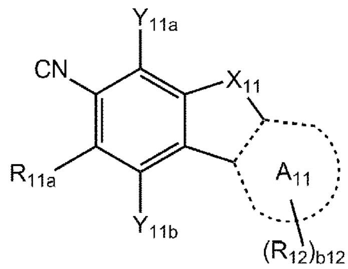

1-16

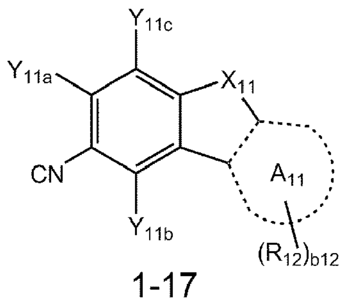

1-17

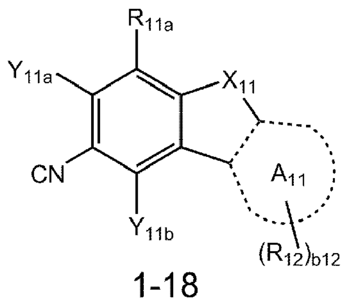

1-18

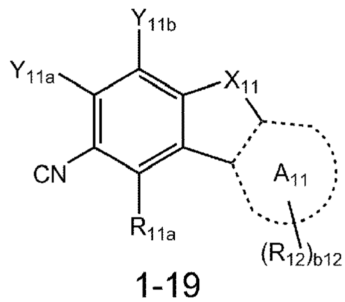

1-19

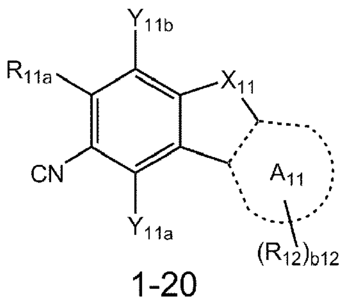

1-20

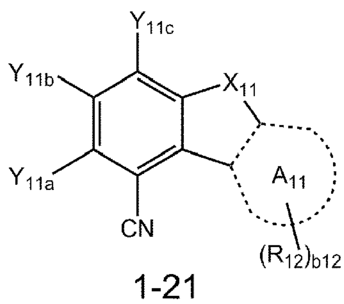

1-21

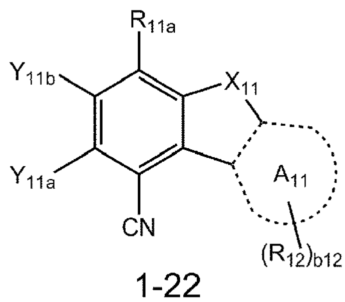

1-22

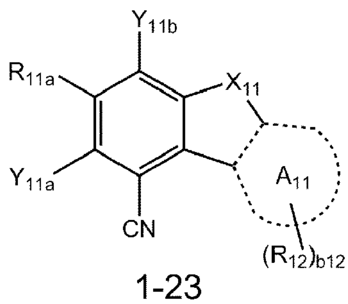

1-23

wherein, in Formulae 1-11 to 1-23,

$Y_{11a}$, $Y_{11b}$, and $Y_{11c}$ are each understood by referring to the definition of $Y_{11}$ in claim 1,
$R_{11a}$ is understood by referring to the definition of $R_{11}$ in claim 1,
$X_{11}$, $A_{11}$, $R_{12}$, and b12 are each understood by referring to claim 1, and
$A_{21}$, $A_{22}$, $X_{21}$, $R_{21}$ to $R_{24}$, b21, and b22 are each understood by referring to claim 1.

**10.** The condensed-cyclic compound of claim 1, wherein the condensed-cyclic compound is Compounds 1 to 336:

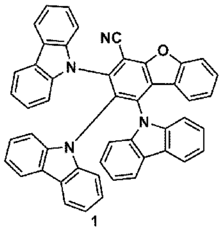
1

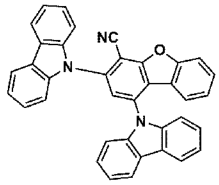
2

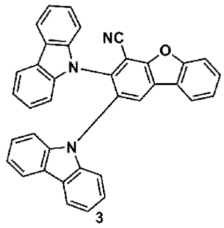
3

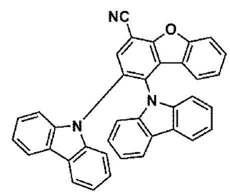
4

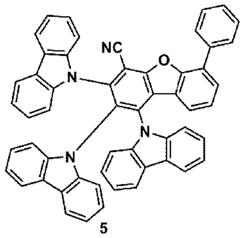
5

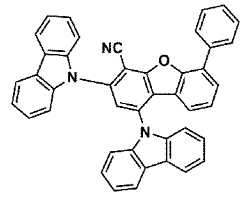
6

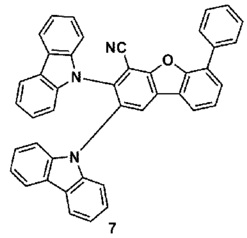
7

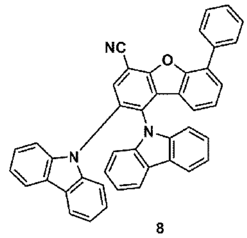
8

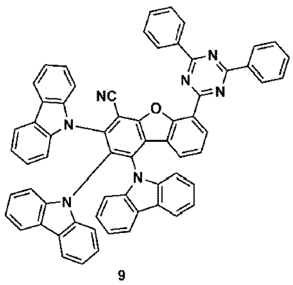
9

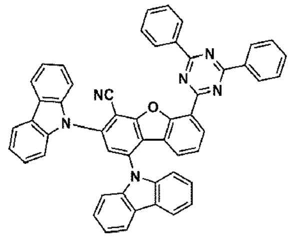
10

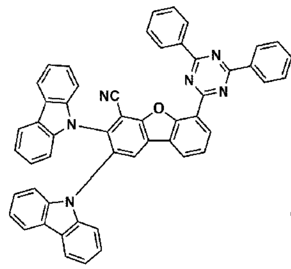
11

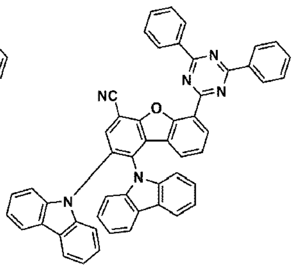
12

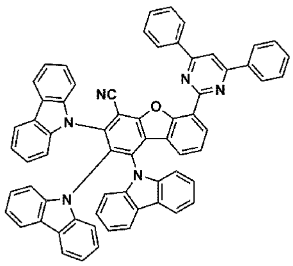
13

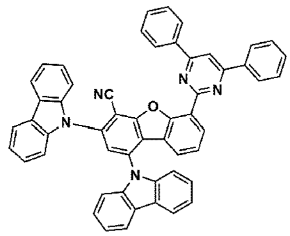
14

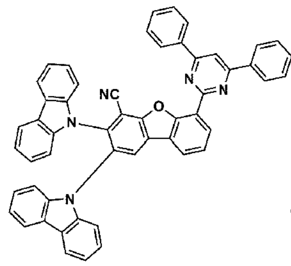
15

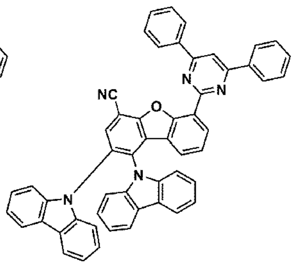
16

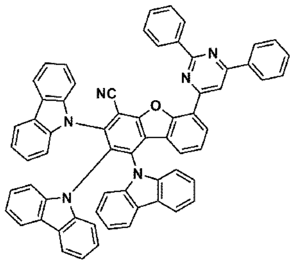
17

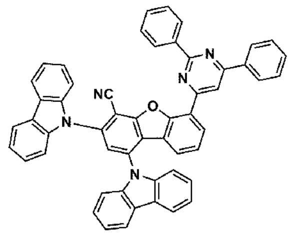
18

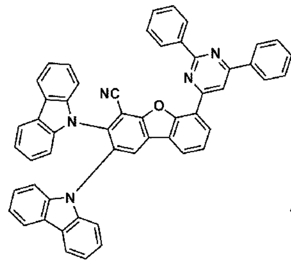
19

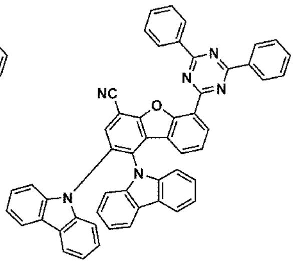
20

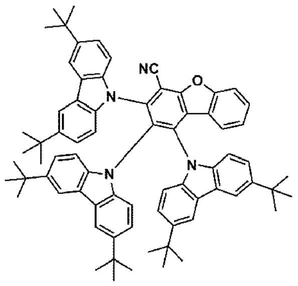

21

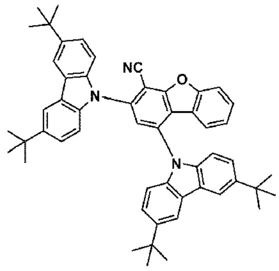

22

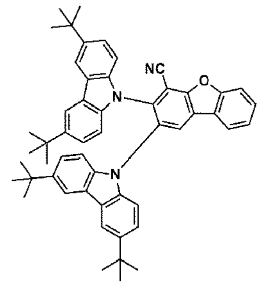

23

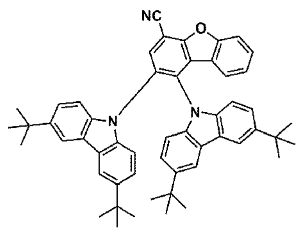

24

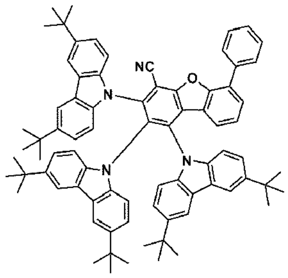

25

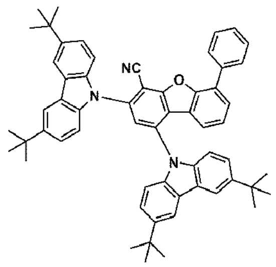

26

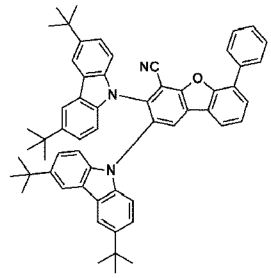

27

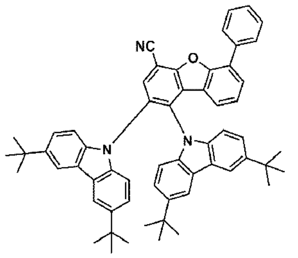

28

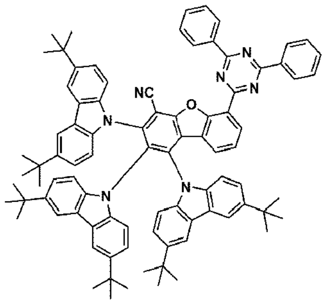

29

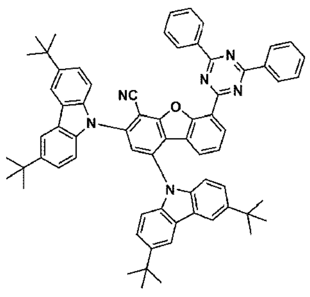

30

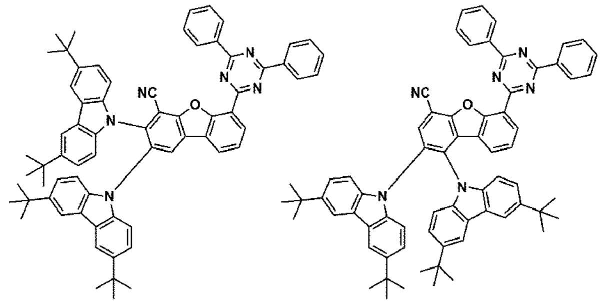

31

32

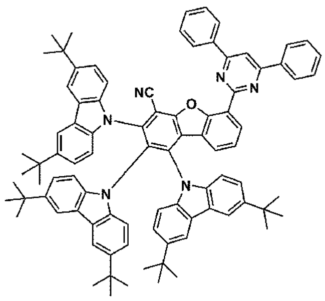

33

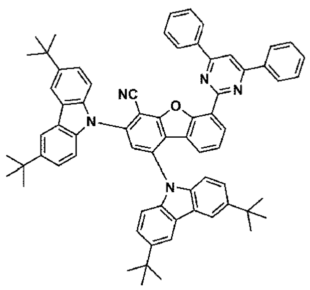

34

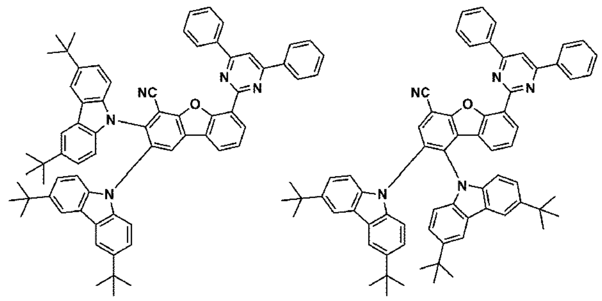

35

36

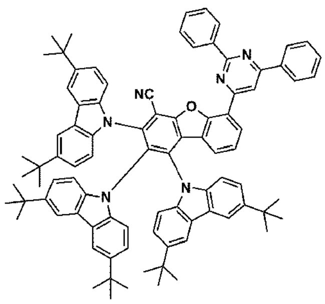 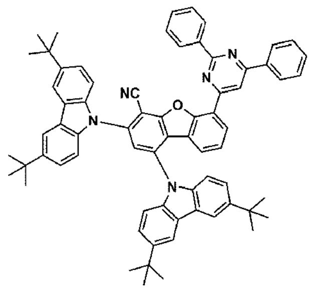 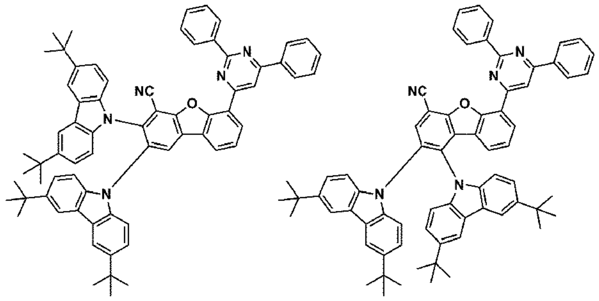

37 38 39 40

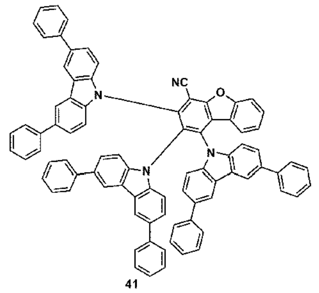 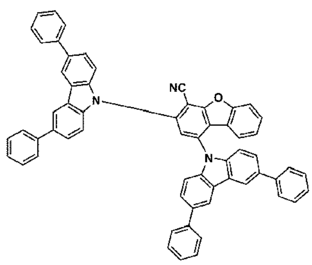 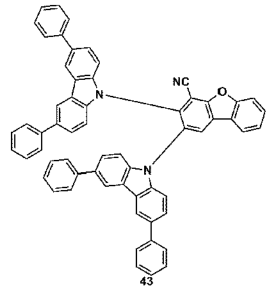 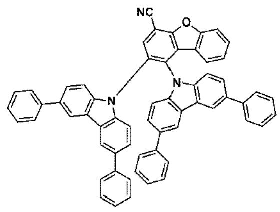

41 42 43 44

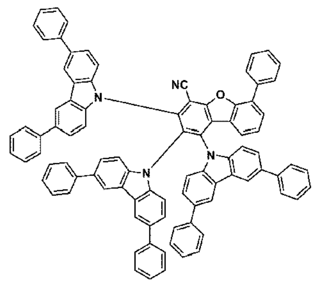 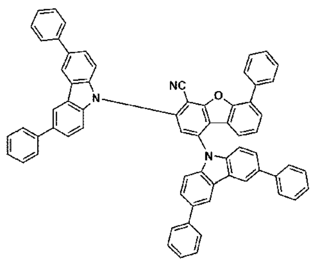 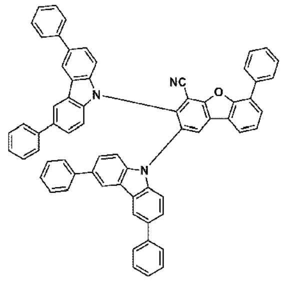 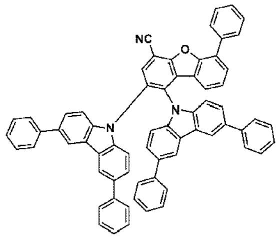

45 46 47 48

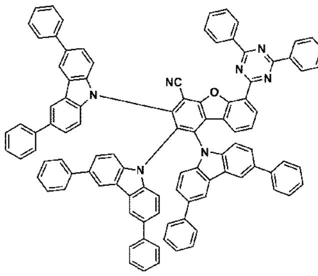 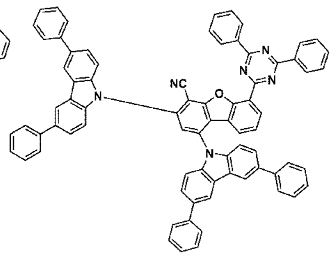 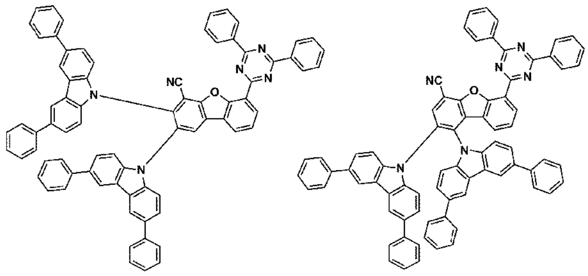

49 50 51 52

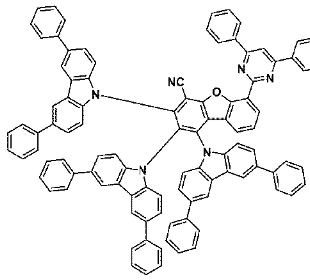

53

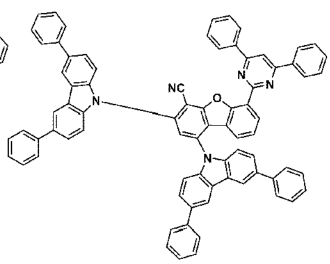

54

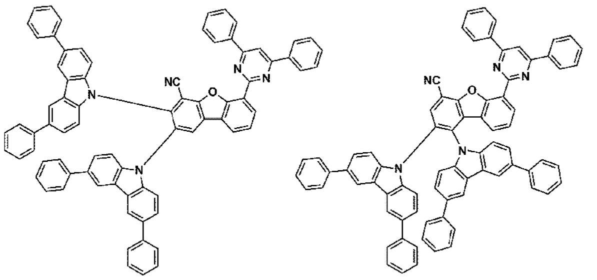

55

56

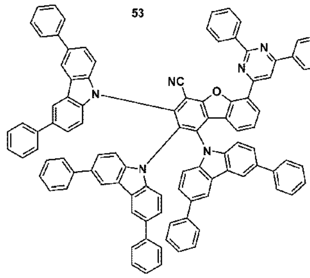

57

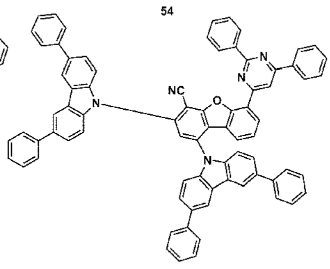

58

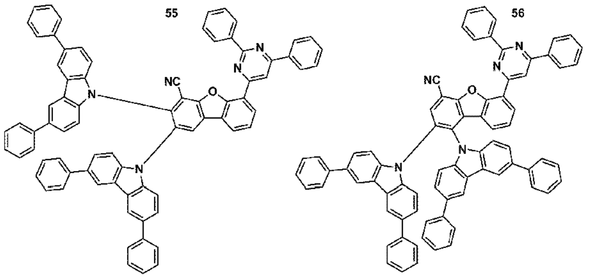

59

60

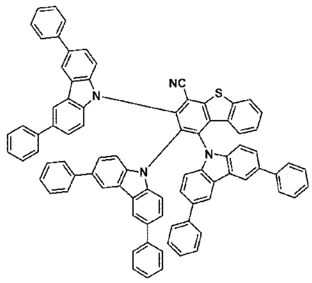

61

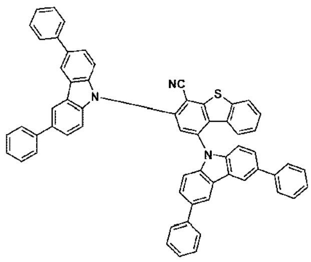

62

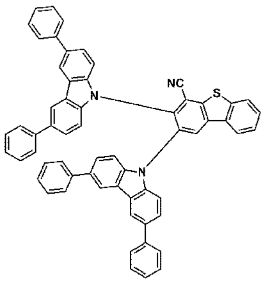

63

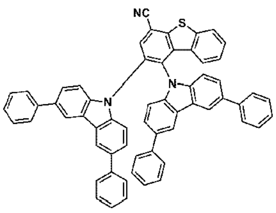

64

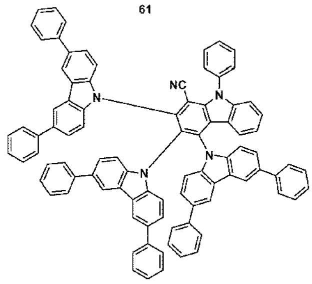

65

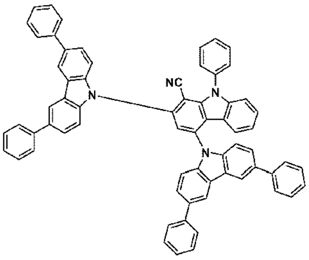

66

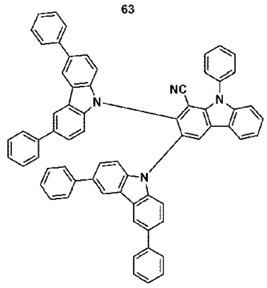

67

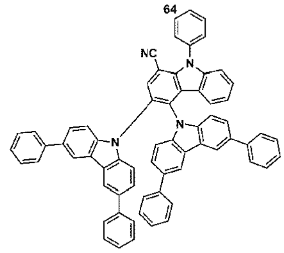

68

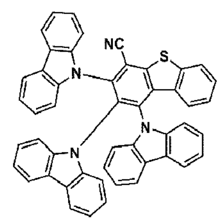

69

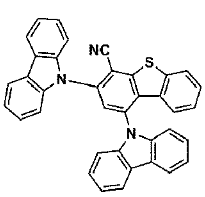

70

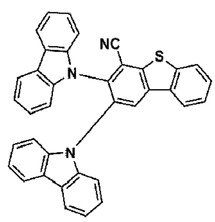

71

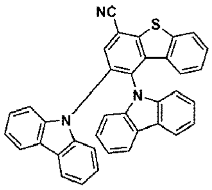

72

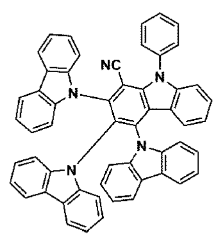
73

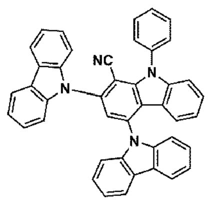
74

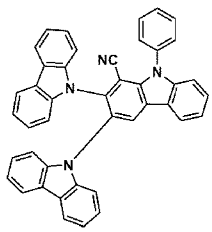
75

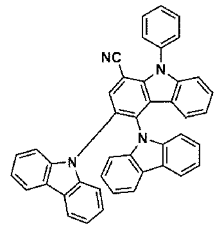
76

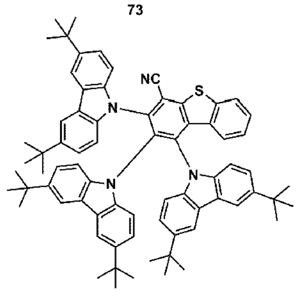
77

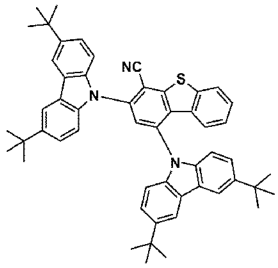
78

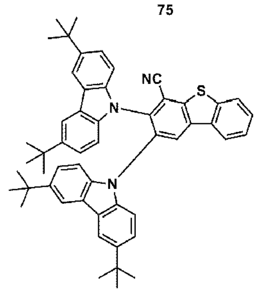
79

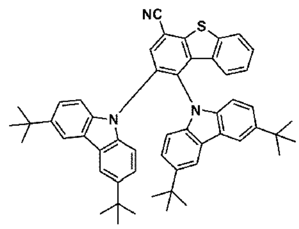
80

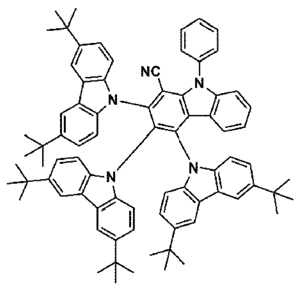
81

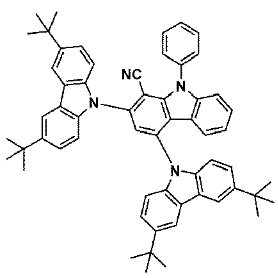
82

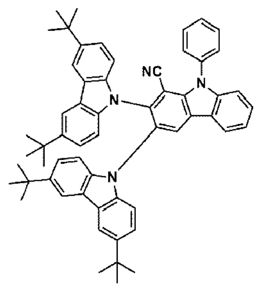
83

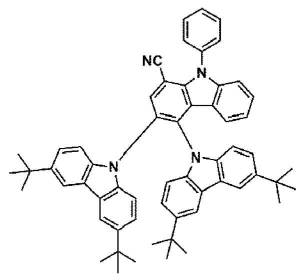
84

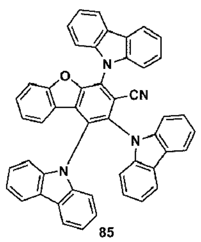
85

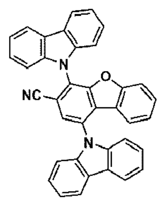
86

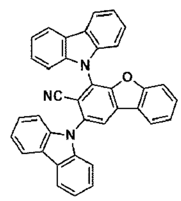
87

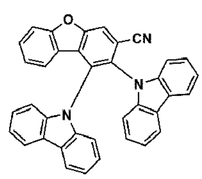
88

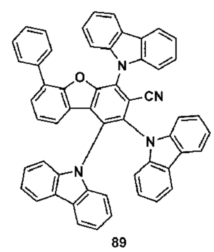
89

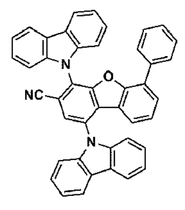
90

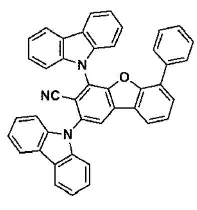
91

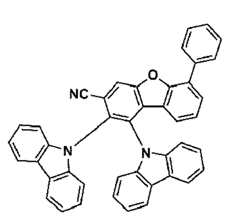
92

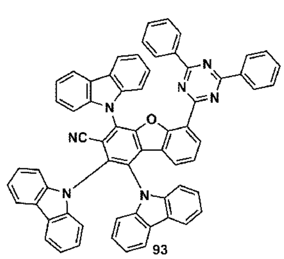
93

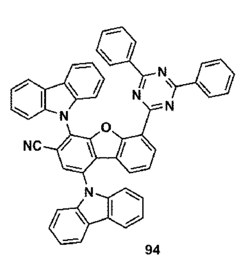
94

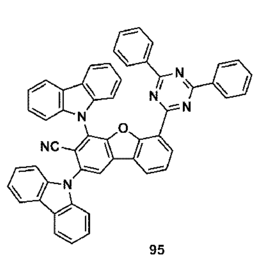
95

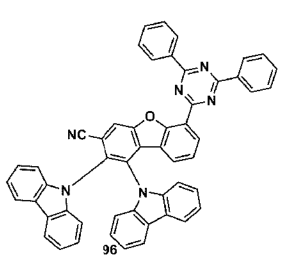
96

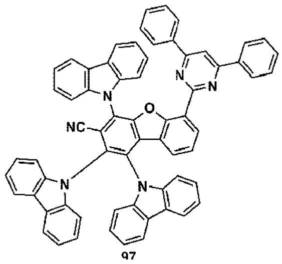
97

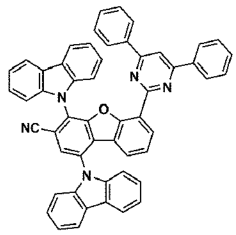
98

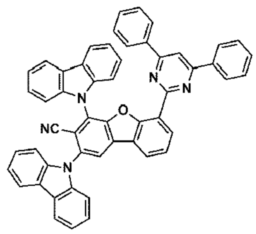
99

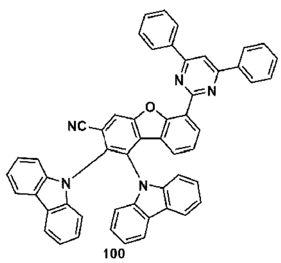
100

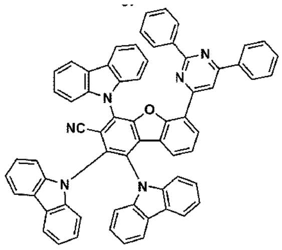
101

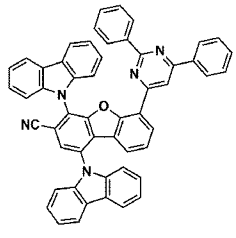
102

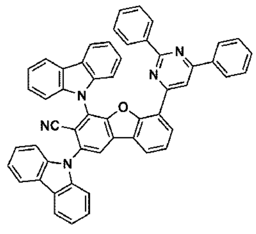
103

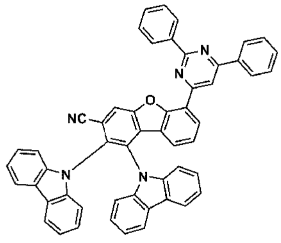
104

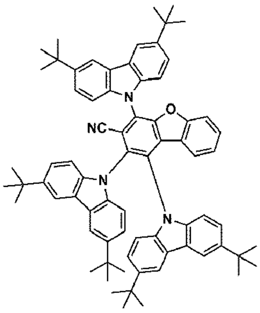
105

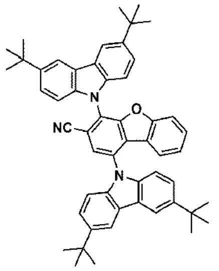
106

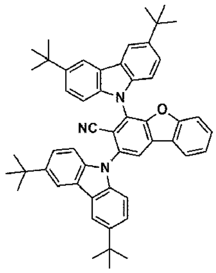
107

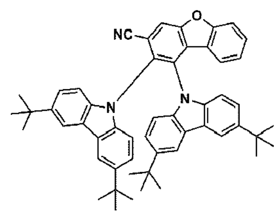
108

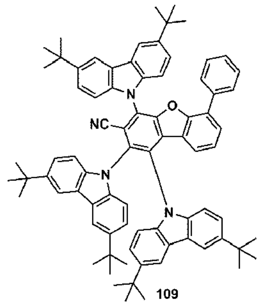
109

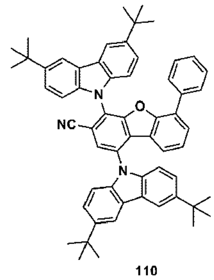
110

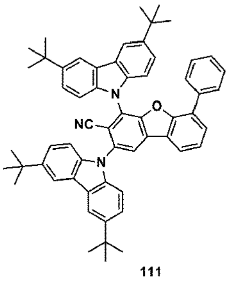
111

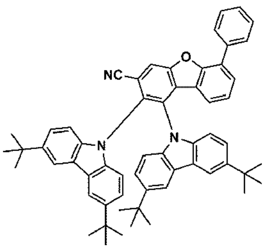
112

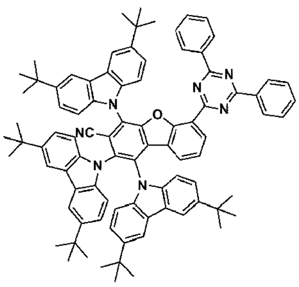
113

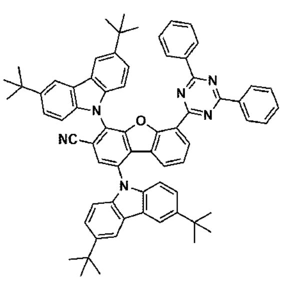
114

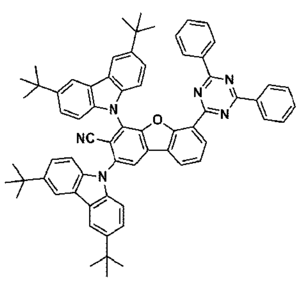
115

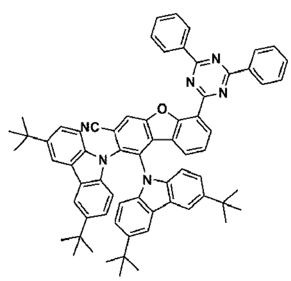
116

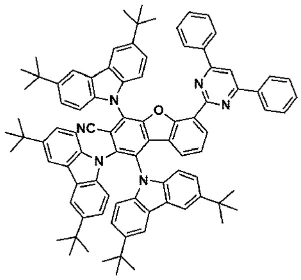

117

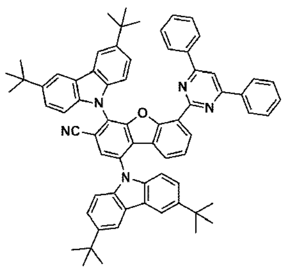

118

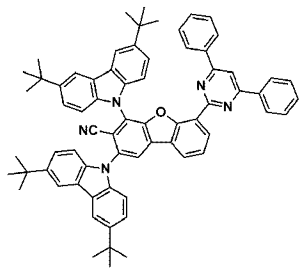

119

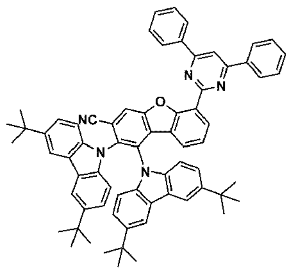

120

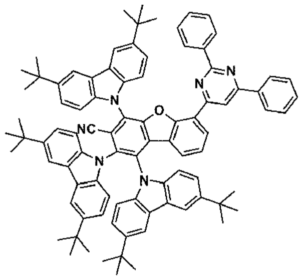

121

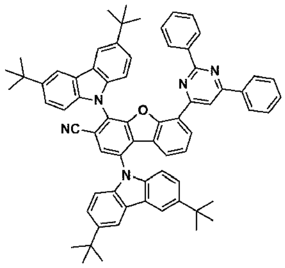

122

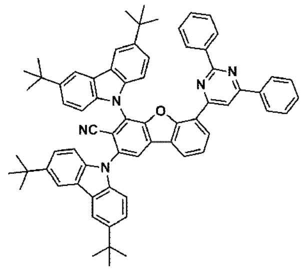

123

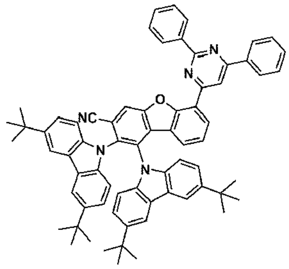

124

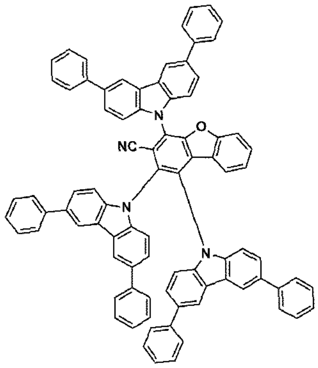

125

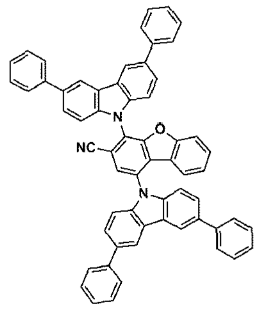

126

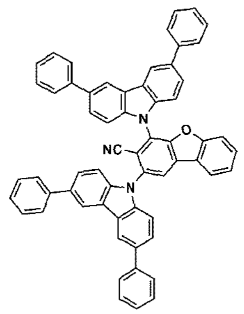

127

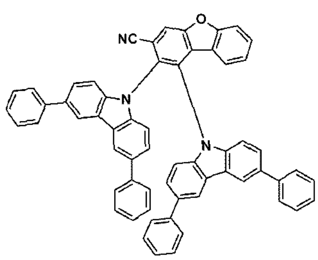

128

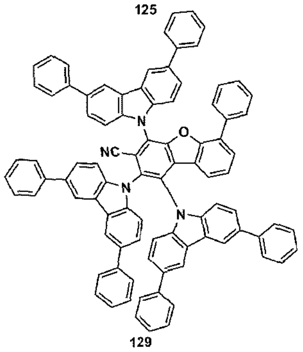

129

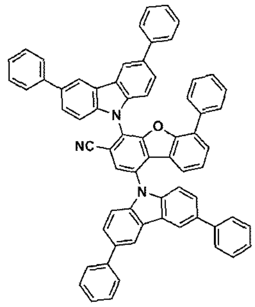

130

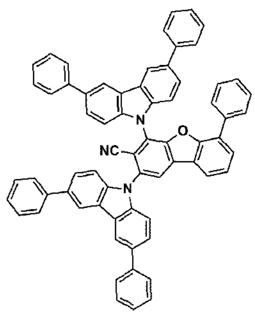

131

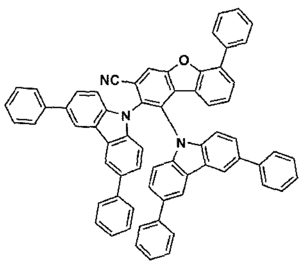

132

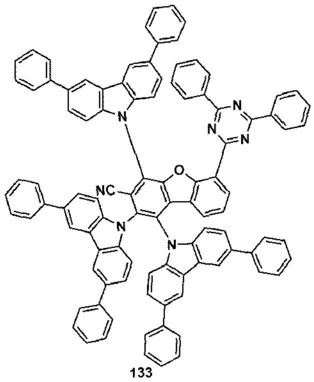
133

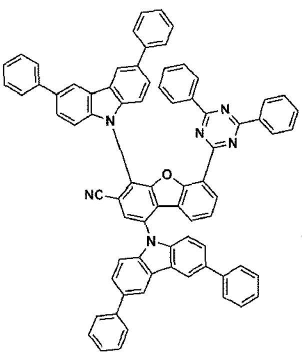
134

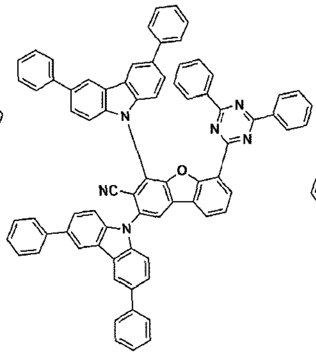
135

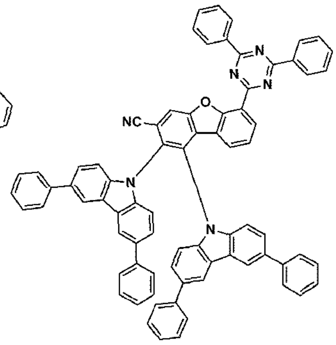
136

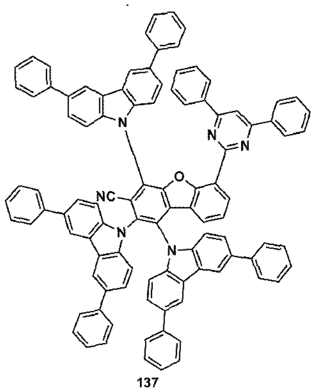
137

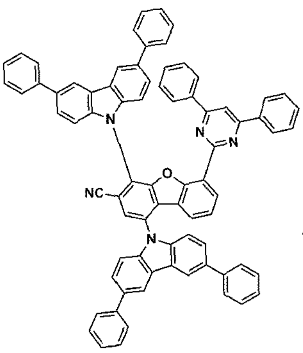
138

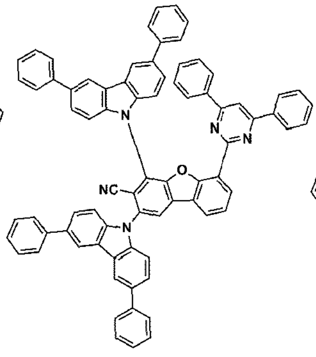
139

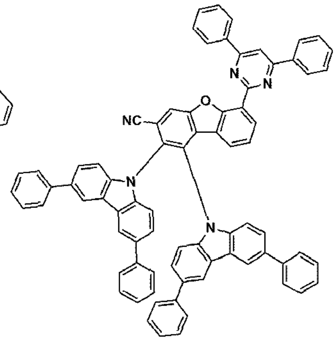
140

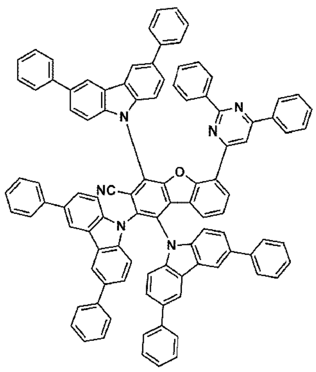
141

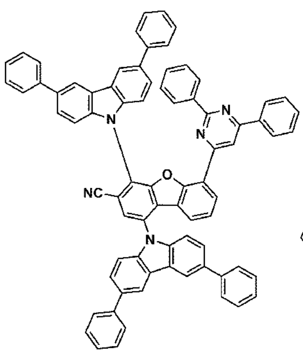
142

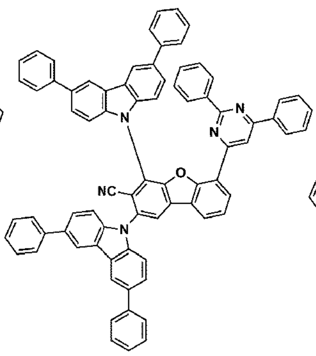
143

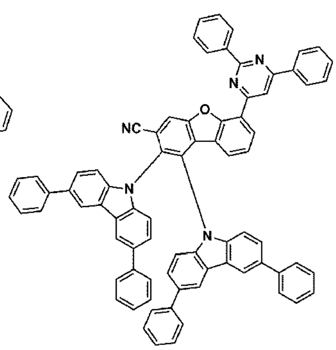
144

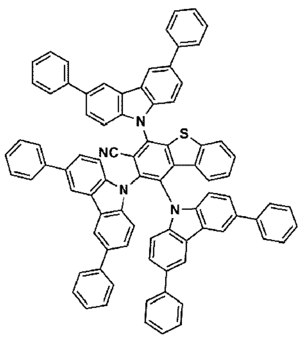
145

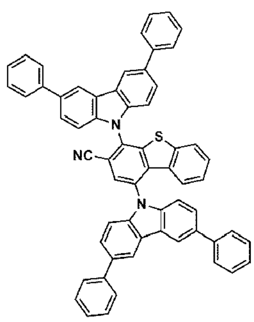
146

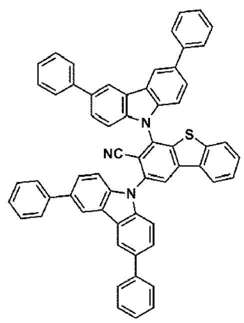
147

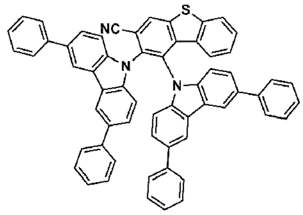
148

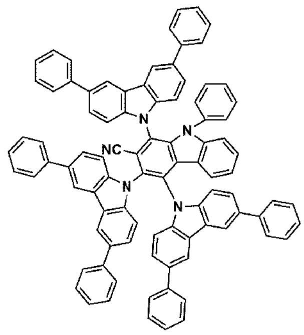

149

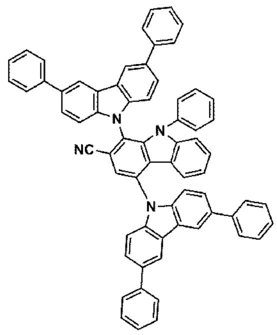

150

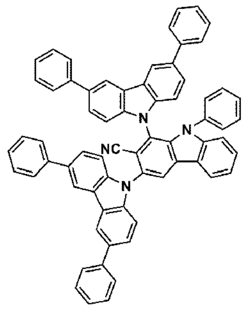

151

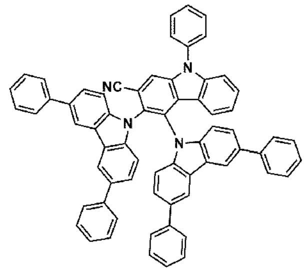

152

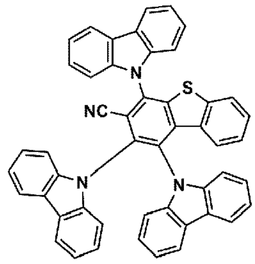

153

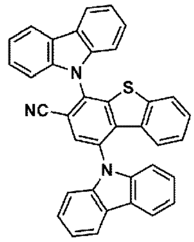

154

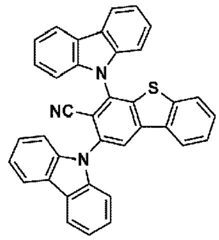

155

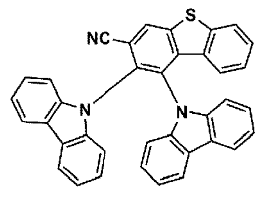

156

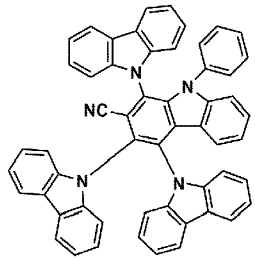

157

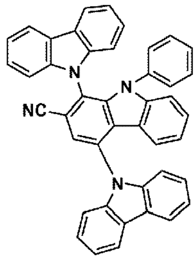

158

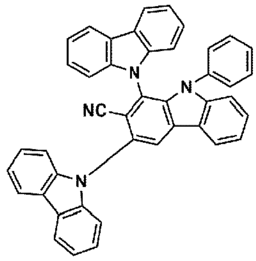

159

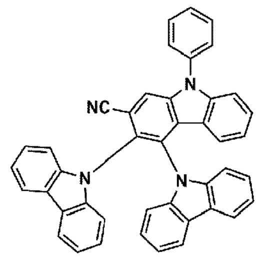

160

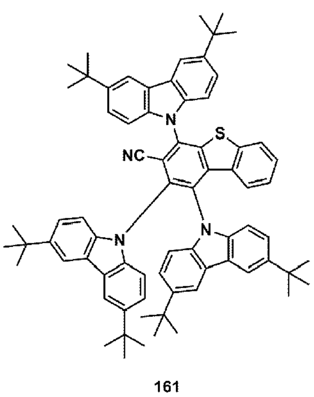

161

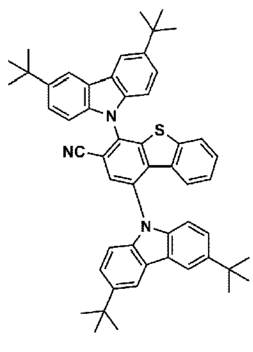

162

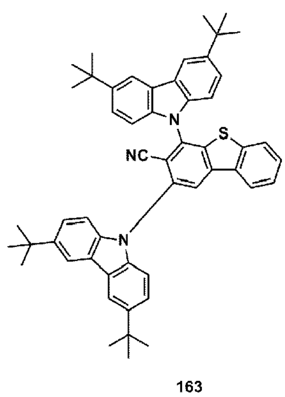

163

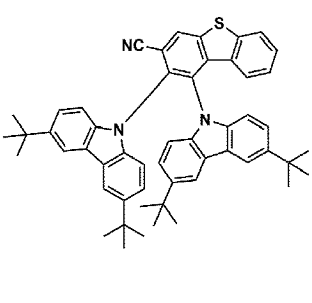

164

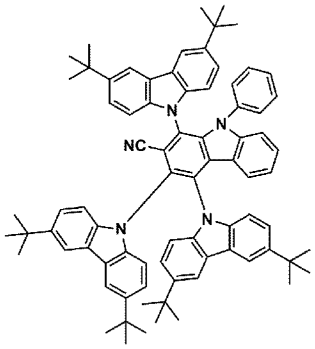
165

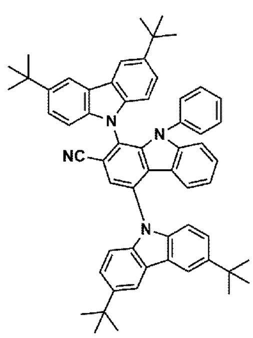
166

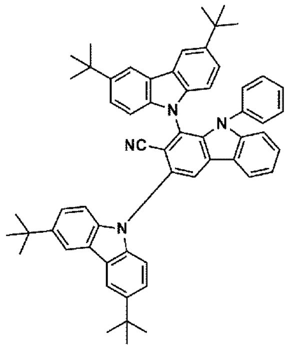
167

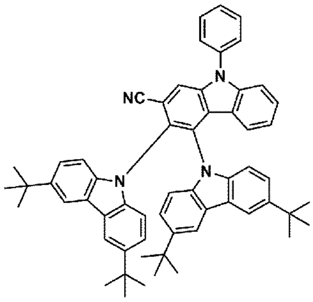
168

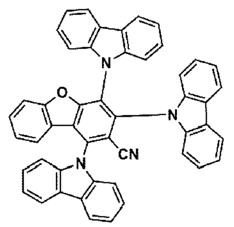
169

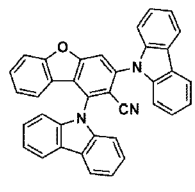
170

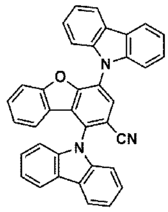
171

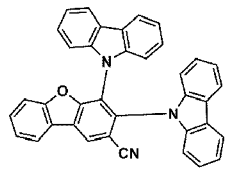
172

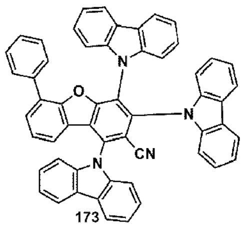
173

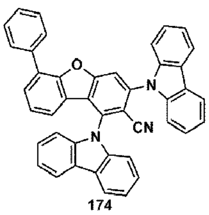
174

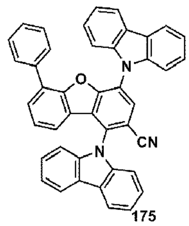
175

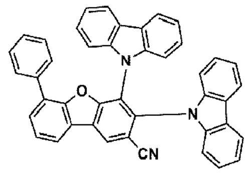
176

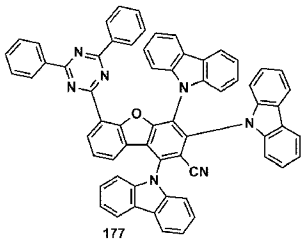
177

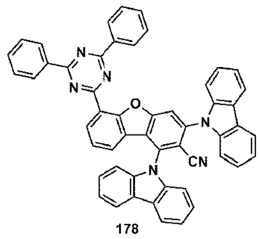
178

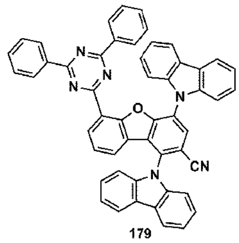
179

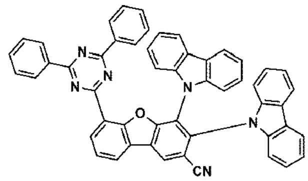
180

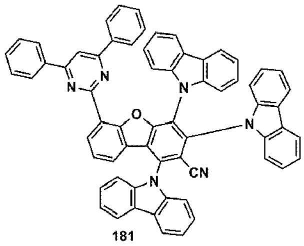
181

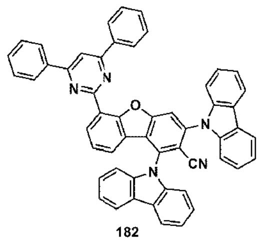
182

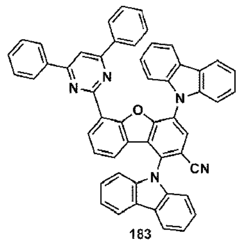
183

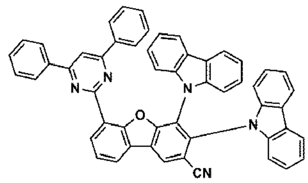
184

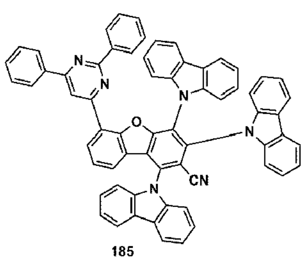
185

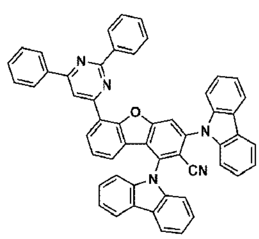
186

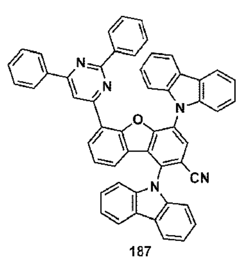
187

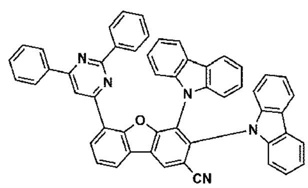
188

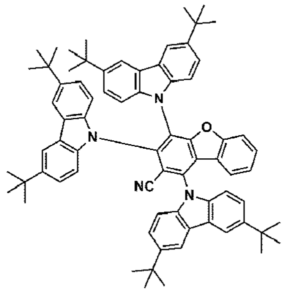

189

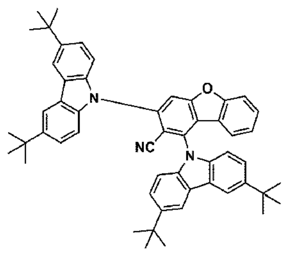

190

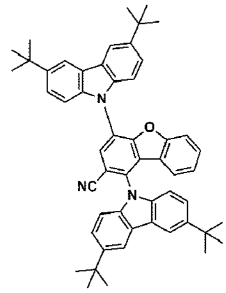

191

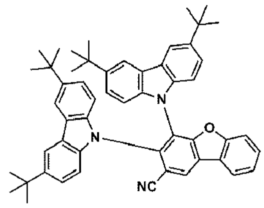

192

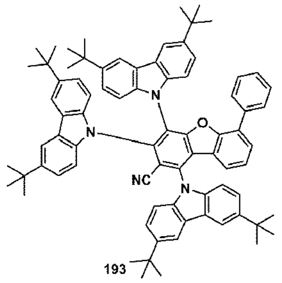

193

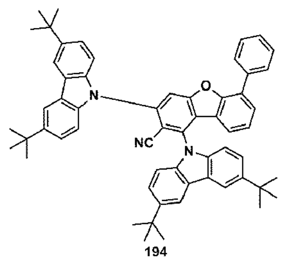

194

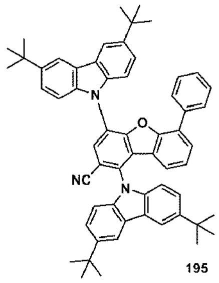

195

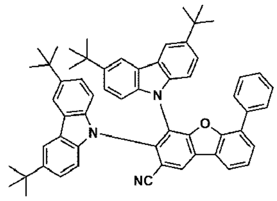

196

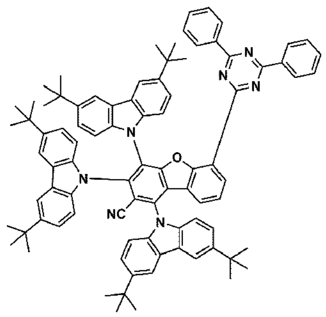

197

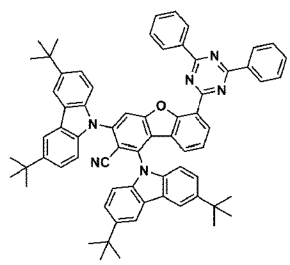

198

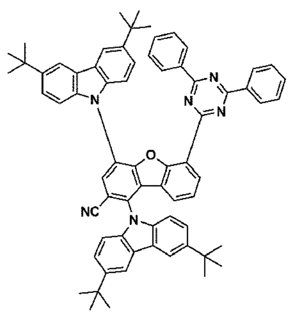

199

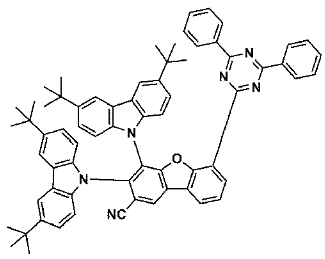

200

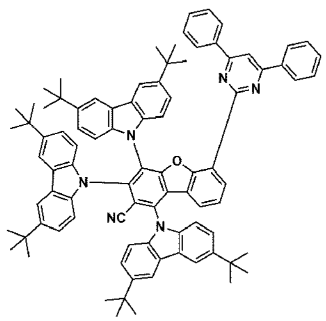

201

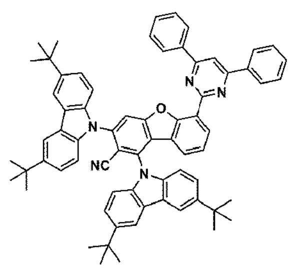

202

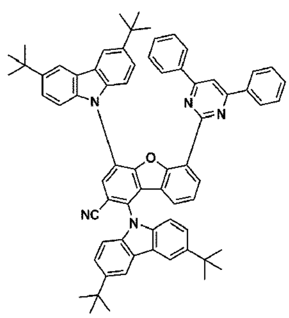

203

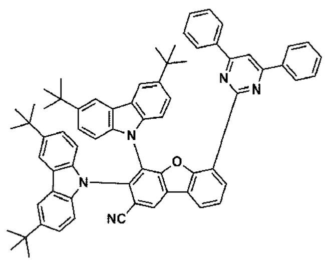

204

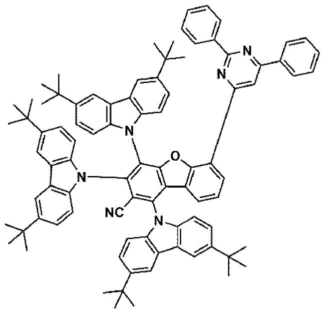
205

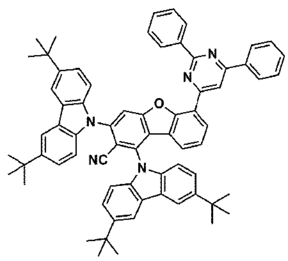
206

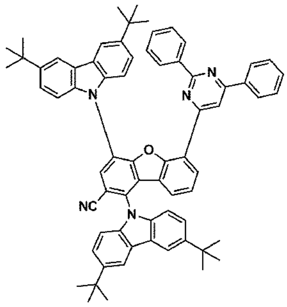
207

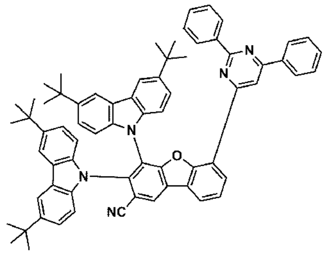
208

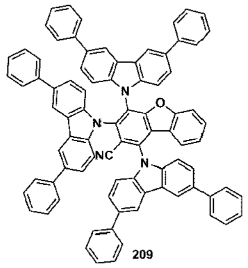
209

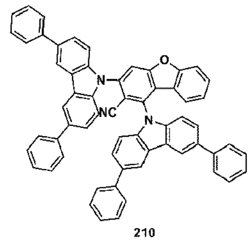
210

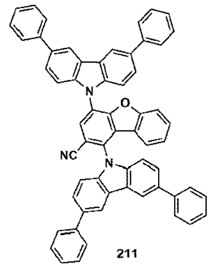
211

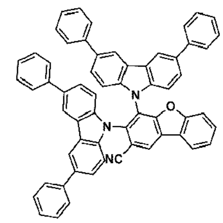
212

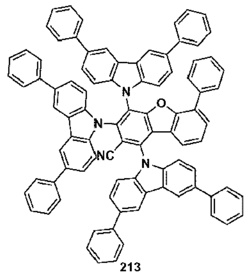
213

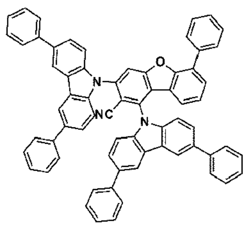
214

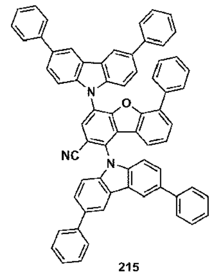
215

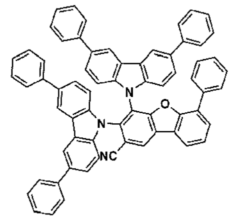
216

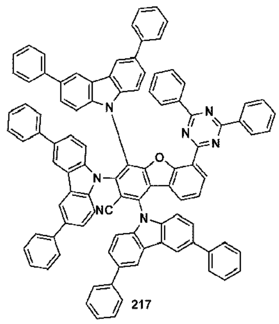
217

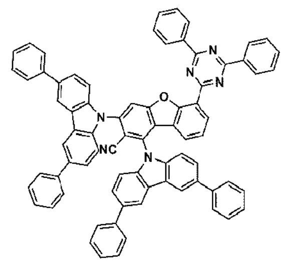
218

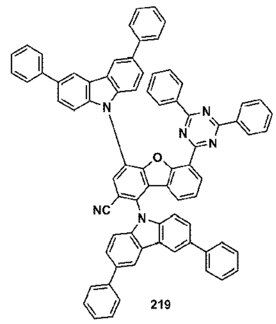
219

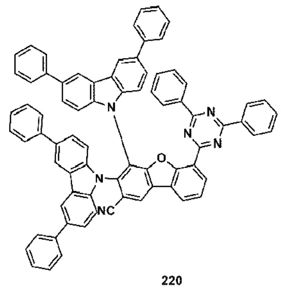
220

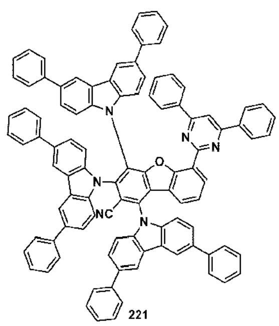
221

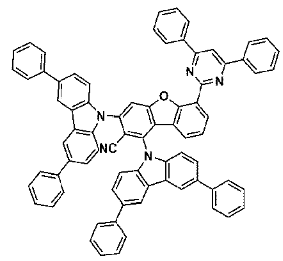
222

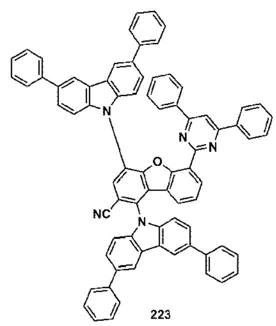
223

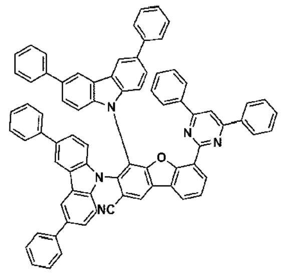
224

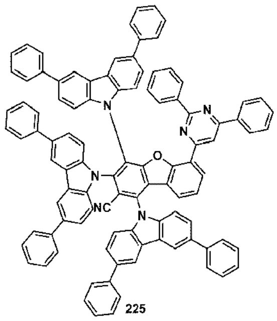

225

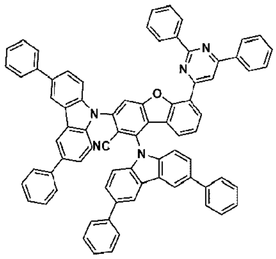

226

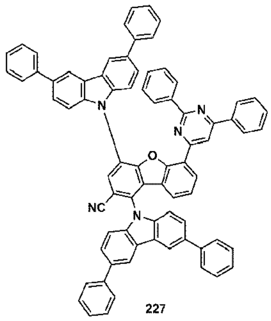

227

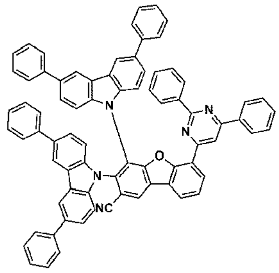

228

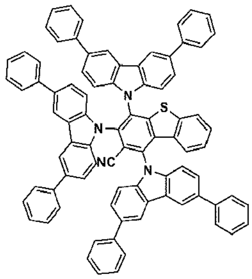

229

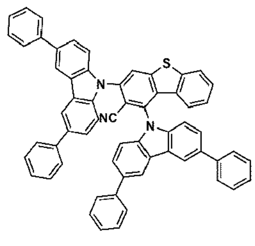

230

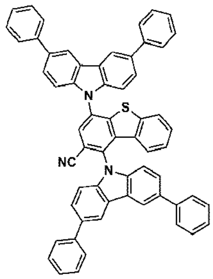

231

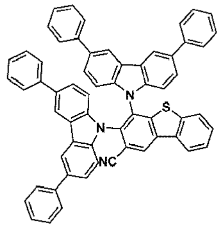

232

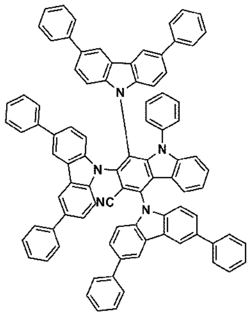

233

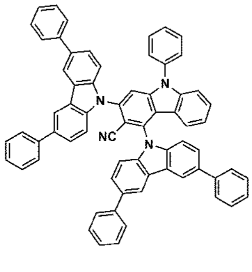

234

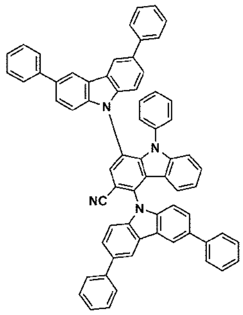

235

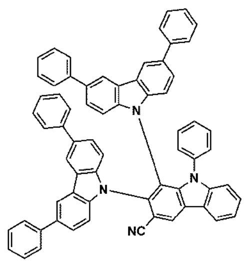

236

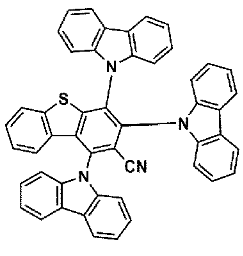

237

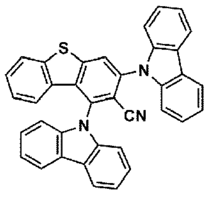

238

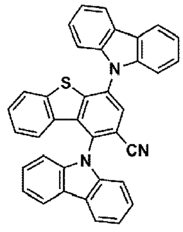

239

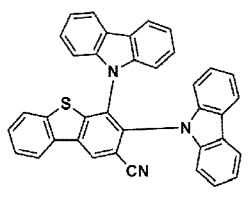

240

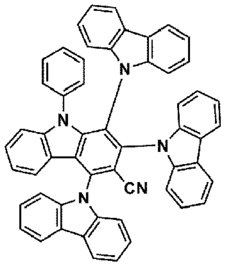

241

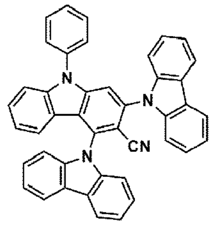

242

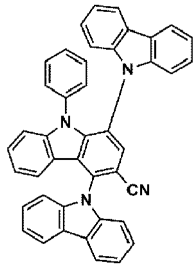

243

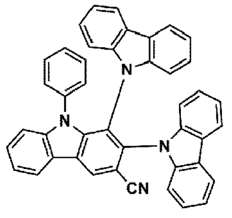

244

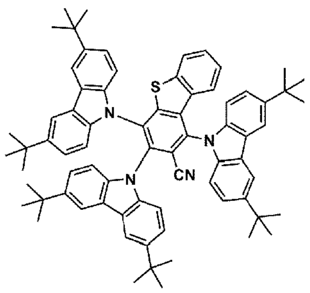

245

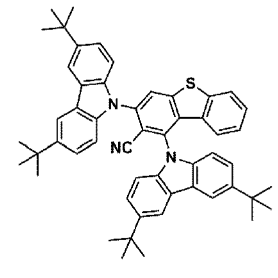

246

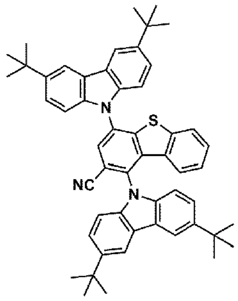

247

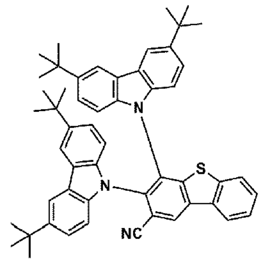

248

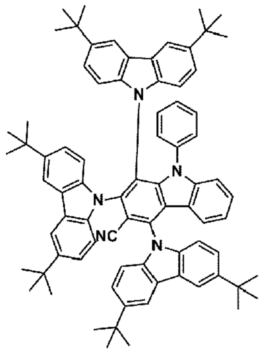

249

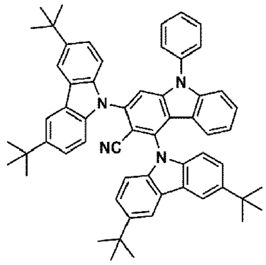

250

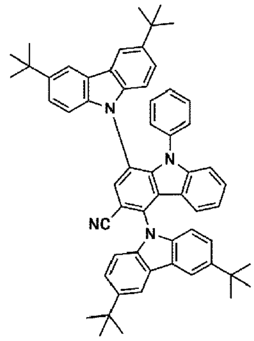

251

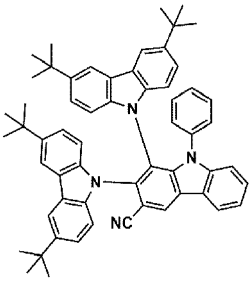

252

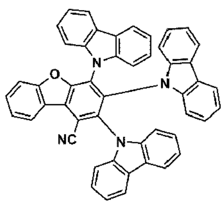

253

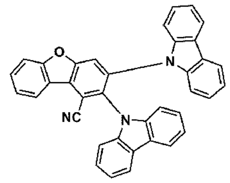

254

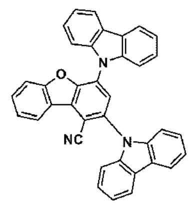

255

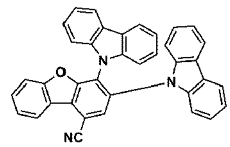

256

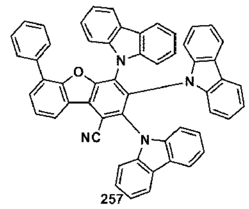

257

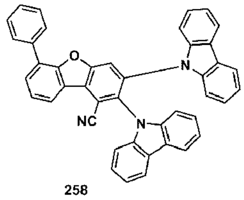

258

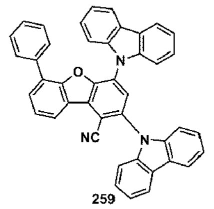

259

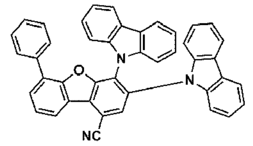

260

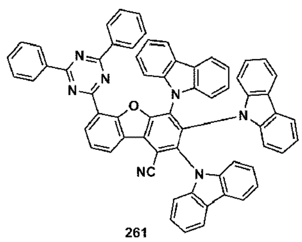

261

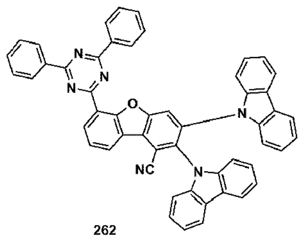

262

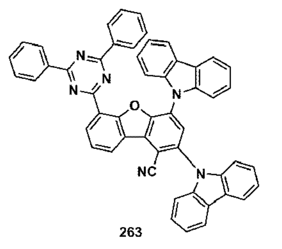

263

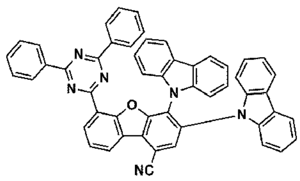

264

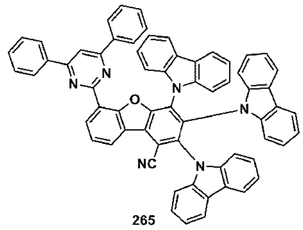

265

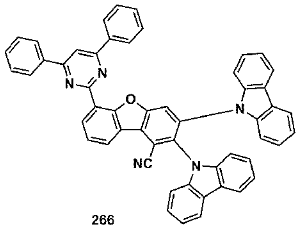

266

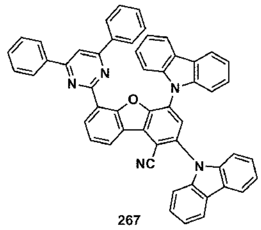

267

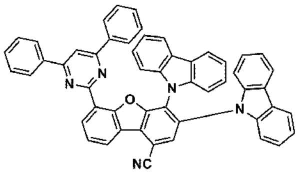

268

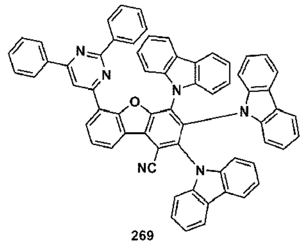

269

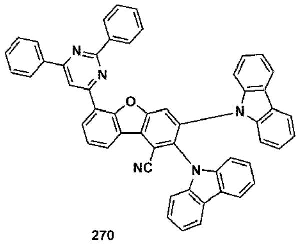

270

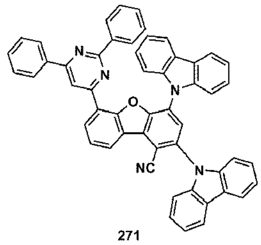

271

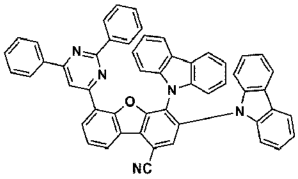

272

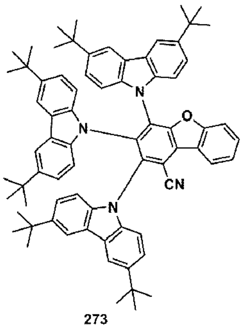

273

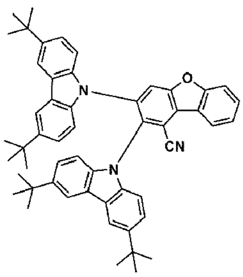

274

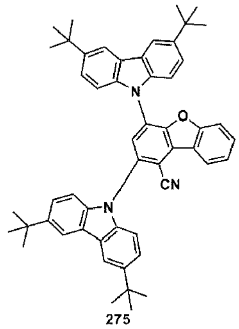

275

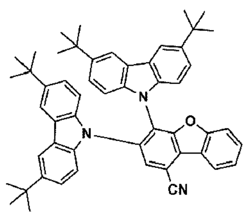

276

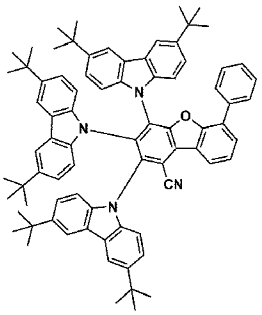

277

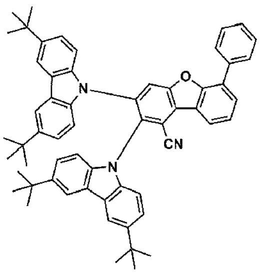

278

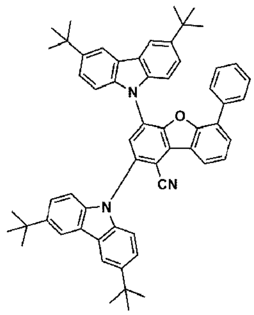

279

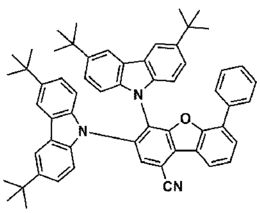

280

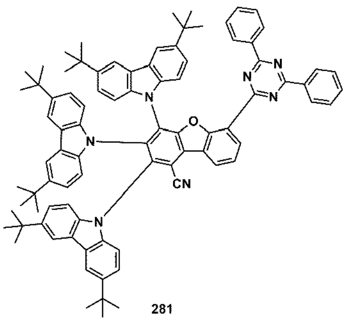

281

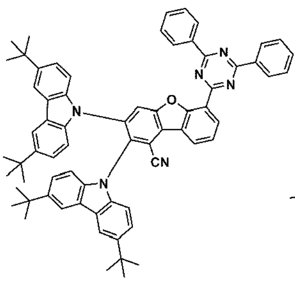

282

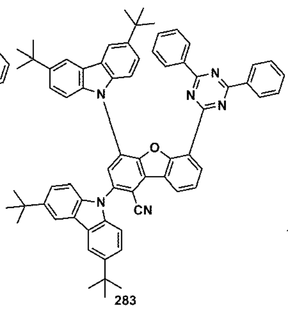

283

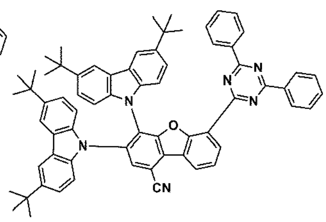

284

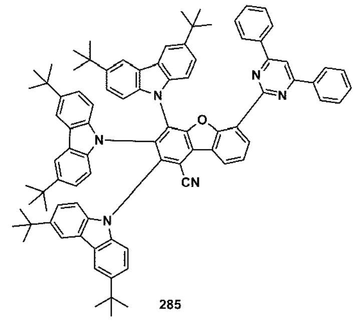
285

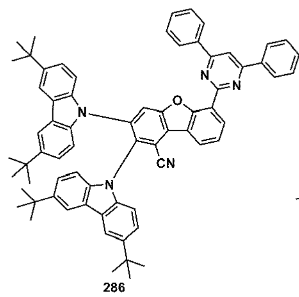
286

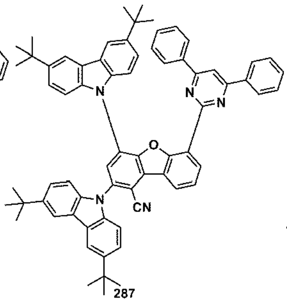
287

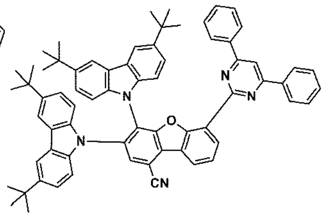
288

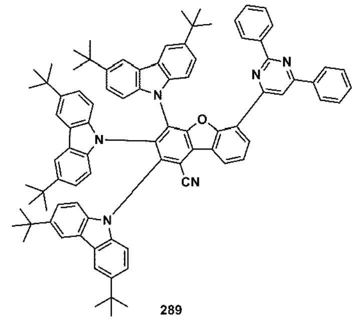
289

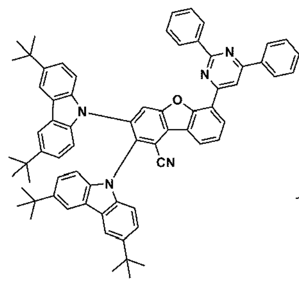
290

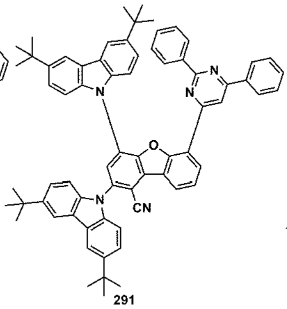
291

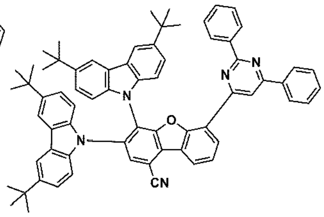
292

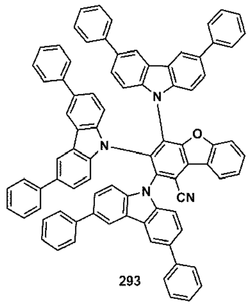
293

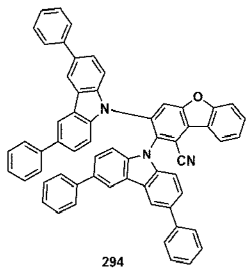
294

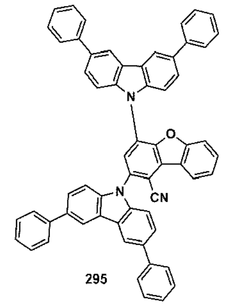
295

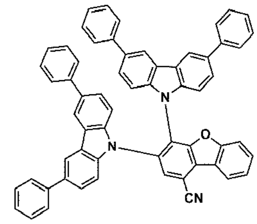
296

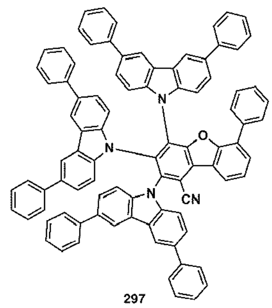
297

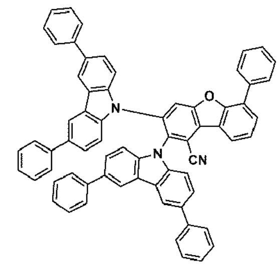
298

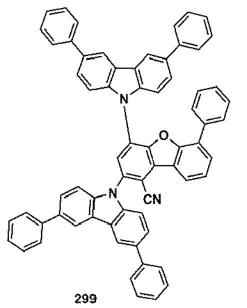
299

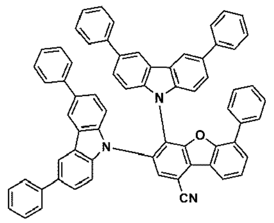
300

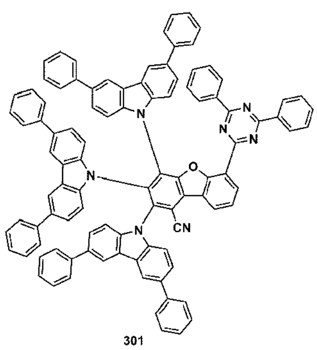
301

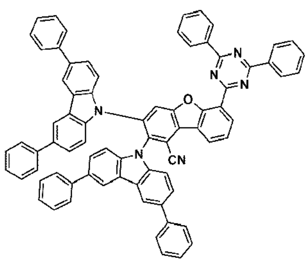
302

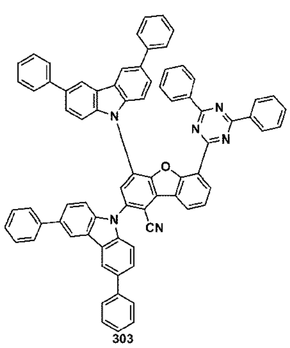
303

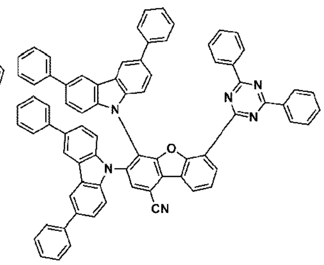
304

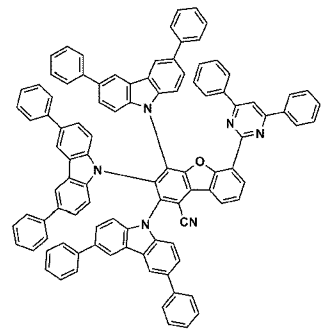

305

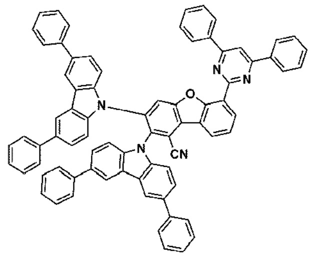

306

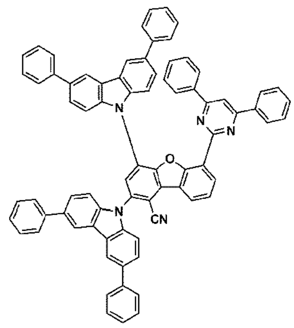

307

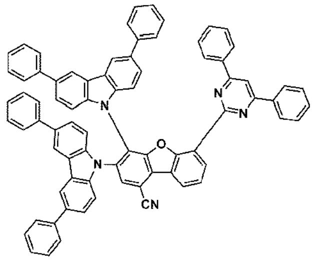

308

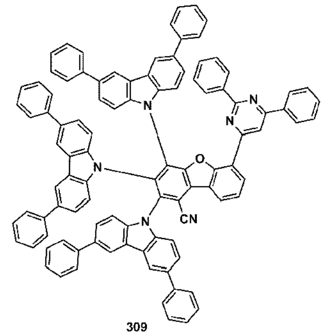

309

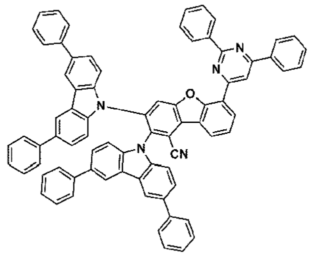

310

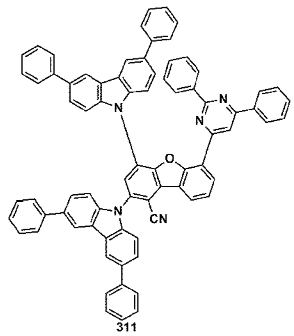

311

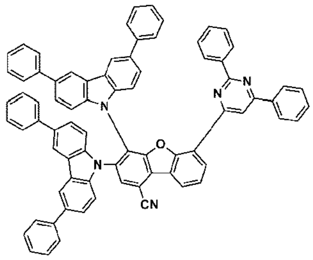

312

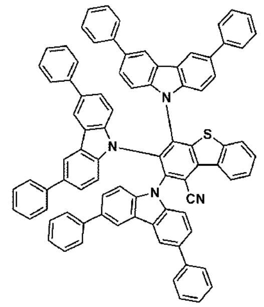

313

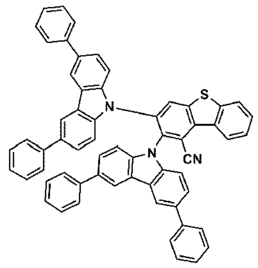

314

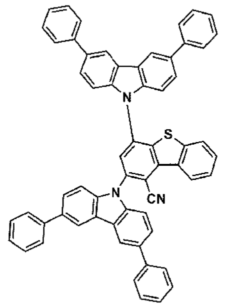

315

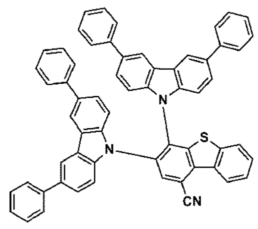

316

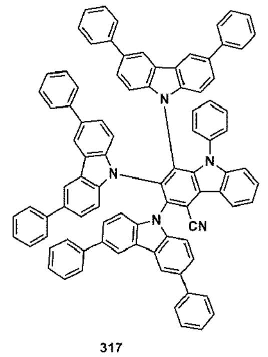

317

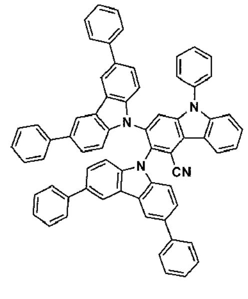

318

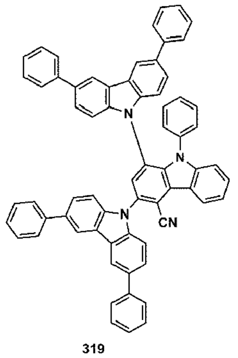

319

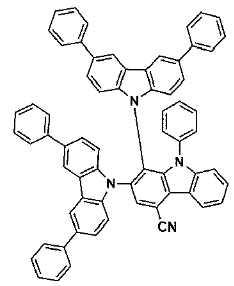

320

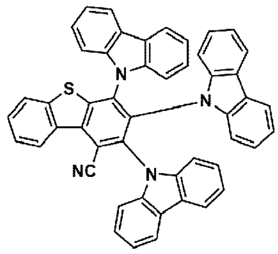

321

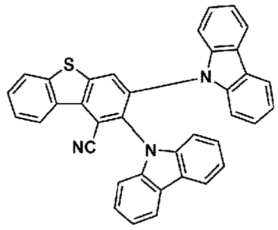

322

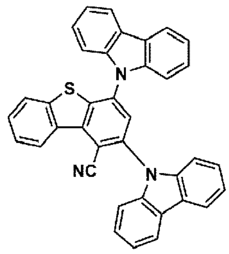

323

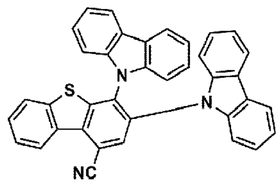

324

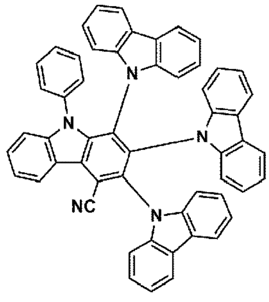

325

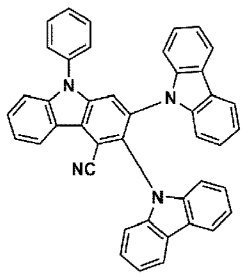

326

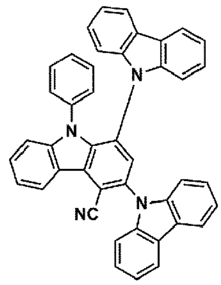

327

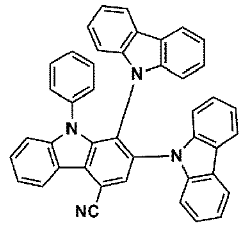

328

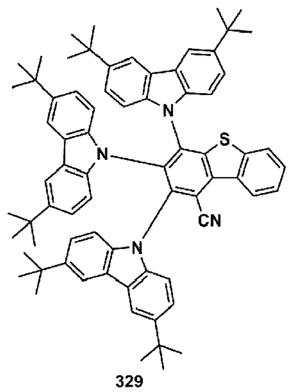

329

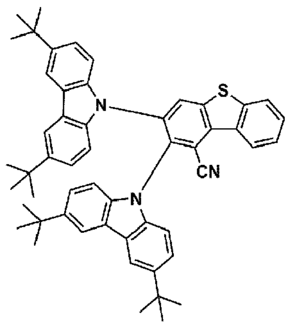

330

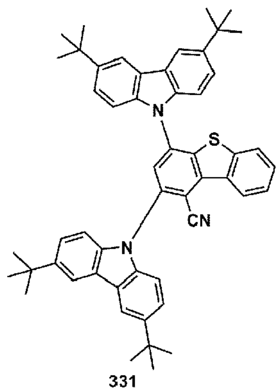

331

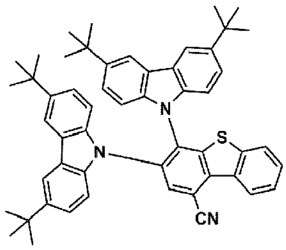

332

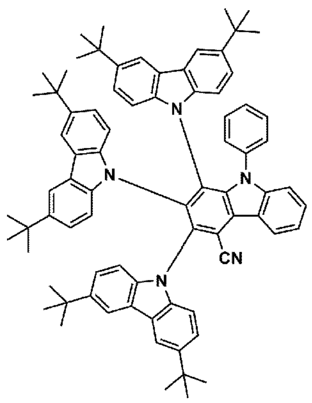

333

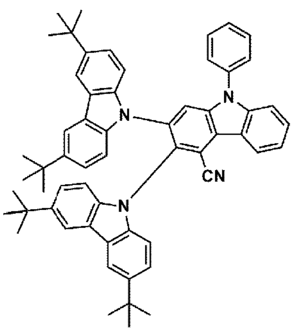

334

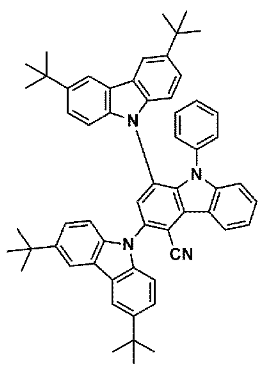

335

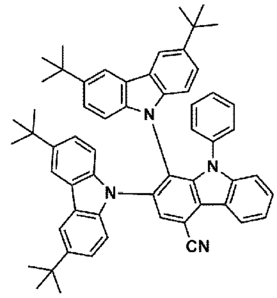

336

.

**11.** An organic light-emitting device comprising:

a first electrode;
a second electrode; and

an organic layer between the first electrode and the second electrode and comprising an emission layer, wherein the organic layer comprises the condensed-cyclic compound of any of claims 1-10.

**12.** The organic light-emitting device of claim 11, wherein
the first electrode is an anode,
the second electrode is a cathode,
the organic layer comprises a hole transport region between the first electrode and the emission layer and an electron transport region between the emission layer and the second electrode,
the hole transport region comprises a hole injection layer, a hole transport layer, an electron blocking layer, or a combination thereof, and
the electron transport region comprises a hole blocking layer, an electron transport layer, an electron injection layer, or a combination thereof.

**13.** The organic light-emitting device of claims 11 or 12, wherein the emission layer comprises the condensed-cyclic compound of any of claims 1-10;
preferably wherein a ratio of a fluorescent emission component to total emission components emitted from the emission layer is 90% or more.

**14.** The organic light-emitting device of claim 13, wherein
the condensed-cyclic compound is a fluorescent emitter, and
a ratio of an emission component emitted from the condensed-cyclic compound to total emission components emitted from the emission layer is 80% or more;
preferably wherein
the emission layer consists of the condensed-cyclic compound alone, or the emission layer further comprises a host.

**15.** The organic light-emitting device of claims 13 or 14, wherein
the emission layer comprises a host and a dopant,
the host comprises the condensed-cyclic compound,
an amount of the host is greater than an amount of the dopant, and
a ratio of an emission component of the dopant to total emission components emitted from the emission layer is 80% or more; and/or
wherein
the emission layer comprises a host, an auxiliary dopant, and a dopant,
the auxiliary dopant comprises the condensed-cyclic compound, and
a ratio of the dopant to total emission components emitted from the emission layer is 80% or more.

FIGURE

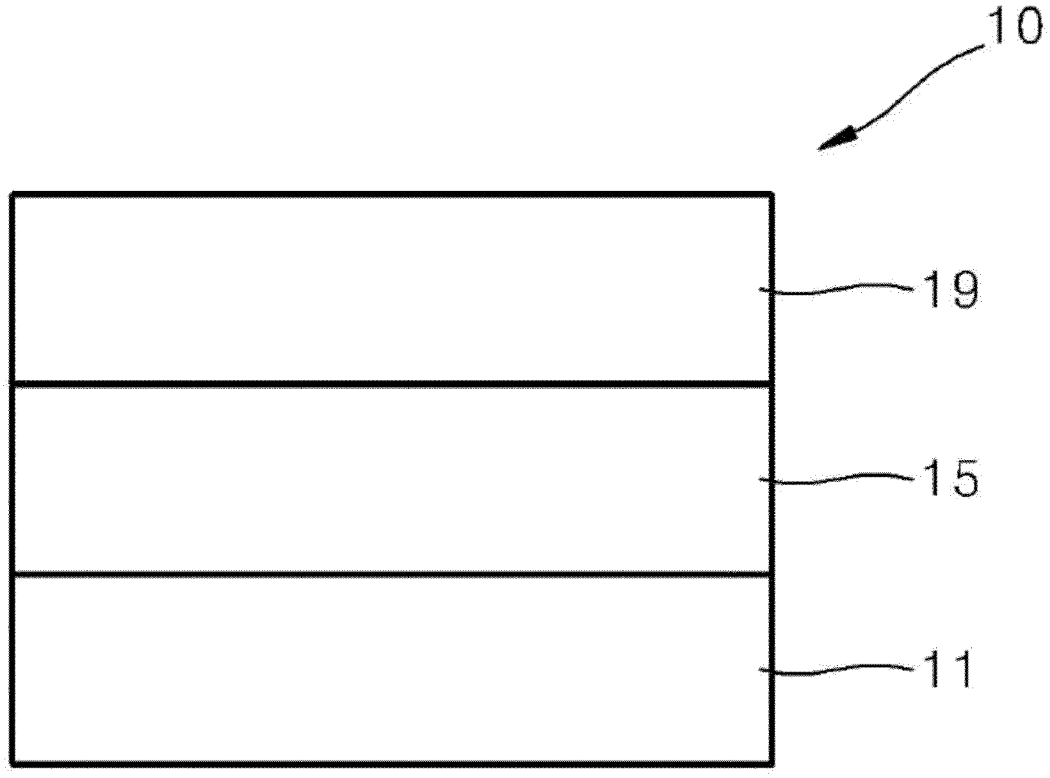

Europäisches Patentamt
European Patent Office
Office européen des brevets

# EUROPEAN SEARCH REPORT

Application Number
EP 19 20 3036

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/166634 A1 (NUMATA MASAKI [KR] ET AL) 14 June 2018 (2018-06-14)<br>* paragraph [0486] - paragraph [0506]; claims 19, 20; tables 1-4 (example 10); compound 936 *<br>----- | 1-15 | INV.<br>C07D403/14<br>C07D405/14<br>C07D409/14<br>C09K11/06<br>H01L51/00 |
| X | US 2016/204361 A1 (MIZUKI YUMIKO [CH] ET AL) 14 July 2016 (2016-07-14)<br>* 1st compound on the right of page 18; paragraphs [0118], [0120]; claims 1-7 *<br>----- | 1-15 | |
| X,P | RAJENDRA KUMAR KONIDENA ET AL: "Molecular design and synthetic approach to C2,C3,C4-modified carbazoles: high triplet energy bipolar host materials for efficient blue phosphorescent organic light emitting diodes",<br>CHEMICAL COMMUNICATIONS,<br>vol. 55, no. 56, 18 June 2019 (2019-06-18), pages 8178-8181, XP055671085,<br>UK<br>ISSN: 1359-7345, DOI: 10.1039/C9CC03843D<br>* abstract; figure 1; compounds 24CzCzCN, 24CzCNCzCN *<br>----- | 1-15 | TECHNICAL FIELDS SEARCHED (IPC)<br>C07D |
| X,P | WO 2019/160315 A1 (LG CHEMICAL LTD [KR] ET AL.) 22 August 2019 (2019-08-22)<br>* paragraphs 191-192, 195-196, 239-240 and 243-244: preparation of compounds 1, 2, 5 and 6;<br>5th compound of page 38 (from left to right), 5th compound of page 40, 4th, 5th, 7th and 8th compound of page 41, 4th and 5th compound of page 43 etc.;<br>claims 2 (formula 3), 5-9 *<br>----- | 1-15 | |

The present search report has been drawn up for all claims

1

| Place of search | Date of completion of the search | Examiner |
|---|---|---|
| Munich | 26 February 2020 | Stroeter, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 19 20 3036

This annex lists the patent family members relating to the patent documents cited in the above-mentioned European search report. The members are as contained in the European Patent Office EDP file on
The European Patent Office is in no way liable for these particulars which are merely given for the purpose of information.

26-02-2020

| Patent document cited in search report | Publication date | Patent family member(s) | Publication date |
|---|---|---|---|
| US 2018166634 A1 | 14-06-2018 | KR 20180068882 A | 22-06-2018 |
| | | US 2018166634 A1 | 14-06-2018 |
| US 2016204361 A1 | 14-07-2016 | CN 105408310 A | 16-03-2016 |
| | | JP 6370791 B2 | 08-08-2018 |
| | | JP WO2015033894 A1 | 02-03-2017 |
| | | KR 20160044458 A | 25-04-2016 |
| | | US 2016204361 A1 | 14-07-2016 |
| | | WO 2015033894 A1 | 12-03-2015 |
| WO 2019160315 A1 | 22-08-2019 | KR 20190098079 A | 21-08-2019 |
| | | WO 2019160315 A1 | 22-08-2019 |

EPO FORM P0459

For more details about this annex : see Official Journal of the European Patent Office, No. 12/82